# EUROPÄISCHE PATENTSCHRIFT

(11) **EP 3 586 632 B1**
(45) Veröffentlichungstag und Bekanntmachung des Hinweises auf die Patenterteilung: **28.09.2022**
(21) Anmeldenummer: 19191309.4
(22) Anmeldetag: 22.06.2012
(51) Int. Cl.: A01N 43/90, A61K 41/00, C07D 475/14

(54) **10H-BENZO[G]PTERIDIN-2,4-DION-DERIVATE, VERFAHREN ZUR HERSTELLUNG UND VERWENDUNG DERSELBEN**
10H-BENZO[G]PTERIDINE-2,4-DIONE DERIVATIVES, METHOD FOR PRODUCING SAME AND USE OF SAME
DÉRIVÉS DE 10H-BENZO[G]PTERIDIN-2,4-DION, LEUR PROCÉDÉ DE PRODUCTION ET D'UTILISATION

(30) Priorität: 22.06.2011 DE 102011105653
(43) Veröffentlichungstag der Anmeldung: 01.01.2020
(62) Teilanmeldung aus: 12735235.9
(73) Patentinhaber: TriOptoTec GmbH, 93053 Regensburg (DE)
(72) Erfinder: MAISCH, Tim, 90425 Nürnberg (DE); SPÄTH, Andreas, 93055 Regensburg (DE)
(74) Vertreter: Louis Pöhlau Lohrentz

(56) Entgegenhaltungen:
- SEIJI SHINKAI ET AL: "Coenzyme models. Part 23. Formation and reactivity of the stable ?quinone form? of flavin in cationic polymer matrices", JOURNAL OF THE CHEMICAL SOCIETY, PERKIN TRANSACTIONS 1, 1. Januar 1980 (1980-01-01), Seite 1622, XP55034011, ISSN: 0300-922X, DOI: 10.1039/P19800001622
- RON BLONDER ET AL: "Application of a Nitrospiropyran-FAD-Reconstituted Glucose Oxidase and Charged Electron Mediators as Optobioelectronic Assemblies for the Amperometric Transduction of Recorded Optical Signals:? Control of the "On"-"Off" Direction of the Photoswitch", JOURNAL OF THE AMERICAN CHEMICAL SOCIETY, Bd. 119, Nr. 49, 1. Dezember 1997 (1997-12-01), Seiten 11747-11757, XP55034326, ISSN: 0002-7863, DOI: 10.1021/ja972663v
- R. B. BARLOW ET AL: "144. A series of 9-dialkylaminoalkylisoalloxazines", JOURNAL OF THE CHEMICAL SOCIETY (RESUMED), 1. Januar 1950 (1950-01-01), Seite 713, XP55034329, ISSN: 0368-1769, DOI: 10.1039/jr9500000713
- CHRISTINA K. REMUCAL ET AL: "Photosensitized Amino Acid Degradation in the Presence of Riboflavin and Its Derivatives", ENVIRONMENTAL SCIENCE & TECHNOLOGY, Bd. 45, Nr. 12, 15. Juni 2011 (2011-06-15) , Seiten 5230-5237, XP55034035, ISSN: 0013-936X, DOI: 10.1021/es200411a

## Beschreibung

Die vorliegende Erfindung betrifft 10H-Benzo[g]pteridin-2,4-dion-Derivate, deren Herstellung und Verwendung.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegen wirken.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen. Die Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Es ist bekannt, dass 10-Methyl-10H-benzo[g]pteridine-2,4-dion-Derivate, Riboflavin und Tetraacetylriboflavin hohe Ausbeuten an Singulett-Sauerstoff haben, wobei jedoch ihre Affinität zu Mikroorganismen gering ist. Es ist weiterhin bekannt, dass Singulett-Sauerstoff lediglich über eine geringe Entfernung diffundieren kann, bevor er reagiert oder abgebaut wird. Daher ist die Inaktivierung von Mikroorganismen durch 10-Methyl-10H-benzo[g]pteridine-2,4-dion-Derivate, Riboflavin und Tetraacetylriboflavin unzureichend.

Weiterhin sind aus der WO 2010/019208 A1 und der WO 2011/008247 A1 zahlreiche Flavin-, Roseoflavin- und Riboflavin-Derivate bekannt, die an Flavinmononucleotid (FMN) Riboswiches binden können. Riboswiches sind RNA-Elemente in untranslatierten Regionen der mRNA von Prokaryoten, Pilzen und Pflanzen, die niedermolekulare Metabolite, beispielsweise FMN, binden und daraufhin die Genexpression regulieren. Beispielsweise wird nach Bindung von FMN an FMN-Riboswiches von Prokaryoten die Expression von Enzymen, die für die Riboflavin- und FMN-Biosynthese verantwortlich sind, reprimiert, wodurch die Riboflavin- und FMN-Biosynthese zum Erliegen kommt. Riboflavin nimmt im Stoffwechsel eine zentrale Rolle ein, da es als Vorstufe für Flavin-Koenzyme dient. Daher führt eine unterdrückte Riboflavin- und FMN-Biosynthese zu einer reduzierten Überlebensfähigkeit. Christina K. Remucal ET AL: "Photosensitized Amino Acid Degradation in the Presence of Riboflavin and Its Derivatives",Environmental Science & Technology, Bd. 45, Nr. 12, 15. Juni 2011 (2011-06-15), Seiten 5230-5237, offenbart die Verwendung von Riboflavin-Derivaten als Photosensibilatoren bei der photodynamischen Therapie und schlägt auch das Derivatizieren von Riboflavin vor.

Die vorliegende Anmeldung betrifft die photodynamische Therapie und die zugrundeliegende Aufgabe ist somit als die Verwendung weiterer Photosensibilisatoren , wo eine solche Wirkung wünschenswert ist, anzusehen. Die Aufgabe der vorliegenden Erfindung wird gelöst durch die Bereitstellung für diese Anwendung einer Verbindung mit der Formel (1): 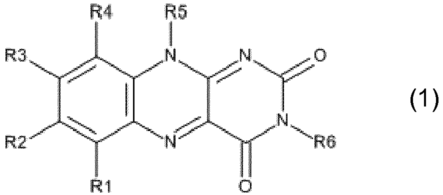 wie in den Ansprüchen definiert. Dabei handelt es sich um die Formel (1), wobei
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
   wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
   wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet
      oder
   wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
   wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen,Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten.

Bei der erfindungsgemäßen Verbindung mit der Formel (1) handelt es sich um ein 10H-Benzo[g]pteridin-2,4-dion- oder Flavin-Derivat, das im Folgenden auch so bezeichnet wird.

Als Gegenion zum positiv geladenen quartären Stickstoffatom kann jedes geeignete Anion verwendet werden. Vorzugsweise werden als Gegenion zum positiv geladenen quartären Stickstoffatom Anionen verwendet, die die Bereitstellung eines pharmakologisch verträglichen Salzes ermöglichen.

Bei einer bevorzugten Ausführungsform der vorliegenden Erfindung ist X in der Verbindung mit der Formel (1) ein organischer Rest mit nur einem quartären Stickstoffatom, das als Gegenion Fluorid, Chlorid, Bromid, lodid, Sulfat, Hydrogensulfat, Phosphat, Dihydrogenphosphat, Hydrogenphosphat, Tosylat, Mesylat, Formiat, Acetat, Oxalat, Benzoat, Citrat und/oder Mischungen davon aufweist.

Offenbart wird die Bereitstellung eines Verfahrens zur Herstellung einer Verbindung nach Anspruch 1, wobei das Verfahren folgende Schritte umfasst:
(A) Reduzieren eines substituierten Nitroanilins der Formel (22) zu einem substituierten o-Phenylendiamin der Formel (23), 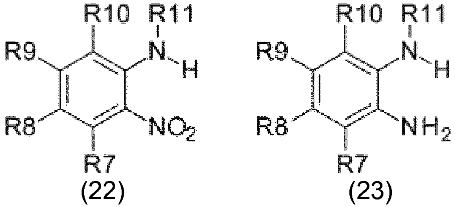 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und wobei der Rest R11 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Kondensieren des in Schritt (A) erhaltenen substituierten o-Phenylendiamin der Formel (23) mit Alloxan oder dessen Hydrat unter Erhalt einer Verbindung mit der Formel (24): 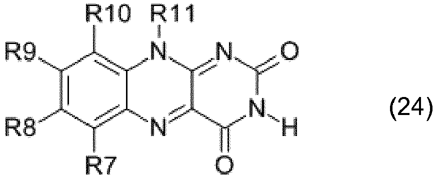
(C) Optional Umsetzen der in Schritt (B) erhaltenen Verbindung der Formel (24) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R gleich oder unabhängig voneinander verschieden sein kann und vorzugsweise Methyl, Ethyl, Propyl oder Butyl ist, bedeutet, unter Erhalt einer Verbindung mit der Formel (25): 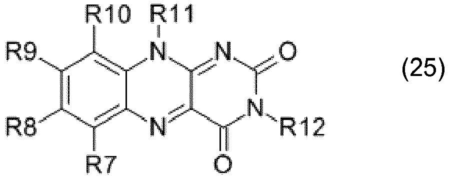
(D) Optional Umsetzen der in Schritt (B) erhaltenen Verbindung der Formel (24) oder der in Schritt (C) erhaltenen Verbindung der Formel (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel - (C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R7 bis R12 ein organischer Rest der allgemeinen Formel -(C(D)(E))h-X oder -(C(D)(E))ₖ-Aryl-(C(D)(E))ₗ-X ist und wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet.

Offenbart wird auch die Bereitstellung eines Verfahrens zur Herstellung einer Verbindung nach Anspruch 1, wobei das Verfahren folgende Schritte umfasst:
(A) Kondensieren eines Amins mit der Formel R11-NH₂ mit einem Chloruracil-Derivate der Formel (26), optional in Gegenwart eines Katalysators, vorzugsweise Lewis-Säure oder Broenstedt-Säure, unter Erhalt einer Verbindung mit der Formel (27): 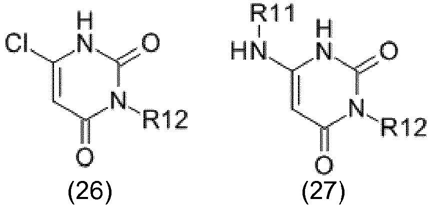 wobei jeder der Reste R11 oder R12 jeweils unabhängig voneinander gleich oder voneinander verschieden sein kann Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Umsetzen der in Schritt (A) erhaltenen Verbindung der Formel (27) mit einer Nitrosoverbindung der Formel (28) unter Erhalt einer Verbindung der Formel (25): 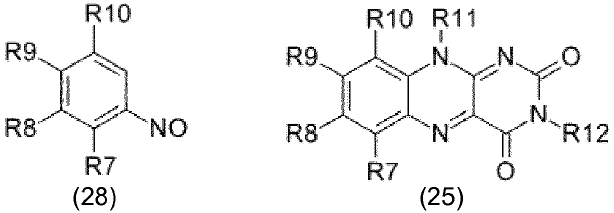 wobei jeder der Reste R7 bis R10 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
(C) Optional Umsetzen der in Schritt (B) erhaltenen Verbindung der Formel (23) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel -(C(D)(E))ₕ-X oder -(C(D)(E))ₖ-Aryl-(C(D)(E))ₗ-X ist und wenn 1 Rest R7 bis R12 ein organischer Rest der allgemeinen Formel - (C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R7 bis R12 ein organischer Rest der allgemeinen Formel -(C(D)(E))h-X oder -(C(D)(E))ₖ-Aryl-(C(D)(E))ₗ-X ist und wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet.

Weitere bevorzugte Ausführungsformen der vorliegenden Erfindung sind in den abhängigen Ansprüchen beschrieben.

Bei einer weiter bevorzugten Ausführungsform enthält das erfindungsgemäße 10H-Benzo[g]pteridin-2,4-dion-Derivats der Formel (1) keine basischen, vorzugsweise neutralen, protonierbaren und/oder positiv geladenen, Stickstoffatome und keine weiteren quartären Stickstoffatome. Als "Photosensibilisator" werden erfindungsgemäß Verbindungen verstanden, die Licht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte Inaktivierung von Zellen oder Mikroorganismen verstanden. Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäßen Verbindung mit der Formel (1) bestimmt werden. Erfindungsgemäß wird unter einer Reduzierung der Lebensfähigkeit verstanden, dass die Anzahl der Mikroorganismen um wenigstens 99,0 %, vorzugsweise um wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 %, verringert wird.

Vorzugsweise wird die Reduzierung der Anzahl der Mikroorganismen gemäß Boyce, J.M. und Pittet, D. ("Guidelines for hand hygiene in healthcare settings.

Recommendations of the Healthcare Infection Control Practices Advisory Committee and the HIPAC/SHEA/APIC/IDSA Hand Hygiene Task Force", Am.J.lnfect.Control 30 (8), 2002, Seite 1 - 46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäßen Verbindung mit der Formel (1) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H.F. und Schwebke, I. ("Leitlinie der Deutschen Vereinigung zur Bekämpfung der Viruskrankheiten (DVV) e.V. und des Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäßen Verbindung mit der Formel (1) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäßen Verbindungen mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäßen Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Bakterien und Bakteriensporen, und eukaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Ein erfindungsgemäßes 10H-Benzo[g]pteridin-2,4-dion-Derivat weist die Formel (1) auf, 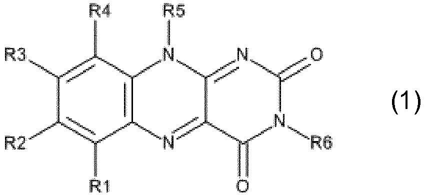 wobei entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X ist, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist
   oder
B) nur 1 Rest R5 oder R6 ein organischer Rest der allgemeinen Formel -(C(D)(E))ₕ-X oder -(C(D)(E))ₖ-Aryl-(C(D)(E))ₗ-X ist, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist.

Bei einer bevorzugten Ausführungsform weist das erfindungsgemäßes 10H-Benzo[g]pteridin-2,4-dion-Derivat der Formel (1) kein neutrales, protonierbares Stickstoffatom, das direkt an den Isoalloxazinring gebunden ist, beispielsweise als Amino-Rest, Methylamino-Rest oder Dimethylamino-Rest, und kein positiv geladenes Stickstoffatom, das direkt an den Isoalloxazinring gebunden ist, beispielsweise als Pyridin-1-ium-1-yl-Rest oder Trimethylammonio-Rest, auf.

Weiter bevorzugt enthält der organische Rest X keine basischen Stickstoffatome, vorzugsweise kein neutrales, protonierbares Stickstoffatom, und/oder kein protonierbares, positiv geladenes Stickstoffatom.

Bei Variante A) des erfindungsgemäßen 10H-Benzo[g]pteridin-2,4-dion-Derivats der Formel (1) ist nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist,
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und
wobei jeder der Reste R5 oder R6 jeweils unabhängig voneinander gleich oder voneinander verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet.

Bei Variante B) des erfindungsgemäßen 10H-Benzo[g]pteridin-2,4-dion-Derivats der Formel (1) ist nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und wobei X ein organischer Rest mit nur einem quartären Stickstoffatom ist,
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Ether mit 2 bis 20 C-Atomen, Thioether mit 2 bis 20 C-Atomen, Aryl mit 5 bis 20 C-Atomen oder Heteroaryl mit 4 bis 20 C-Atomen, das keine N-Atome enthält, bedeutet und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten

Bei einer weiteren bevorzugten Ausführungsform des erfindungsgemäßen 10H-Benzo[g]pteridin-2,4-dion-Derivats der Formel (1) ist entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest ist, der nur ein quartäres Stickstoffatom enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1, R2, R3 oder R4, die nicht ein organischer Rest der allgemeinen Formel -(C(D)(E))ₕ-X oder -(C(D)(E))ₖ-Aryl-(C(D)(E))ₗ-X sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
      oder
B) nur der Rest R6 ein organischer Rest der allgemeinen Formel -(C(D)(E))ₕ-X oder -(C(D)(E))ₖ-Aryl-(C(D)(E))ₗ-X,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest ist, der nur ein quartäres Stickstoffatom enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Reste R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform des erfindungsgemäßen 10H-Benzo[g]pteridin-2,4-dion-Derivats der Formel (1) ist nur der Reste R5 ein nichtcyclischer Polyolrest der allgemeinen Formel -CH₂(CH(OZ))_{f}CH₍₃₋ₑ₎(OZ)ₑ , wobei e 0, 1 oder 2 ist und f eine ganze Zahl von 1 bis 10, vorzugsweise 1 bis 4, bedeutet und wobei Z Wasserstoff, Alkyl mit 1 bis 20 C-Atomen, Carbonsäurester mit 1 bis 20 C-Atomen oder ein Rest X ist, wobei X ein organischer Rest mit nur einem quartären Stickstoffatom ist, mit der Maßgabe, dass nur 1 Rest Z ein organischer Rest X mit nur einem quartären Stickstoffatom ist,
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer bevorzugten Ausführungsform des 10H-Benzo[g]pteridin-2,4-dion-Derivats der Formel (1) ist der organische Rest X mit nur einem quartären Stickstoffatom ein Rest der allgemeinen Formel (2): 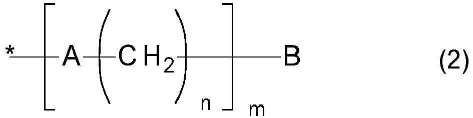 wobei A ein Sauerstoff- oder ein Schwefelatom ist und wobei n eine ganze Zahl von 1 bis 8 und m eine ganze Zahl von 0 bis 100, vorzugsweise 0-59, vorzugsweise 0-10, bedeuten und wobei B ein Rest der Formel (3), (4a), (4b), (5a) oder (5b): 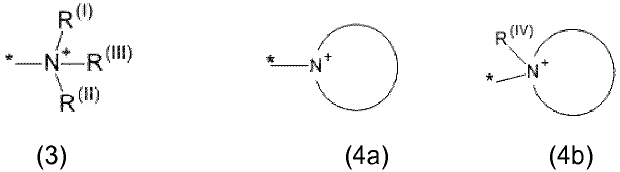 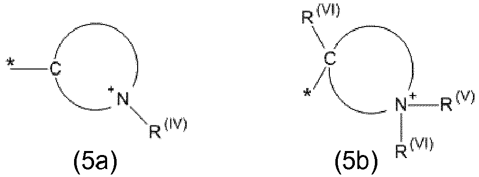 ist und wobei jeder der Reste R^{(I)}, R^{(II)}, R^{(III)}, R^{(IV)} und R^{(V)} unabhängig voneinander ein Arylrest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, oder ein Thioetherrest, der geradkettig oder verzweigt sein kann, mit 2 bis 20 C-Atomen ist und wobei der Rest R^{(VI)} Wasserstoff ein Arylrest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, oder ein Thioetherrest, der geradkettig oder verzweigt sein kann, mit 2 bis 20 C-Atomen ist und wobei der Rest mit der Formel (4a) und der Rest mit der Formel (5a): 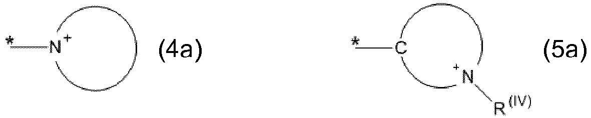 einen substituierten oder unsubstituierten heterocyclischen Rest mit 5 bis 7 Ringatomen, die 1 Stickstoffatom und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 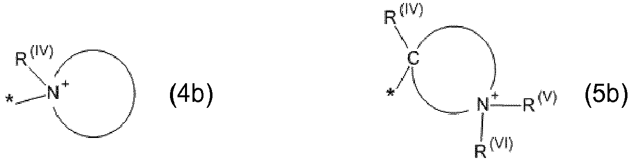 einen substituierten oder unsubstituierten heterocyclischen Rest mit 5 bis 7 Ringatomen, die 1 Stickstoffatom und mindestens 1 Kohlenstoffatom sowie optional 1 oder 2 Sauerstoff- oder Schwefelatom umfassen, darstellt.

Bei einer weiteren bevorzugten Ausführungsform wird der Rest der Formel (5a): aus der Gruppe, die aus Resten der Formeln (7a), (7b) und (7c): 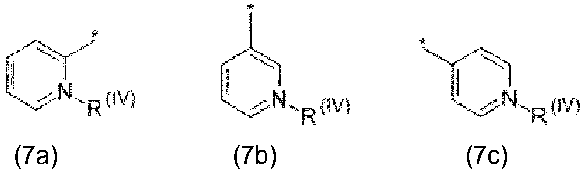 besteht, ausgewählt, wobei R^{(IV)} jeweils ein Arylrest mit 5 bis 20 C-Atomen, beispielsweise Phenyl oder Benzyl, Alkylrest, der geradkettig oder verzweigt sein kann, mit 1 bis 20 C-Atomen, beispielsweise Methyl, Ethyl, n-Propyl, i-Propyl, n-Butyl oder t-Butyl, Alkenylrest, der geradkettig oder verzweigt sein kann, mit 2 bis 20 C-Atomen, Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, Etherrest, der geradkettig oder verzweigt sein kann, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl oder Thioetherrest, der geradkettig oder verzweigt sein kann, mit 2 bis 20 C-Atomen, beispielsweise Methylsulfanylmethyl, Ethylsulfanylmethyl, 2-Ethylsulfanylethyl oder 3-Methylsulfanylpropyl sein kann.

Bei einer weiteren bevorzugten Ausführungsform wird der Rest der Formel (4a) aus der Gruppe, die aus Resten der Formeln (8a), (8b) und (8c): 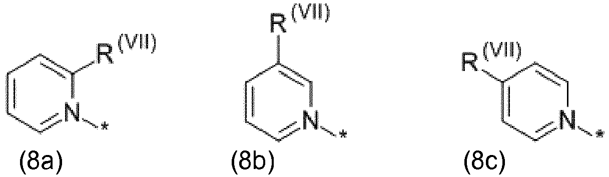 besteht, ausgewählt, wobei R^{(VII)} jeweils Wasserstoff, ein Arylrest mit 5 bis 20 C-Atomen, beispielsweise Phenyl oder Benzyl, ein Alkylrest, der geradkettig oder verzweigt sein kann, mit 1 bis 20 C-Atomen, beispielsweise Methyl, Ethyl, n-Propyl, i-Propyl, n-Butyl oder t-Butyl, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, ein Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, oder ein Thioetherrest, die geradkettig oder verzweigt sein kann, mit 2 bis 20 C-Atomen, beispielsweise Methylsulfanylmethyl, Ethylsulfanylmethyl, 2-Ethylsulfanylethyl oder 3-Methylsulfanylpropyl, sein kann.

Bei einer weiteren bevorzugten Ausführungsform ist der Rest der Formel (4b) 1-Methylpyrrolidin-1-ium-1-yl, 1-Methylpiperazin-1-ium-1-yl oder 4-Methylmorpholin-4-ium-4-yl.

Bei einer weiteren bevorzugten Ausführungsform werden die Reste R^{(I)}, R^{(II)}, R^{(III)}, R^{(IV)} und R^{(V)} unabhängig voneinander aus der Gruppe, die aus Wasserstoff und Alkylgruppen der allgemeinen Formel -(CH₂)ₙ-CH₃, wobei n eine ganze Zahl von 0 bis 19, vorzugsweise von 1 bis 17, ist, besteht, ausgewählt.

Bei einer weiteren bevorzugten Ausführungsform werden die Reste R^{(I)}, R^{(II)}, R^{(III)}, R^{(IV)} und R^{(V)} unabhängig voneinander ausgewählt aus der Gruppe, die aus Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl besteht, ausgewählt.

Bei einer besonders bevorzugten Ausführungsform werden die Reste R^{(I)}, R^{(II)}, R^{(III)}, R^{(IV)} und R^{(V)} unabhängig voneinander aus der Gruppe, die aus Methyl, Ethyl, Prop-1-yl, But-1-yl, Pent-1-yl, Hex-1-yl, Hept-1-und Oct-1-yl besteht, ausgewählt.

Bei einer weiteren bevorzugten Ausführungsform sind die Reste R^{(I)}, R^{(II)}, R^{(III)}, R^{(IV)} und R^{(V)} unabhängig voneinander Wasserstoff oder der Rest mit der Formel (10): 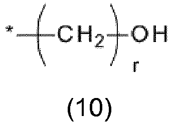 besteht, wobei r jeweils eine ganze Zahl von 1 bis 20, vorzugsweise von 1 bis 8, weiter bevorzugt von 1 bis 4, bedeutet, ausgewählt.

Bei einer weiteren bevorzugten Ausführungsform des erfindungsgemäßen 10H-Benzo[g]pteridin-2,4-dion-Derivats der Formel (1) wird der organischer Rest der allgemeinen Formel -(C(D)(E))ₕ-X aus der Gruppe, die aus den Resten der Formel (12a) bis (15b) besteht, 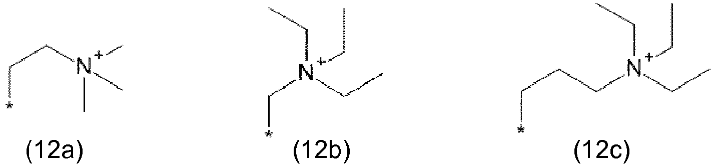 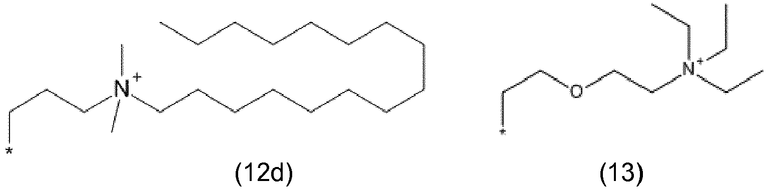 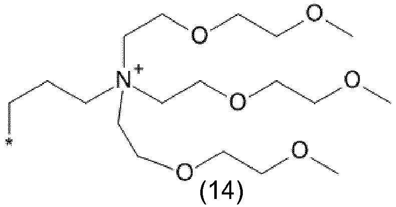 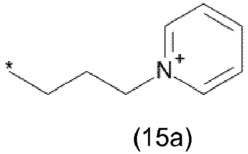 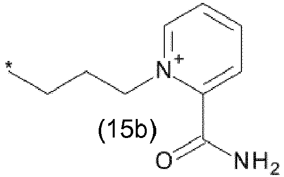 ausgewählt.

Bei einer weiter bevorzugten Ausführungsform sind die Reste R1 und R4, die jeweils unabhängig voneinander gleich oder voneinander verschieden sein können, Wasserstoff oder Methyl und nur 1 Rest R2, R3, R5 oder R6 ist ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist.

Bei einer weiteren bevorzugten Ausführungsform wird das erfindungsgemäße 10H-Benzo[g]pteridin-2,4-dion-Derivats der Formel (1) aus der Gruppe, die aus den Verbindung mit den Formeln (30) bis (43) besteht, ausgewählt:

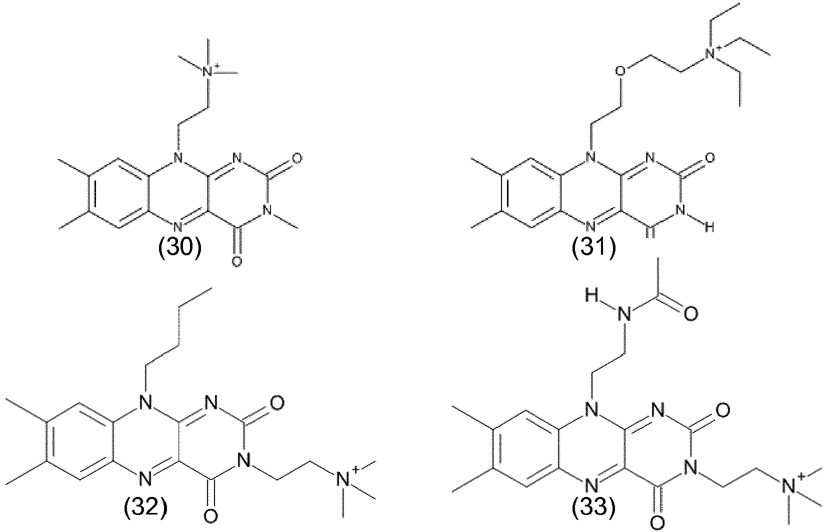

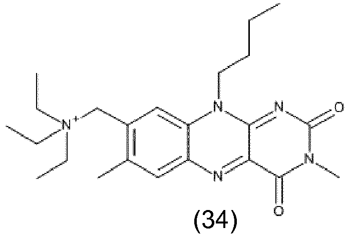

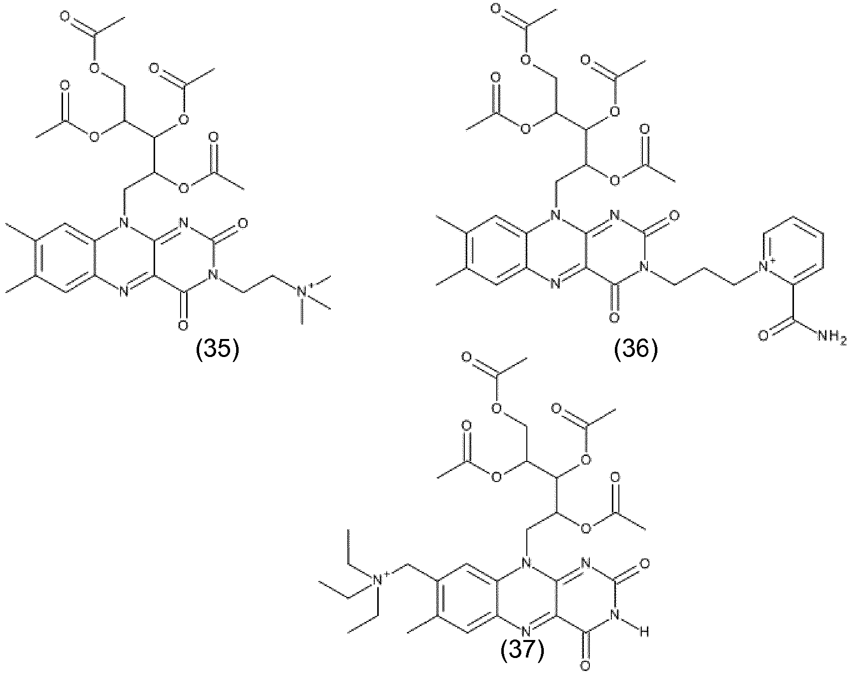

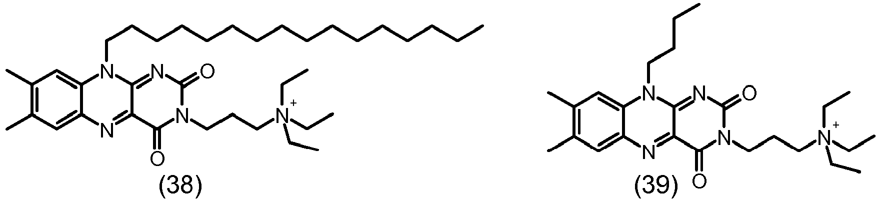

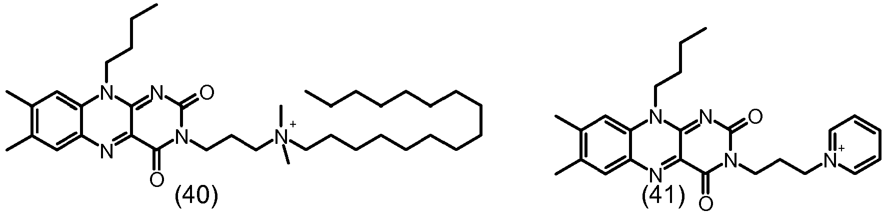

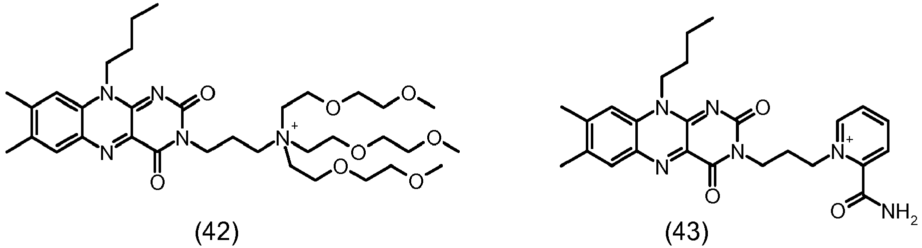

Beim erfindungsgemäßen 10H-Benzo[g]pteridin-2,4-dion-Derivat der Formel (1) können vorgenannte Aldehydreste, Ketonreste, Carbonsäurereste, Carbonsäureamidreste, Thioesterreste, Cycloalkylreste, Cycloalkenylreste, Alkylreste und Akenylreste geradkettig oder verzweigt, vorzugsweise geradkettig, sein und sowohl unsubstituiert oder mit wenigstens einem Rest, der Halogen, vorzugsweise Chlor, Brom, Iod oder Fluor, Thiol, Nitro, Hydroxy, Sulfanyl, Alkyloxy, vorzugsweise Methoxy, Ethoxy, n-Propyloxy, i-Propyloxy, n-Butyloxy oder n-Pentyloxy, Alkylsulfanyl, vorzugsweise Methylsulfanyl, Ethylsulfanyl, n-Propylsulfanyl, i-Propylsulfanyl, n-Butylsulfanyl oder n-Pentylsulfanyl oder Alkanoyloxy, vorzugsweise Formyloxy, Acetoxy oder n-Propanoyloxy ist, substituiert sein.

Bei einer weiter bevorzugten Ausführungsform werden die vorgenannte Alkylreste jeweils aus der Gruppe, die aus Methyl, Ethyl, n-Propyl, n-Butyl, n-Pentyl, n-Hexyl, n-Heptyl und n-Octyl besteht, ausgewählt.

Bei einer weiter bevorzugten Ausführungsform können vorgenannte Alkylreste nichtcyclische Polyolreste der allgemeinen Formel -CH₂(CH(OH))_{g}CH₂OH sein, wobei g eine ganze Zahl von 1 bis 10, vorzugsweise 1 bis 4, ist. Weiter bevorzugt werden die nichtcyclischen Polyolreste aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt.

Beim erfindungsgemäßen 10H-Benzo[g]pteridin-2,4-dion-Derivats der Formel (1) können vorgenannte Cycloalkylreste und Cycloalkenylreste Sauerstoff- und/oder Schwefelatome, als Ringatome aufweisen und sowohl unsubstituiert oder mit wenigstens einem Rest, der aus Hydroxyl, Sulfanyl, Alkyloxy, vorzugsweise Methoxy, Ethoxy, n-Propyloxy, i-Propyloxy, n-Butyloxy oder n-Pentyloxy, Alkylsulfanyl, vorzugsweise Methylsulfanyl, Ethylsulfanyl, n-Propylsulfanyl, i-Propylsulfanyl, n-Butylsulfanyl oder n-Pentylsulfanyl oder Alkanoyloxy, vorzugsweise Formyloxy, Acetoxy oder n-Propanoyloxy ist, substituiert sein.

Bei einer weiter bevorzugten Ausführungsform werden die vorgenannten Cycloalkylreste und Cycloalkenylreste, die Sauerstoff- und/oder Schwefelatome, als Ringatome aufweisen, jeweils aus der Gruppe, die aus Tetrahydrofuranyl, Tetrahydrothiophenyl, Tetrahydropyranyl, Tetrahydrothiopyranyl, Dioxolanyl und Dioxanyl besteht, ausgewählt.

Bei einer bevorzugten Ausführungsform des erfindungsgemäßen 10H-Benzo[g]pteridin-2,4-dion-Derivats der Formel (1) weisen die Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf.

Bei einer bevorzugten Ausführungsform des erfindungsgemäßen 10H-Benzo[g]pteridin-2,4-dion-Derivats der Formel (1) werden die vorgenannten Arylreste aus der Gruppe, die aus Phenyl, Benzyl, Naphthyl, Anthracenyl, Phenanthrenyl und Pyrenyl besteht, ausgewählt.

Bei einer bevorzugten Ausführungsform des erfindungsgemäßen 10H-Benzo[g]pteridin-2,4-dion-Derivats der Formel (1) weisen die vorgenannten Alkenylreste 2 bis 17 C-Atome, weiter bevorzugt 2 bis 13 C-Atome, weiter bevorzugt 2 bis 9 C-Atome, weiter bevorzugt 2 bis 5 C-Atome, auf. Bei einer weiter bevorzugten Ausführungsform werden die vorgenannten Alkenylreste aus der Gruppe, die aus Ethenyl, n-Propenyl und n-Butenyl besteht, ausgewählt.

Bei einer weiteren bevorzugten Ausführungsform des erfindungsgemäßen 10H-Benzo[g]pteridin-2,4-dion-Derivats der Formel (1) weisen die vorgenannten Aldehyde 1 bis 17 C-Atome, weiter bevorzugt 1 bis 13 C-Atome, weiter bevorzugt 1 bis 9 C-Atome, weiter bevorzugt 1 bis 5 C-Atome, auf. Bei einer weiter bevorzugten Ausführungsform werden die vorgenannten Aldehyde aus der Gruppe, die aus Methanal-1-yl (Formyl), Ethanal-1-yl (2-Oxoethyl), n-Propanal-1-yl (3-Oxopropyl) und n-Butanal-1-yl (4-Oxobutyl) besteht, ausgewählt.

Bei einer weiteren bevorzugten Ausführungsform des erfindungsgemäßen 10H-Benzo[g]pteridin-2,4-dion-Derivats der Formel (1) weisen die vorgenannten Ketone 2 bis 17 C-Atome, weiter bevorzugt 3 bis 14 C-Atome, weiter bevorzugt 3 bis 9 C-Atome, auf. Bei einer weiter bevorzugten Ausführungsform werden die vorgenannte Ketone aus der Gruppe, die aus Dimethylketyl, Methyl-Ethyl-ketyl, Ethyl-Methyl-ketyl, Diethylketyl, Methyl-Propyl-ketyl, Ethyl-Propyl-ketyl, Propyl-Methyl-ketyl, Propyl-Ethyl-ketyl und Dipropyl-ketyl, das geradkettig oder verzweigt sein kann, besteht, ausgewählt.

Bei einer weiter bevorzugten Ausführungsform können vorgenannte Aldehydreste und/oder Ketonreste Monosaccharidreste, vorzugsweise Pentose- oder Ketosereste, sein.

Vorzugsweise haben geeignete Monosaccharidreste 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt leiten sich geeignete Monosaccharidreste von Monosacchariden ab, die aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt werden.

Bei einer weiteren bevorzugten Ausführungsform des erfindungsgemäßen 10H-Benzo[g]pteridin-2,4-dion-Derivats der Formel (1) weisen die vorgenannten Carbonsäureester 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureester aus der Gruppe, die aus Ethylester, n-Propylester, i-Propylester, n-Butylester, sec.-Butylester, tert.-Butylester und Benzylester besteht, ausgewählt.

Bei einer weiteren bevorzugten Ausführungsform des erfindungsgemäßen 10H-Benzo[g]pteridin-2,4-dion-Derivats der Formel (1) weisen die vorgenannten Carbonsäureamide 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureamide aus der Gruppe, die aus Amid, N-Methylamid, N-Ethylamid, N-(n-Propyl)amid, N-(i-Propyl)amid, N-(n-Butyl)amid, N-(sec.-Butyl)amid, N-(tert.-Butyl)amid, N-Phenylamid, N-Benzylamid, N,N-Dimethylamid, N-Methyl-N-Ethyl-amid, N,N-Diethylamid, N-Methyl-N-(n-Propyl)amid, N-Methyl-N-(i-Propyl)amid, N-Methyl-N-(n-Butyl)amid, N-Methyl-N-(sec.-Butyl)amid, N-Methyl-N-(tert.-Butyl)amid, N-Ethyl-N-(n-Propyl)amid, N-Ethyl-N-(i-Propyl)amid, N-Ethyl-N-(n-Butyl)amid, N-Ethyl-N-(sec.-Butyl)amid, N-Ethyl-N-(tert.-Butyl)amid, N-(n-Propyl)-N-(n-Propyl)amid, N-(n-Propyl)-N-(i-Propyl)amid, N-(n-Propyl)-N-(n-Butyl)amid, N-(n-Propyl)-N-(sec.-Butyl)amid, N-(n-Propyl)-N-(tert.-Butyl)amid, N-(i-Propyl)-N-(n-Propyl)amid, N-(i-Propyl)-N-(i-Propyl)amid, N-(i-Propyl)-N-(n-Butyl)amid, N-(i-Propyl)-N-(sec.-Butyl)amid, N-(i-Propyl)-N-(tert.-Butyl)amid, N-(n-Butyl)-N-(n-Propyl)amid, N-(n-Butyl)-N-(i-Propyl)amid, N-(n-Butyl)-N-(n-Butyl)amid, N-(n-Butyl)-N-(sec.-Butyl)amid, N-(n-Butyl)-N-(tert.-Butyl)amid, N-(sec.-Butyl)-N-(n-Propyl)amid, N-(sec.-Butyl)-N-(i-Propyl)amid, N-(sec.-Butyl)-N-(n-Butyl)amid, N-(sec.-Butyl)-N-(sec.-Butyl)amid, N-(sec.-Butyl)-N-(tert.-Butyl)amid, N-(tert.-Butyl)-N-(n-Propyl)amid, N-(tert.-Butyl)-N-(i-Propyl)amid, N-(tert.-Butyl)-N-(n-Butyl)amid, N-(tert.-Butyl)-N-(sec.-Butyl)amid, N-(tert.-Butyl)-N-(tert.-Butyl)amid, N,N-Diphenylamid, N,N-Dibenzylamid, N-Phenyl-N-Benzylamid, N-Methyl-N-Phenylamid, N-Methyl-N-Benzylamid, N-Ethyl-N-Phenylamid, N-Ethyl-N-Benzylamid, N-Phenyl-N-(n-Propyl)amid, N-Phenyl-N-(i-Propyl)amid, N-Phenyl-N-(n-Butyl)amid, N-Phenyl-N-(sec.-Butyl)amid, N-Phenyl-N-(tert.-Butyl)amid, N-Benzyl-N-(n-Propyl)amid, N-Benzyl-N-(i-Propyl)amid, N-Benzyl-N-(n-Butyl)amid, N-Benzyl-N-(sec.-Butyl)amid und N-Benzyl-N-(tert.-Butyl)amid besteht, ausgewählt.

Bei einer weiteren bevorzugten Ausführungsform des erfindungsgemäßen 10H-Benzo[g]pteridin-2,4-dion-Derivats der Formel (1) wird der vorgenannten Heteroarylrest, der kein Stickstoffatom enthält, mit 4 bis 20 C-Atomen aus der Gruppe, die aus Thiophenyl, Furanyl, Benzothiofuranyl und Benzofuranyl besteht, ausgewählt.

Bei einer weiteren bevorzugten Ausführungsform des erfindungsgemäßen 10H-Benzo[g]pteridin-2,4-dion-Derivats der Formel (1) weisen die vorgenannten Etherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Etherreste beispielsweise aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl, i-Propoxymethyl, tert.-Butyloxymethyl, Dioxa-3,6-heptyl und Benzyloxymethyl besteht, ausgewählt. Bei einer weiter bevorzugten Ausführungsform können die vorgenannten Etherreste einfache Etherreste, Oligoetherreste, Polyetherreste oder Mischungen davon sein.

Bei einer weiteren bevorzugten Ausführungsform des erfindungsgemäßen 10H-Benzo[g]pteridin-2,4-dion-Derivats der Formel (1) weisen die vorgenannten Thioetherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Thioetherreste beispielsweise aus der Gruppe, Methylsulfanylmethyl, Methylsulfanylethyl, 3-Methylsulfanyl-n-propyl, Ethylsulfanylmethyl, n-Propylsulfanylmethyl, 2-Ethylsulfanylethylsulfanylmethyl, 2-(2-ethylsulfanylethylsulfanyl)ethyl, 2-Methylsulfanylpropyl, tert.-Butylsulfanymethyl und Benzylsulfanymethyl besteht, ausgewählt.

Bei einer weiteren bevorzugten Ausführungsform weist das erfindungsgemäße 10H-Benzo[g]pteridin-2,4-dion-Derivats der Formel (1) ein Molekulargewicht von weniger als 1300 g/mol, vorzugsweise weniger als 990 g/mol, weiter bevorzugt weniger als 810 g/mol, weiter bevorzugt weniger als 690 g/mol, noch weiter bevorzugt weniger als 610 g/mol, noch weiter bevorzugt weniger als 600 g/mol, noch weiter bevorzugt weniger als 570 g/mol, auf.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1), sowohl die reinen Verbindungen als auch die Isomerengemische in jedem Verhältnis.

Bei einer weiteren bevorzugten Ausführungsform wird das erfindungsgemäße 10H-Benzo[g]pteridin-2,4-dion-Derivat der Formel (1) aus der Gruppe, die aus den Verbindungen mit den Formeln (30) bis (43) besteht, ausgewählt:

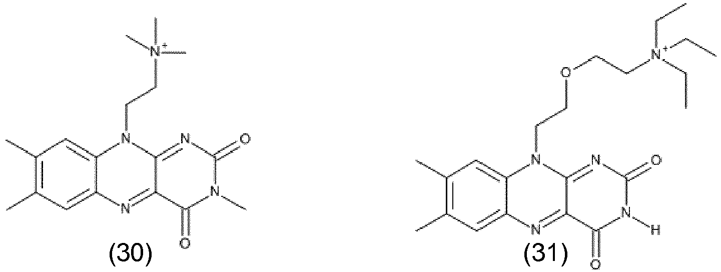

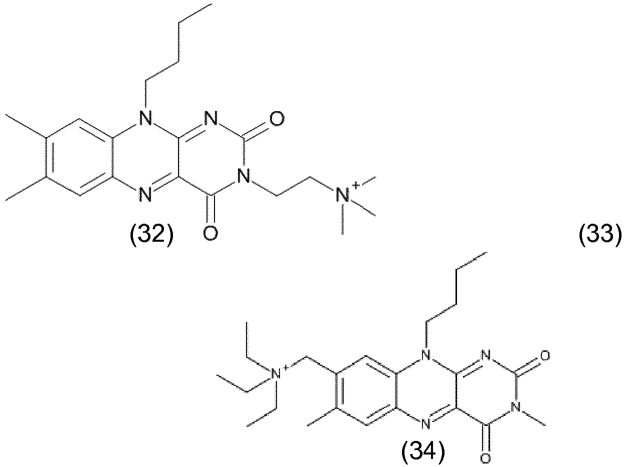

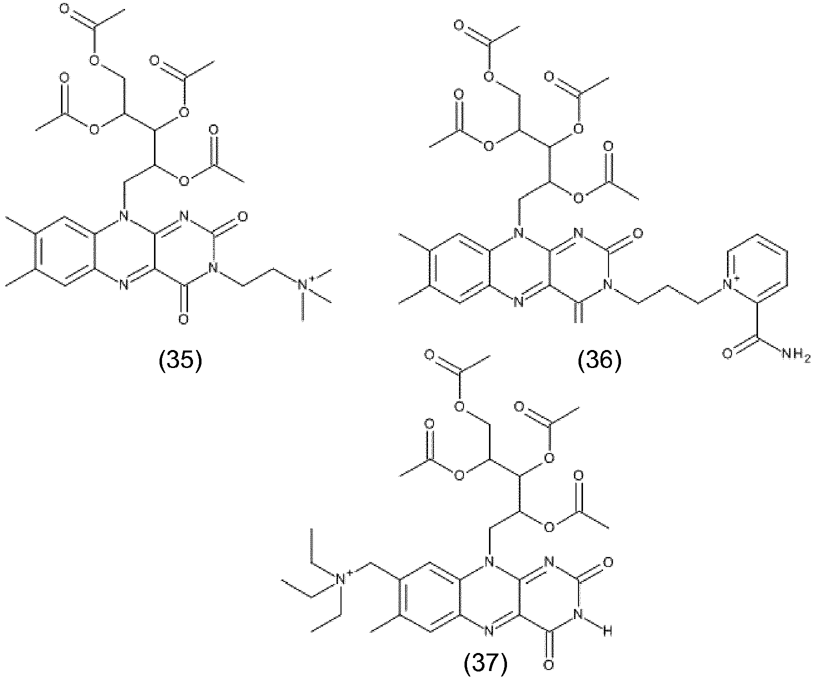

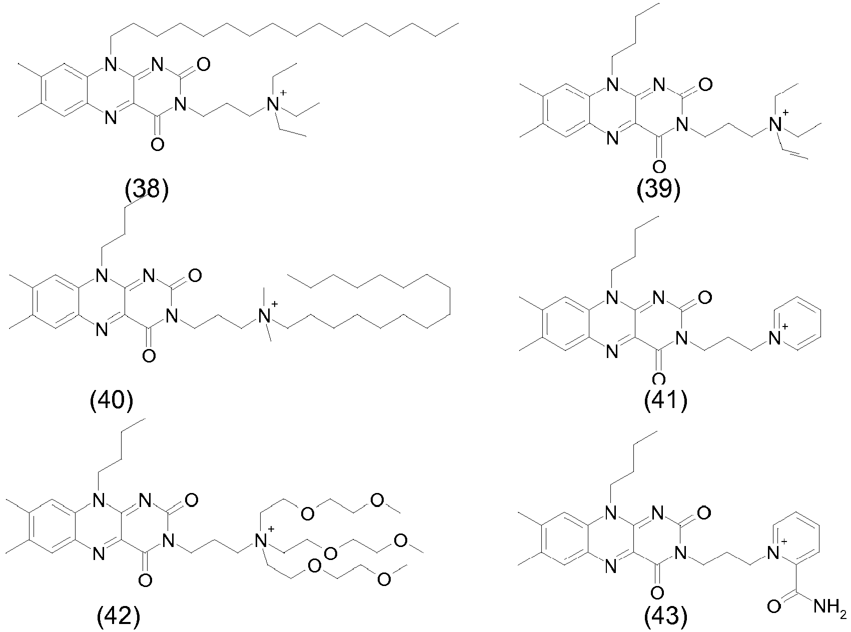

Eine Variante des Verfahrens zur Herstellung eines 10H-Benzo[g]pteridin-2,4-dion-Derivats der Formel (1), umfasst folgende Schritte:
(A) Reduzieren eines substituierten Nitroanilins der Formel (22) zu einem substituierten o-Phenylendiamin der Formel (23), vorzugsweise durch Wasserstoff und Palladium auf Aktivkohle oder mit Zinn(II)chlorid, 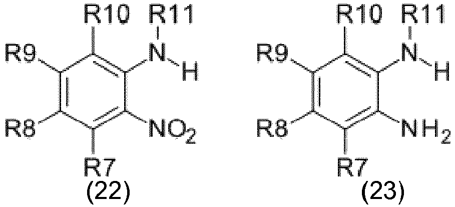 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und
   wobei der Rest R11 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Kondensieren des in Schritt (A) erhaltenen substituierten o-Phenylendiamin der Formel (23) mit Alloxan oder dessen Hydrat unter Erhalt einer Verbindung mit der Formel (24), optional in Gegenwart eines Katalysators, vorzugsweise Lewis-Säure oder Broenstedt-Säure, weiter bevorzugt Essigsäure in Gegenwart von Borsäure, 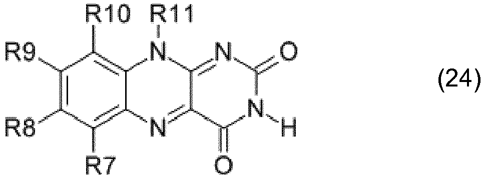
(C) Optional Umsetzen der in Schritt (B) erhaltenen Verbindung der Formel (24) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25): 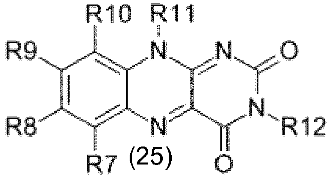
(D) Optional Umsetzen der in Schritt (B) erhaltenen Verbindung der Formel (24) oder der in Schritt (C) erhaltenen Verbindung der Formel (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel - (C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R1 bis R6 ein organischer Rest der allgemeinen Formel -(C(D)(E))h-X oder -(C(D)(E))ₖ-Aryl-(C(D)(E))ₗ-X ist und wobei h jeweils eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X jeweils ein organischer Rest mit nur einem quartären Stickstoffatom ist und Aryl jeweils einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Eine weitere Variante des Verfahrens zur Herstellung eines 10H-Benzo[g]pteridin-2,4-dion-Derivats der Formel (1), umfasst folgende Schritte:
(A) Herstellen eines substituierten Anilins mit der Formel (102a) oder (102b) durch (a) Peptidkupplung/Reduktion an ein Anilin mit der Formel (101) oder (b) reduktive Aminierung eines Anilins mit der Formel (101) mit Aldehyden oder (c) Pd-katalysierte Kupplung von einem Halogenid der Formel (104) an ein Amine der Formel R11-NH₂ oder (d) Pd-katalysierte Kupplung von einem Amin der Formel (100) an ein Halogenid der Formel R11-NH₂
   a) 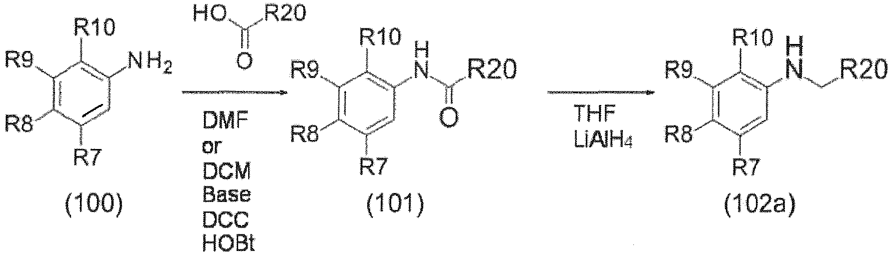
   b) 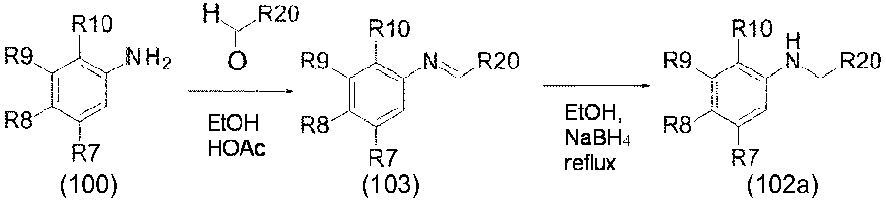
   c) 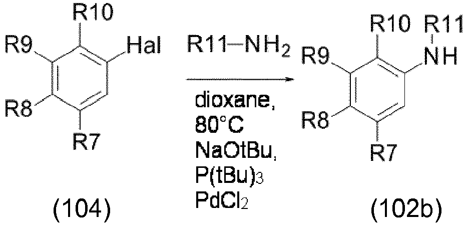
   d) 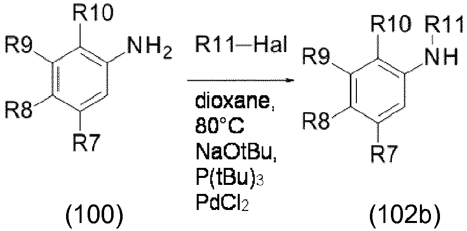 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und
      wobei der Rest R11 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
      wobei der Rest R20 Wasserstoff, Alkyl mit 1 bis 19 C-Atomen, Alkenyl mit 2 bis 19 C-Atomen, Ether mit 1 bis 19 C-Atomen, Thioether mit 1 bis 19 C-Atomen, Cycloalkyl mit 3 bis 19 C-Atomen, Cycloalkenyl mit 3 bis 19 C-Atomen, Aryl mit 5 bis 19 C-Atomen, Heteroaryl mit 4 bis 19 C-Atomen, das keine N-Atome enthält, oder ein organischen Rest der allgemeinen Formel -(C(D)(E))ₕ₋₁-OH oder -(C(D)(E))ₖ₋₁-Aryl-(C(D)(E))ₗ₋₁-OH, -(C(D)(E))ₕ₋₁-X oder -(C(D)(E))ₖ₋₁-Aryl-(C(D)(E))ₗ₋₁-X bedeutet, und
      wobei der Rest Hal Fluor, Chlor, Brom oder Iod bedeutet,
(B) Umsetzen des in Schritt (A) erhaltenen substituierten Anilins mit der Formel (102a) mit Violursäure unter Erhalt einer Verbindung der Formel (24z): 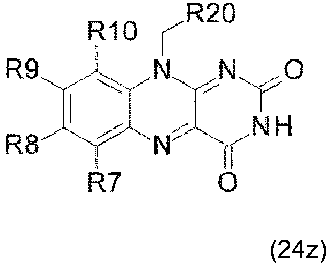 oder
   Umsetzen des in Schritt (A) erhaltenen substituierten Anilins mit der Formel (102b) mit Violursäure unter Erhalt einer Verbindung der Formel (24): 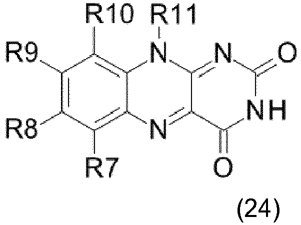
(C) Optional Umsetzen der in Schritt (B) erhaltenen Verbindung der Formel (24z) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25z): 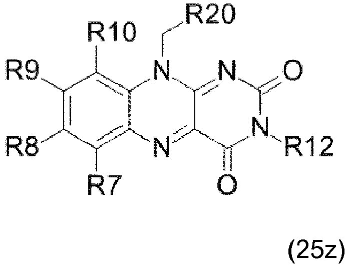 oder
   Umsetzen der in Schritt (B) erhaltenen Verbindung der Formel (24) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (25),: 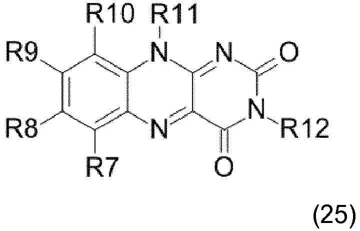
(D) Optional Umsetzen der in Schritt (B) erhaltenen Verbindung der Formel (24z) oder (24) oder der in Schritt (C) erhaltenen Verbindung der Formel (25z) oder (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 bzw. R7 bis R11 und R20 ein organischer Rest der allgemeinen Formel -(C(D)(E))ₕ-X oder-(C(D)(E))ₖ-Aryl-(C(D)(E))ₗ-X ist und wenn 1 Rest R7 bis R12 bzw. R7 bis R11 oder R20 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R1 bis R6 ein organischer Rest der allgemeinen Formel -(C(D)(E))ₕ-X oder -(C(D)(E))ₖ-Aryl-(C(D)(E))ₗ-X ist und wobei h jeweils eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X jeweils ein organischer Rest mit nur einem quartären Stickstoffatom ist und Aryl jeweils einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Eine weitere Variante des Verfahrens zur Herstellung eines 10H-Benzo[g]pteridin-2,4-dion-Derivats der Formel (1), umfasst folgende Schritte:
(A) Kondensieren eines Amins mit der Formel R11-NH₂ mit einem Chloruracil-Derivate der Formel (26), optional in Gegenwart eines Katalysators, vorzugsweise Lewis-Säure oder Broenstedt-Säure, unter Erhalt einer Verbindung mit der Formel (27): 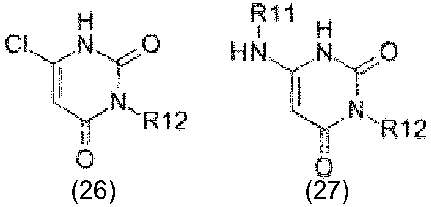 wobei jeder der Reste R11 oder R12 jeweils unabhängig voneinander gleich oder voneinander verschieden sein kann und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Umsetzen der in Schritt (A) erhaltenen Verbindung der Formel (27) mit einer Nitrosoverbindung der Formel (28) unter Erhalt einer Verbindung der Formel (25): 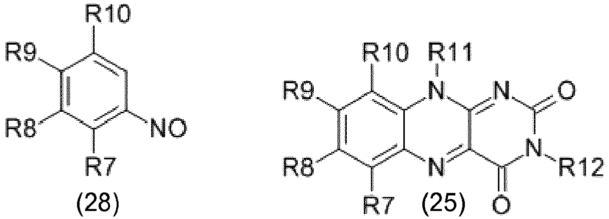 wobei jeder der Reste R7 bis R10 jeweils unabhängig voneinander gleich oder voneinander verschieden sein können und Wasserstoff, Halogen, Hydroxyl, Thiol, Nitro, Carboxy, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
(C) Optional Umsetzen der in Schritt (B) erhaltenen Verbindung der Formel (25) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel -(C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R7 bis R12 ein organischer Rest der allgemeinen Formel -(C(D)(E))ₕ-X oder -(C(D)(E))ₖ-Aryl-(C(D)(E))ₗ-X ist und wobei h eine ganze Zahl von 1 bis 20 und k, l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet.

Bei einer bevorzugten Ausführungsform der beiden Verfahrensvarianten ist keiner der Reste R7 bis R12 bzw. R7 bis R11 und R20 ein organischer Rest der allgemeinen Formel -(C(D)(E))ₕ-X oder -(C(D)(E))ₖ-Aryl-(C(D)(E))ₗ-X und 1 Rest R7 bis R10 ist Methyl wobei das Verfahren dann folgende Schritte umfassen kann,
(A) Radikalisches Halogenieren der Verbindung (24) oder (25) in Gegenwart eines Radikalstarters, vorzugsweise eines Peroxides oder einer AzoVerbindung, unter Erhalt einer Verbindung mit der Formel (24a-d) oder (25a-d): 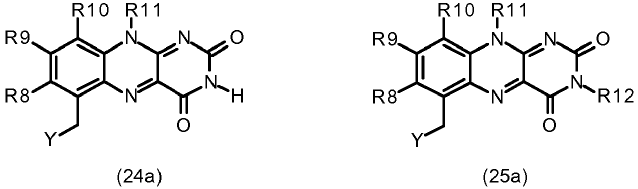 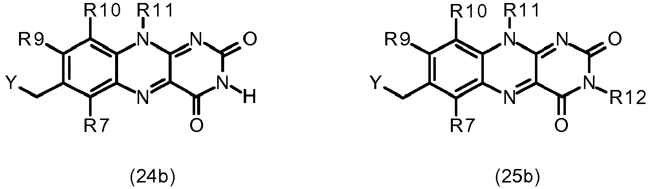 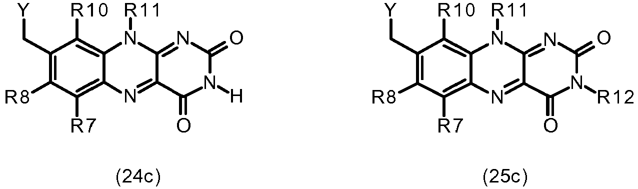 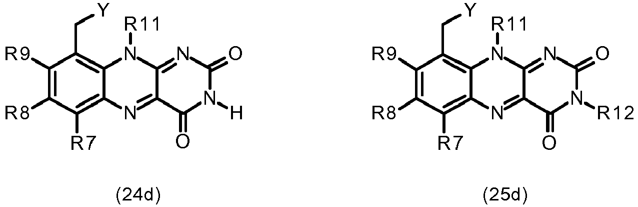 wobei der Rest Y Cl, Br oder I bedeutet, und
(B) Umsetzen der in Schritt (A) erhaltenen Verbindung mit der Formel (24a-d) oder (25a-d) mit einer organischen Verbindung, die wenigstens ein tertiäres Stickstoffatom enthält, unter Erhalt des erfindungsgemäßen 10H-Benzo[g]pteridin-2,4-dion-Derivats der Formel (1).

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Geeignete Methoden zur Entfernung der Amino-Schutzgruppe PG sind aus dem Stand der Technik bekannt. Beispielsweise kann Benzyloxycarbonyl (Cbz) durch katalytische Hydrierung unter hydrogenolytischer Spaltung der Benzyl-Heteroatom-Bindung mit anschließender Decarboxylierung der so entstehenden instabilen Carbaminsäure oder Behandlung mit Säuren wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Bei einer weiteren bevorzugten Ausführungsform finden die Schritte (B) und/oder (C) in Gegenwart eines oder mehrerer Lösungsmittel statt. Der Schritt (B) kann beispielsweise in Gegenwart von Dichlormethan (DCM) oder Tetrahydrofuran (THF) durchgeführt werden, vorzugsweise in Gegenwart einer Base, wie zum Beispiel Kaliumcarbonat. Alternativ kann der Schritt (B) beispielsweise in Gegenwart von Dimethylformamid (DMF) und Triphenylphosphin (PPh3) und/oder Kaliumiodid durchgeführt werden.

Der Schritt (C) kann beispielsweise in Gegenwart von Wasser/Dichlormethan oder Toluol/Tetrabutylammoiumiodid (TBAI) durchgeführt werden.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer systemischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Bei einer bevorzugten Ausführungsform wird wenigstens ein 10H-Benzo[g]pteridin-2,4-dion-Derivat der Formel (1) oder ein pharmakologisch verträgliches Salz und/oder Ester und/oder Komplex davon als Photosensibilisator bei der photodynamischen Inaktivierung von Mikroorganismen, vorzugsweise bei der photodynamischen Therapie, verwendet.

Das erfindungsgemäße 10H-Benzo[g]pteridin-2,4-dion-Derivat der Formel (1) weist nach Bestrahlung mit elektromagnetischer Strahlung geeigneter Wellenlänge und Energiedichte eine hohe Ausbeute an Singulett-Sauerstoff auf.

Vorzugsweise liegt die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 MW/cm², weiter bevorzugt von 1 mW/cm² bis 1kW/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Vorzugsweise wird die elektromagnetische Strahlung durch eine Strahlungsquelle erzeugt, die aus der Gruppe, die aus Sonne und künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass das erfindungsgemäße 10H-Benzo[g]pteridin-2,4-dion-Derivat der Formel (1) oder pharmakologisch verträgliche Salze und/oder Ester und/oder Komplexe davon vorzugsweise eine hohe Affinität zu Mikroorganismen aufweist.

Auf Grund der Affinität kann das erfindungsgemäße 10H-Benzo[g]pteridin-2,4-dion-Derivats der Formel (1) an Mikroorganismen effektiv binden und lokal ausreichend Singulett-Sauerstoff erzeugen, um die Mikroorganismen zu inaktivieren, vorzugsweise abzutöten.

Bei dieser bevorzugten Verwendung als Photosensibilisator wird wenigstens ein erfindungsgemäßes 10H-Benzo[g]pteridin-2,4-dion-Derivat der Formel (1) von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäßen 10H-Benzo[g]pteridin-2,4-dion-Derivates der Formel (1) an Mikroorganismen ebenfalls eine Anfärbung oder Lokalisierung von Mikroorganismen. Dadurch kann vorzugsweise auch der Verlauf der Inaktivierung von Mikroorganismen oder der Dekolonisation verfolgt werden.

Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der Inaktivierung von einzelligen oder mehrzelligen Mikroorganismen, die vorzugsweise aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden, verwendet.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon, vorzugsweise in einer pharmazeutischen Zubereitung, bei der Desinfektion und/oder Dekolonisierung von Haut- oder Weichteiloberflächen verwendet, wobei vorzugsweise die Hautintegrität erhalten bleibt.

Bei einer weiteren bevorzugten Ausführungsform ist wenigstens ein erfindungsgemäßes 10H-Benzo[g]pteridin-2,4-dion-Derivat der Formel (1) oder ein pharmakologisch verträgliches Salz und/oder Ester und/oder Komplex davon in einer pharmazeutischen Zubereitung zur topischen, vorzugsweise nasalen, oralen, analen, vaginalen oder dermalen, Applikation vorhanden.

Unter topischer Applikation wird auch die Anwendung am oder im Ohr, vorzugsweise Außenohr, verstanden. Das Außenohr umfasst den Ohrknorpel, die Ohrmuschel, das Ohrläppchen und den äußeren Gehörgang oder auch Ohrkanal und die Außenseite des Trommelfells.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Wundheilung, beispielsweise bei Heilungsstörungen nach chirurgischen Interventionen, verwendet.

Vorzugsweise wird wenigstens ein erfindungsgemäßes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der Desinfektion und/oder Reduktion der Keimzahl in infizierten Wunden verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre verwendet.

Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Bei einer bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon als Photosensibilisator bei der photodynamischen Inaktivierung von Mikroorganismen in der Mundhöhle verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der Reinigung von Zähnen, Zahnersatz und/oder Zahnspangen verwendet.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der nasalen Dekolonisierung von Mikroorganismen verwendet.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d.h. Entfernung der Mikroorganismen, in der Regel auch die Kolonisation an anderen Körperstellen.

Des Weiteren betrifft die vorliegende Erfindung eine pharmazeutische Zusammensetzung enthaltend wenigstens ein erfindungsgemäßes 10H-Benzo[g]pteridin-2,4-dion-Derivat der Formel (1) oder ein pharmakologisch verträgliches Salz und/oder Ester und/oder Komplex davon.

Vorzugsweise wird die pharmazeutische Zusammensetzung durch Mischen von mindestens einer Verbindung der Formel (1) oder ein pharmakologisch verträgliches Salz und/oder Ester und/oder Komplex davon mit einem oder mehreren physiologisch annehmbaren Hilfsstoff(en) hergestellt und in eine geeignete Darreichungsform gebracht.

Eine geeignete Darreichungsformen der erfindungsgemäßen pharmazeutischen Zusammensetzung wird vorzugsweise aus der Gruppe, die aus Salbe, Creme, Gel, Lotion, Schüttelmixtur, Lösung, beispielsweise in Tropfen- oder Sprayform, Puder, Mikrokapsel und Paste besteht, ausgewählt.

Die erfindungsgemäße pharmazeutische Zusammensetzung kann topisch, vorzugsweise nasal, oral, anal, vaginal oder dermal, angewendet werden.

Als physiologisch annehmbare Hilfsstoffe kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Bei einer weiteren bevorzugten Ausführungsform enthält die pharmazeutische Zusammensetzung eine effektive Menge wenigstens eines erfindungsgemäßen 10H-Benzo[g]pteridin-2,4-dion-Derivates der Formel (1) oder eines pharmakologisch verträglichen Salzes und/oder Esters und/oder Komplexes davon, wobei die effektive Menge von 0,01 µg bis 1000 µg pro Gramm der Zusammensetzung, vorzugsweise von 0,1 µg bis 500 µg pro Gramm der Zusammensetzung, umfasst.

Bei einer bevorzugten Ausführungsform der Erfindung umfasst die pharmazeutische Zusammensetzung wenigstens ein erfindungsgemäßes 10H-Benzo[g]pteridin-2,4-dion-Derivat der Formel (1) oder ein pharmakologisch verträgliches Salz und/oder Ester und/oder Komplex davon und wenigstens einen weiteren pharmazeutisch aktiven Bestandteil.

Vorzugsweise wird der wenigstens eine weitere pharmazeutisch aktive Bestandteil aus der Gruppe, die aus Antibiotika, Antimikotika, Virustatika, Antihistaminika, Sympathomimetika, Antihämorrhagika, Emmolientia und Hautschutzmittel, Analgetika, Desinfektionsmittel, Immunsera und Immunglobuline, Antiparasitäre Substanzen, Insektizide, Repellenzien und Corticosteroide besteht, ausgewählt.

Bei einer weiteren bevorzugten Ausführungsform wird wenigstens ein erfindungsgemäßes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat der Formel (1) oder ein pharmakologisch verträgliches Salz und/oder Ester und/oder Komplex davon durch den Anwender selbst aufgebracht und, optional, nachfolgend mit einer geeigneten Strahlenquelle, die elektromagnetische Strahlung geeigneter Wellenlänge und Energiedichte erzeugt, bestrahlt.

Bei einer weiter bevorzugten Verwendung des wenigstens einen erfindungsgemäßen 10H-Benzo[g]pteridin-2,4-dion-Derivates der Formel (1) oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäßes 10H-Benzo[g]pteridin-2,4-dion-Derivat der Formel (1) oder ein pharmakologisch verträgliches Salz und/oder Ester und/oder Komplex davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäßen 10H-Benzo[g]pteridin-2,4-dion-Derivat der Formel (1) oder eines pharmakologisch verträgliches Salzes und/oder Esters und/oder Komplexes davon weitere Bestandteile, vorzugsweise Absorbtionsmittel, beispielsweise Calciumalginat oder Polyurethanschaum, oder weitere pharmazeutisch wirksame Substanzen.

Die Erfindung wird nachfolgend durch Figuren und Beispiele erläutert, ohne hierauf beschränkt zu sein.

### Beispiel 1) Herstellung verschiedener 10H-Benzo[g]pteridin-2,4-dion-Derivate.

### Überblick über die Synthesen

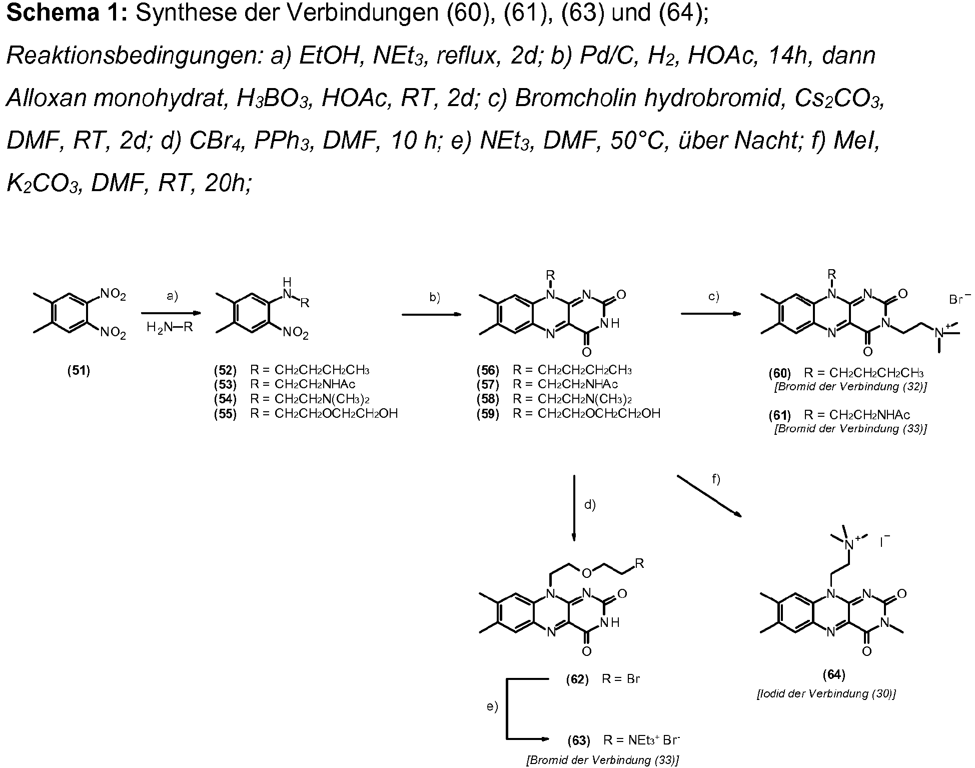

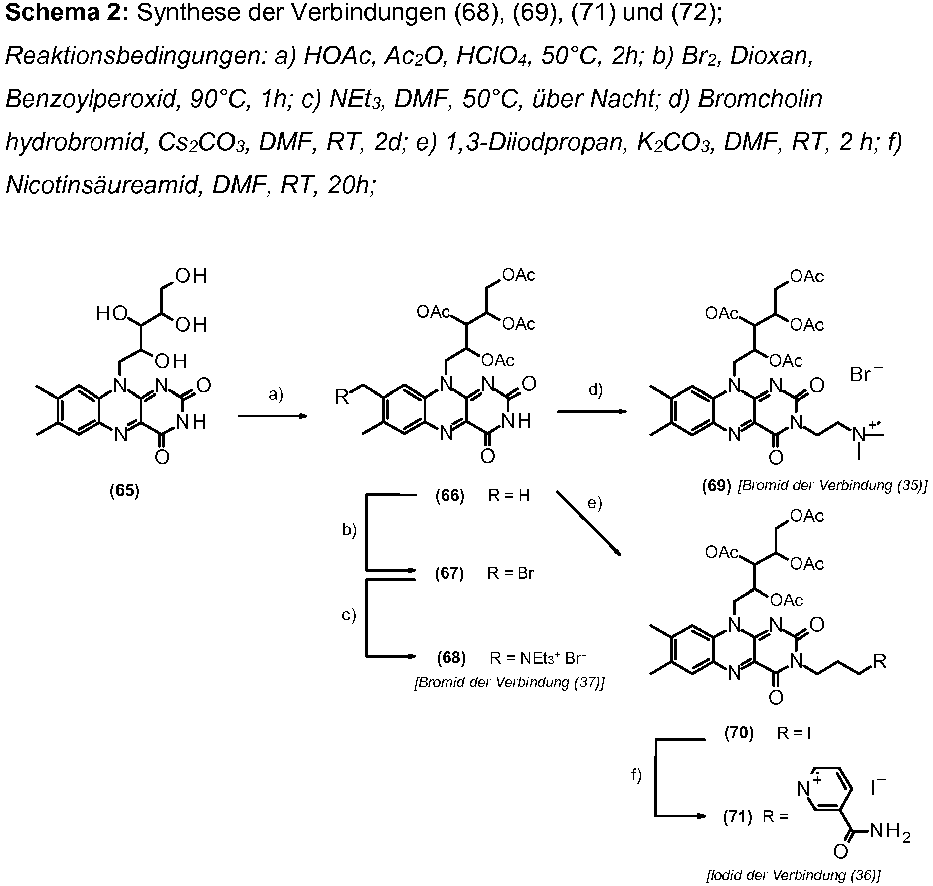

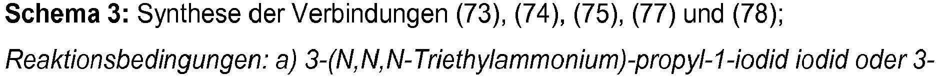

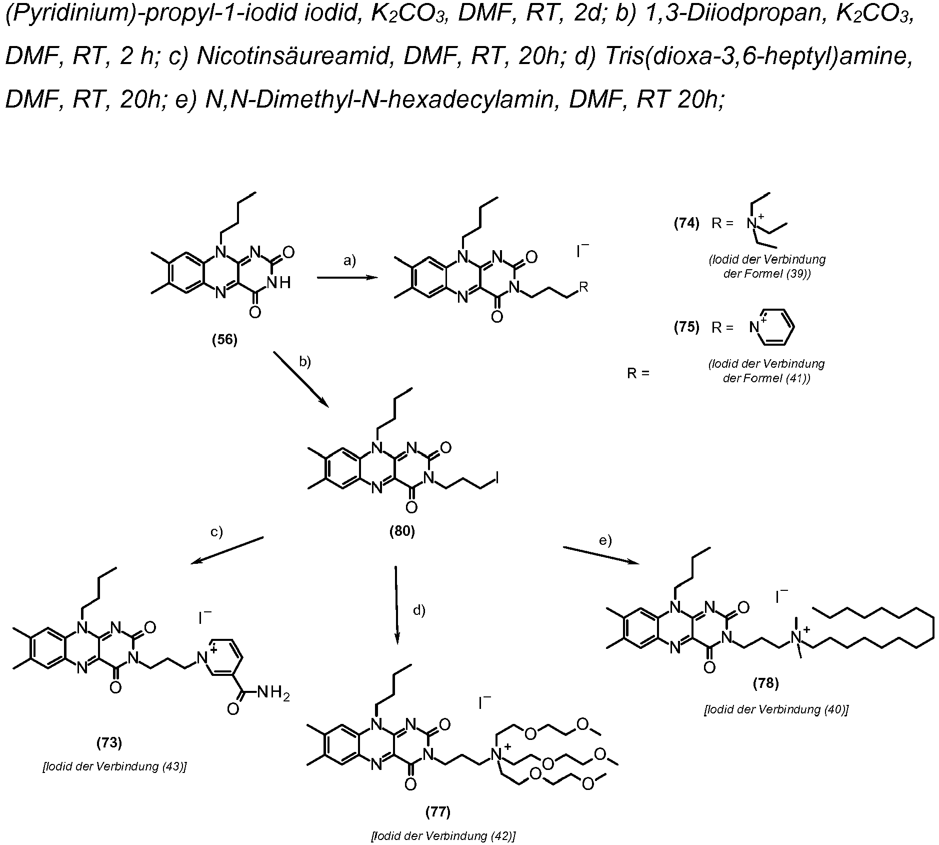

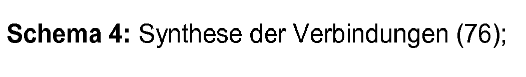

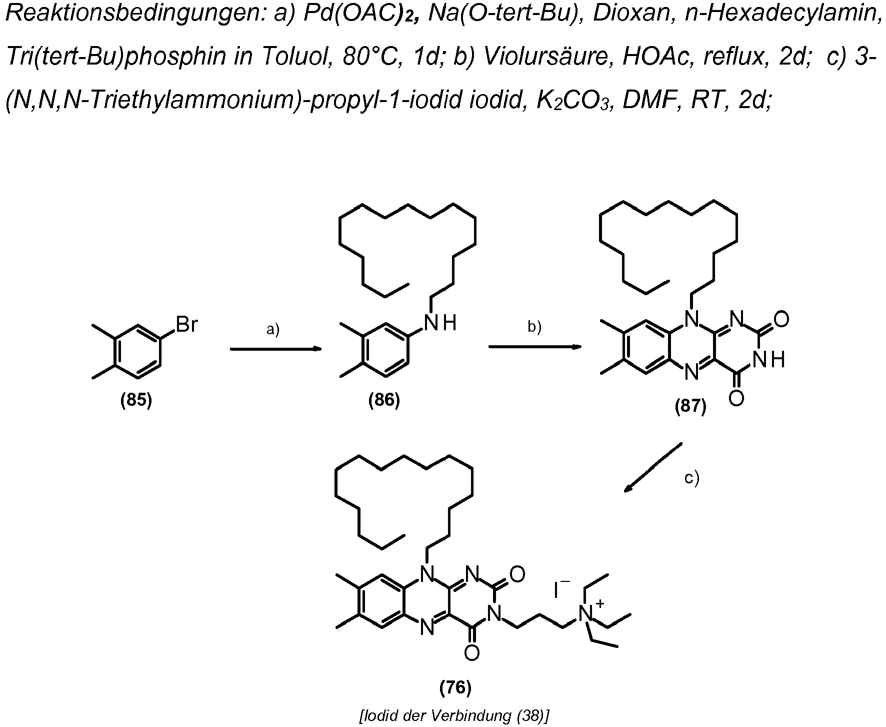

Alle verwendeten Chemikalien wurden von gängigen Anbietern käuflich erworben (TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm × 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt.

NMR-Spektren wurden auf einem Bruker-Spektrometer Avance 300 (300 MHz [¹H-NMR], 75 MHz [¹³C-NMR]) (Bruker Corporation, Billerica, US) gemessen.

Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für ¹H-NMR, 0.1 ppm für ¹³C-NMR und 0.1 Hz für Kopplungskonstanten. Das verwendete Lösemittel ist jeweils für jedes Spektrum aufgeführt.

Die IR-Spektren wurden an einem Biorad Spektrometer Excalibur FTS 3000 (Bio-Rad Laboratories GmbH, München, DE) aufgenommen.

ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als Ionisierungsgas für FAB.

Schmelzpunkte wurden mit Hilfe des Schmelzpunktmessgerätes Büchi SMP-20 (Büchi Labortechnik GmbH, Essen, DE) unter Verwendung einer Glaskapillare bestimmt.

Sämtliche UV/Vis-Spektren wurden mit einem Varian Cary 50 Bio UV/VIS Spektrometer, Fluoreszenzspektren mit einem Varian Cary Eclipse Spektrometer aufgenommen.

Die Lösungsmittel für Absorbtions- und Emissionsmessungen wurden in spezieller spektroskopischer Reinheit von Acros oder Baker bzw. Uvasol von Merck gekauft. Milliporewasser (18 MΩ, Milli Q_{Plus}) wurde für alle Messungen verwendet.

1-N-Acetyl-aminoethylamin^{[i]}, 1,2-Dinitro-4,5-dimethyl-benzen (**51**)^{[ii]}, Butyl-(4,5-dimethyl-2-nitro-phenyl)-amin **(52)** und 10-Butyl-7,8-dimethyl-10H-benzo[g]pteridin-2,4-dion (**56**)^{[iii]}, N-[2-({[(tert-Butyl)oxy]carbonyl}amino)ethyl]-4,5-dimethyl-2-nitroanilin^{[iv]}, N-(3'-Oxabut-1'-yl)-4,5-dimethyl-2-nitroanilin und 10-(2'-Methoxyethyl)-7,8-dimethyl-10H-benzo[g]pteridine-2,4-dion (**65**)^{[v]}, Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester (**70**)^{[vi]}, Riboflavin tetraacetat (**66**)^{[vii]} und 8a-Bromo-tetraacetylriboflavin (**17**)^{[viii]}, wurden nach literaturbekannten Vorschriften hergestellt:
[i] M.Jasinski, G. Mloston, P. Mucha, A.Linden, H.Heimgartner, Helv. Chim. Acta 2007, 90, 1765 - 1779
[ii] a) A. Monge, J. A. Palop, A. López de Ceráin, V. Senador, F. J. Martinez-Crespo, Y. Sainz, S. Narro, E. Garcia, C. de Miguel, M. González, E. Hamilton, A. J. Barker, E. D. Clarke, D. T. Greenhow, J. Med. Chem. 1995, 38, 1786-1792; b) T. Sugaya, K. Nobuyuki, A. Sakaguchi, S. Tomioka, Synthesis 1995, 1257-1262; c) R. R. Holmes, R. P. Bayer, J. Am. Chem. Soc. 1960, 82, 3454-3456.
[iii] O.Wiest, Ch.B. Harrison, N.J. Saettel, R. Cibulka, M. Sax, B. König, J. Org. Chem. 2004, 69, 8183-8185
[iv] J. Butenandt, R. Epple, E.-U. Wallenborn, A.P.M. Eker, V. Gramlich, T. Carell, Chem. Eur. J. 2000, 6, No. 1, 62-72
[v] R. Epple, E.-U. Wallenborn, T. Carell, J. Am. Chem. Soc. 1997, 119, 7440-7451.
[vi] A. Barthel, L. Trieschmann, D. Ströhl, R. Kluge, G. Böhm, Rene Csuk, Arch. Pharm. Chem. Life Sci. 2009, 342, 445 - 452.
[vii] McCormick, D. B. J. Heterocycl. Chem. 1970, 7, 447-450.
[viii] M.C. Falk, P.G. Johnson, D.B. McCormick, Biochemistry, 1976, 15(3), 639-645

Bromocholin hydrobromid ist kommerziell mit einem Reinheitsgrad >98% erhältlich (TCI Deutschland GmbH, Eschborn, Deutschland) und wurde ohne weitere Reinigung verwendet.

### Allgemeine Vorschrift I): Umsetzung von 1,2-Dinitro-4,5-dimethyl-benzen zu substituierten 4,5-Dimethyl-2-Nitroanilinen

1,2-Dinitro-4,5-dimethyl-benzen **(51)** (3,92g, 20 mmol) und das entsprechende primäre Amin (100 mmol) weurden in trockenem Ethanol (100 mL) und frisch destilliertem Triethylamin (50 mL) 2 Tage bei 90°C Ölbadtemperatur unter Stickstoff refluxiert. Nach Abkühlen auf Raumtemperatur wurde das Lösungsmittelgemisch bei vermindertem Druck abgezogen und der Rückstand getrocknet.

**Tabelle 1: verwendete Amine**

| Beispiel | Zielverbindunq | primäres Amin |
|---|---|---|
| | (52) | H₂NCH₂CH₂CH₂CH₃ |
| la) | (53) | H2NCH2CH2NHAc |
| Ib) | (54) | H₂NCH₂CH₂N(CH₃)₂ |
| Ic) | (55) | H2NCH2CH2OCH2CH2OH |

### Ia) N'-(4,5-Dimethyl-2-nitro-phenyl)-N,N-acetyl-ethane-1,2-diamine (53):

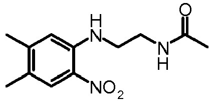

Das Rohprodukt wurde aus Ethanol umkristallisiert und es wurden 3,54 g rotorange Kristallnadeln erhalten (71 % der Theorie, 141 mmol).

**¹H-NMR** (300 MHz, CDCl₃): δ [ppm] = 2,03 (s, 3 H), 2,15 (s, 3 H), 2,25 (s, 3 H), 3.18 (t, 2 H, J = 6.0 Hz), 3.39 (t, 2 H, J = 5.8 Hz), 6.94 (s, 1 H), 7,02 (m, 1 H), 7.84 (s, 1 H), 8.08 (bs, 1 H); - **MS** (ESI-MS, H₂O/MeOH + 0.1 % TFA): m/z (%) = 252.1 (100, (MH⁺)); - **MG** = 251,29 g/mol - **MF** = C₁₂H₁₇N₃O₃

### Ib) N'-(4,5-Dimethyl-2-nitro-phenyl)-N,N-dimethyl-ethane-1,2-diamine (54):

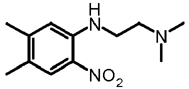

Das Rohprodukt wurde aus Ethanol umkristallisiert. Man erhält 2,99 g orange Kristallnadeln (63 % der Theorie, 126 mmol).

**¹H-NMR** (300 MHz, CDCl₃): δ [ppm] = 2,17 (s, 3 H), 2,26 (s, 3 H), 2,32 (s, 6 H), 2,71 (t, 2 H, J = 544 Hz), 3,32 (m, 2 H), 6,61 (s, 1 H), 7,91 (s, 1 H), 8,19 (bs, 1 H); - **MS** (CI-MS, NH₃): m/z (%) = 238.1 (100, (MH⁺)); - **MG** = 237,30 g/mol - **MF** = C₁₂H₁₉N₃O₂

### Ic) 2-[2-(4,5-Dimethyl-2-nitro-phenylamino)-ethoxyl-ethanol (55):

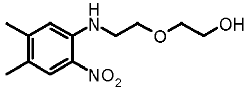

Das Rohprodukt wurde durch Säulenchromatographie an Kieselgel mit Ethylacetat / Petrolether 1:1 gereinigt unter Erhalt eines roten Öls, welches nach längerem Stehen zu einem orangen Feststoff erstarrt (4,31 g, 86 % der Theorie, 16,9 mmol).

**¹H-NMR** (300 MHz, CDCl₃): δ [ppm] = 2,02 (s, 3 H), 2,11 (s, 3 H), 2,98 (bs, 1 H), 3,37 (t, 2 H, J = 5,4 Hz), 3,54 (t, 2 H, J = 5,4 Hz), 3,69 (m, 4 H), 6,49 (s, 1 H), 7,70 (s, 1 H), 8,01 (m, 1 H) ; - **MS** (ESI-MS, H₂O/MeOH + 0.1 % TFA): m/z (%) = 255.1 (58, (MH⁺)), 509.3 (100, (2MH⁺)); - **MG** = 254,29 g/mol - **MF** = C₁₂H₁₈N₂O₄

### Allgemeine Vorschrift II): Umsetzung von substituierten 2-Nitroanilinen zu den entsprechenden monosubstituierten 10H-benzo[g]pteridin-2,4-dion-Derivaten

Das jeweils oben unter la) bis Ic) erhaltene 2-Nitroanilin **(53)** bis **(55)** (10 mmol) wurde in Essigsäure (100 mL) gelöst. Palladium auf Aktivkohle (100 mg, 10% Pd) wurde zugegeben und der Ansatz wurde 14 h bei Raumtemperatur im Autoklaven in Wasserstoffatmosphäre bei 20 bar gerührt. Die farblose Lösung wurde in einen Schlenckkolben mit Stickstoffatmosphäre gefiltert, Alloxan monohydrat (4,00 g, 25 mmol) und Borsäure (15,50 g, 250 mmol) wurden zugegeben. Der Kolben wurde mit Alufolie umwickelt und der Ansatz 2 Tage bei Raumtemperatur unter Stickstoff im Dunklen gerührt. Die orangegelbe Suspension wurde mit Wasser (200 mL) verdünnt und viermal mit Dichlormethan (je 150 mL) extrahiert. Die vereinigten organischen Phasen wurden mit Wasser (100 mL) gewaschen, über Magnesiumsulfat getrocknet und einrotiert.

### IIa) 10-(2-N-Acetyl-amino-ethyl)-7,8-dimethyl-10H-benzo[g]pteridine-2,4-dion (57)

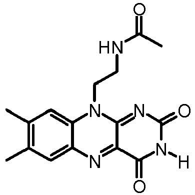

Der Rückstand wurde aus Essigsäure und Wasser (1:1) umkristallisiert. Man erhält das Produkt als orangen Feststoff (2,56 g, 7,82 mmol, 78 % der Theorie).

**¹H-NMR** (300 MHz, DMSO-d6): δ [ppm] = 2,03 (s, 3 H), 2,41 (s, 3 H), 2.54 (s, 3 H), 3,32 (t, 2 H, J = 5,4 Hz), 3,46 (t, 2 H, J = 5,4 Hz), 7.79 - 7,83 (s, 1 H), 11.32 (s, 1 H); - **MS** (ESI-MS, H₂O/MeOH + 0.1 % TFA): m/z (%) = 328.0 (100, (MH⁺)); - **MG** = 327,35 g/mol - **MF** = C₁₆H₁₇N₅O₃

### IIb) 10-(2-Dimethylamino-ethyl)-7,8-dimethyl-10H-benzo[g]pteridine-2,4-dion (58)

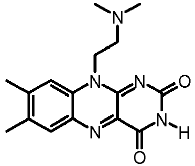

Der Rückstand wurde durch Säulenchromatographie an Kieselgel mit Ethylacetat / Methanol 20:1 → 5:2 gereinigt unter Erhalt eines orangen Feststoffs (2,22 g, 7,07 mmol, 71 % der Theorie).

**¹H-NMR** (300 MHz, DMSO-d6): δ [ppm] = 2.34 (s, 3 H), 2.43 (s, 3 H), 2,91 (s, 6 H), 3,39 (m, 2 H), 4,61 (m, 2 H), 7.52 (s, 1 H), 7.78 (s, 1 H), 11.38 (s, 1 H); - **MS** (ESI-MS, H₂O/MeOH + 0.1 % TFA): m/z (%) = 314.0 (100, (MH⁺)); - **MG** = 313,36 g/mol - **MF** = C₁₆H₁₉N₅O₂

### IIc) 10-[2-(2-Hydroxy-ethoxy)-ethyl]-7,8-dimethyl-10H-benzo[g]pteridine-2,4-dion (59)

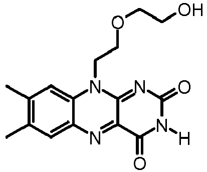

Der Rückstand wurde durch Säulenchromatographie an Kieselgel mit Ethylacetat / Methanol 20:1 → 5:2 gereinigt. Man erhält 1,98 g orangen Feststoff (6,0 mmol, 60 % der Theorie).

**¹H-NMR** (300 MHz, DMSO-d6): δ [ppm] = 2,42 (s, 3 H), 2,53 (s, 3 H), 3,28 (t, 2 H, J = 5,4 Hz), 3,49 (t, 2 H, J = 5,4 Hz), 3,66 (m, 4 H), 7.76 (s, 1 H), 7.88 (s, 1 H), 11.33 (s, 1 H); - **MS** (ESI-MS, H₂O/MeOH + 0.1 % TFA): m/z (%) = 331.1 (100, (MH⁺)), 661.4 (61, (2MH⁺)); - **MG** = 330,35 g/mol - **MF** = C₁₆H₁₈N₄O₄

### III) 10-[2-(2-Bromo-ethoxy)-ethyl]-7,8-dimethyl-10H-benzo[g]pteridine-2,4-dion (62)

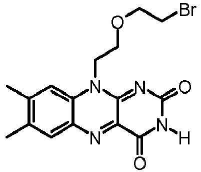

Das unter IIc) erhaltene 10-[2-(2-Hydroxy-ethoxy)-ethyl]-7,8-dimethyl-10H-benzo[g]pteridine-2,4-dion **(59)** (80 mg, 0.25 mmol) wurde zusammen mit Tetrabrommethan (100 mg, 0.3 mmol) in trockenem DMF (5 mL) gelöst. Triphenylphosphin (168 mg, 0.5 mmol) wurde bei 0°C in mehreren kleinen Portionen zugegeben. Der Ansatz wurde über Nacht bei Raumtemperatur gerührt und anschließend das Lösungsmittel bei vermindertem Druck abgezogen. Der Rückstand wurde durch präparative Dünnschichtchromatographie mit Essigsäureethylester / Methanol 25:1 gereinigt. Es wurden 72 mg eines gelben Feststoffs erhalten (0.186 mmol, 73% der Theorie).

**¹H-NMR** (300 MHz, DMSO-d6): δ [ppm] = 2,40 (s, 3 H), 2,51 (s, 3 H), 3,23 (t, 2 H, J = 5,4 Hz), 3,56 (t, 2 H, J = 5,4 Hz), 3,61 (m, 2 H), 3,87 (m, 2 H), 7.78 (s, 1 H), 7.93 (s, 1 H), 11.30 (s, 1 H); - **MS** (ESI-MS, H₂O/MeOH + 0.1 % TFA): m/z (%) = 393.1 & 395.1 (100, (MH⁺)); - **MF:** C₁₆H₁₇BrN₄O₃ - **FW:** 393.24 g/mol;

### IV) Trimethyl-[2-(3,7,8-trimethyl-2,4-dioxo-3,4-dihydro-2H-benzo[g]pteridin-10-yl)-ethyl]-ammonium iodid (64)

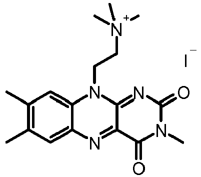

Das unter IIb) erhaltene Flavin **(58)** (331 mg, 1.0 mmol) wurde in trockenem DMF (20 mL) gelöst, Caesium carbonat (488 mg, 1.5 mmol) und Methyliodid (1.42 g, 10.0 mmol) wurden zugegeben und der Ansatz für 20 h bei Raumtemperatur im Dunklen gerührt. Die Suspension wurde mit Chloroform (100 mL) verdünnt und mit Wasser (30 mL) gewaschen. Die organische Phase wurde über Magnesiumsulfat getrocknet und die Lösungsmittel wurden bei vermindertem Druck abgezogen. Das Rohprodukt wurde durch präparative Dünnschichtchromatographie an Kieselgel (CHCl₃/MeOH-4:1) gereinigt.

Ausbeute: 263 mg eines orangen Feststoffes (0,56 mmol, 56 % der Theorie) R_{f} = 0.1 (CHCl₃:MeOH - 4:1)

**¹H-NMR** (300 MHz, DMSO-d6): δ [ppm] = 2.36 (s, 3 H), 2.42 (s, 3 H), 3,17 (bs, 9 H), 3,41 (m, 2 H), 3,48 (s, 3 H), 4,36 (m, 2 H), 7.56 (s, 1 H), 7.79 (s, 1 H); - **MS** (ESI-MS, H₂O/MeOH + 0.1 % TFA): m/z (%) = 342.1 (100, (M⁺)); - **MG** = 342.42 + 126.90 g/mol - **MF** = C₁₈H₂₄N₅O₂I

### V) {2-[2-(7,8-Dimethyl-2,4-dioxo-3,4-dihydro-2H-benzo[g]pteridin-10-yl-ethoxy]-ethyl-triethyl-ammonium bromid (63)

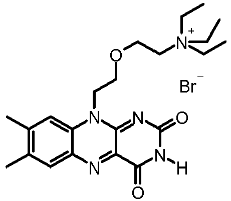

Das unter III) erhaltene Flavin **(62)** (393 mg, 1.0 mmol) wurde in trockenem DMF (20 mL) gelöst, Triethylamin (1.01 g, 10.0 mmol) wurde zugegeben und der Ansatz über Nacht bei 50°C im Dunklen gerührt. Die Suspension wurde mit Chloroform (100 mL) verdünnt und mit Wasser (30 mL) gewaschen. Die organische Phase wurde über Magnesiumsulfat getrocknet und die Lösungsmittel wurden bei vermindertem Druck abgezogen. Das Rohprodukt wurde durch präparative Dünnschichtchromatographie an Kieselgel (CHCl₃/MeOH - 4:1) gereinigt.

Ausbeute: 141 mg mikrokristalliner oranger Feststoff (0,284 mmol, 28 % der Theorie) R_{f} = 0.1 (CHCl₃:MeOH - 4:1)

**¹H-NMR** (300 MHz, DMSO-d6): δ [ppm] = 1,26 (t, 9 H, J = 7,4 Hz), 2,42 (s, 3 H), 2,53 (s, 3 H), 3,22 - 3,37 (m, 8 H), 3,46 (t, 2 H, J = 5,4 Hz), 3,68 (m, 4 H), 7.78 (s, 1 H), 7.91 (s, 1 H), 11.31 (s, 1 H); - **MS** (ESI-MS, H₂O/MeOH + 0.1 % TFA): m/z (%) = 414.1 (100, (M⁺)); - **MG** = 414.53 + 79.90 g/mol - **MF** = C₂₂H₃₂N₅O₃Br

### Allgemeine Vorschrift VI): Funktionalisierung von Flavinen in Position 3 durch Bromocholin hydrobromid

Das jeweils angegebene Flavin (1,0 mmol) wurde in trockenem DMF (20 mL) gelöst, Caesium carbonat (1,63 g, 5 mmol) und Bromocholin hydrobromid (0.5 g, 2.0 mmol) wurden zugegeben und der Ansatz für 1d bei Raumtemperatur im Dunklen gerührt. Es wurde erneut Bromocholin hydrobromid (0.5 g, 2.0 mmol) zugegeben und der Ansatz einen weiteren Tag bei Raumtemperatur im Dunklen gerührt. Das DMF wurde abgezogen und der Rückstand in Chloroform/Methanol 6:1 (50 mL) suspendiert. Die Suspension wurde gefiltert und das Filtrat wurde bis zur Trockene eingeengt. Das Rohprodukt wurde durch präparative Dünnschichtchromatographie an Kieselgel (CHCl₃/MeOH - 4:1) und durch Umkristallisation aus Wasser gereinigt.

### VIa) [2-(10-Butyl-7,8-dimethyl-2,4-dioxo-4,10-dihydro-2H-benzo[g]pteridin-3-yl)-ethyl]-trimethyl-ammonium bromid (60)

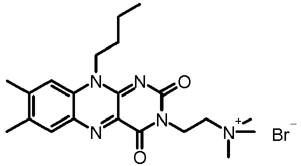

Aus 10-Butyl-7,8-dimethyl-10H-benzo[g]pteridin-2,4-dion **(56)** (310 mg, 1.0 mmol) durch Umsetzung gemäß der allgemeinen Vorschrift VI) als oranger Feststoff in einer Ausbeute von 161 mg (0,347 mmol, 35 % der Theorie) erhalten.

R_{f} = 0.1 (CHCl₃:MeOH - 4:1)

**¹H-NMR** (300 MHz, DMSO-d6): δ [ppm] = 0,97 (t, 3 H, J = 7.3 Hz), 1,48 (m, 2 H), 1,70 (m, 2 H), 2,41 (s, 3 H), 2,51 (s, 3 H), 3,14 - 3,35 (m, 8 H), 3,95 (t, 2 H, J = 6 Hz), 4,56 (t, 2 H, J = 7,8 Hz), 7,77 (s, 1 H), 7,89 (s, 1 H); - **MS** (ESI-MS, H₂O/MeOH + 0.1 % TFA): m/z (%) = 384.1 (100, (M⁺)); - **MG** = 384.51 + 79.90 g/mol - **MF** = C₂₁H₃₀N₅O₂Br

### VIb) {2-[10-(2-Acetylamino-ethyl)-7,8-dimethyl-2,4-dioxo-4,10-dihydro-2H-benzo[g]pteridin-3-yl]-ethyl}-trimethyl-ammonium bromid (61)

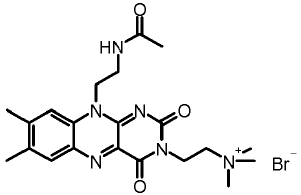

Das unter IIa) erhaltene Flavin (57) (330 mg, 1.0 mmol) ergab bei Umsetzung nach der allgemeinen Vorschrift IV) 178 mg eines orangen Feststoffes (0,361 mmol, 36 % der Theorie)

R_{f} = 0.1 (CHCl₃:MeOH - 4:1)

**¹H-NMR** (300 MHz, DMSO-d6): δ [ppm] = 2,03 (s, 3 H), 2,41 (s, 3 H), 2,51 (s, 3 H), 3,16 - 3,32 (m, 8 H), 3.46 (t, 2 H, J = 5.6 Hz), 4,66 (t, 2 H, J = 5.6 Hz), 3,96 (t, 2 H, J = 6 Hz), 6,81 (m, 1 H), 7,75 (s, 1 H), 7,88 (s, 1 H); - **MS** (ESI-MS, H₂O/MeOH + 0.1 % TFA): m/z (%) = 413.1 (100, (M⁺)); - **MG** = 413.50 + 79.90 g/mol - **MF** = C₂₁H₂₉N₆O₃Br

### VII) {2-[7,8-Dimethyl-2,4-dioxo-10-(2,3,4,5-tetraacetoxy-pentyl)-4,10-dihydro-2H-benzo[g]pteridin-3-yl]-ethyl}-trimethyl-ammonium bromid (69)

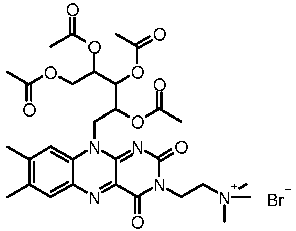

Riboflavin tetraacetat **(66)** (540 mg, 1.0 mmol) ergab bei Umsetzung nach der allgemeinen Vorschrift IV) 191 mg eines hellbraunen Feststoffes (0,268 mmol, 27 % der Theorie)

R_{f} = 0.1 (CHCl₃:MeOH - 4:1)

**¹H-NMR** (300 MHz, MeOD): δ [ppm] = 1,71 (s, 3 H), 2,02 (s, 3 H), 2,21 (s, 3 H), 2,29 (s, 3 H,), 2,42 (s, 3 H), 2,52 (s, 3 H), 3,12 - 3,27 (m, 8 H), 3,94 (t, 2 H, J = 5,8 Hz), 4,20 - 4,28 (m, 1 H), 4,52 (m, 1 H), 5,18 (m, 2 H), 5,44 (m, 1 H), 5,54 (m, 1 H), 5,68 (m, 1 H), 7,89 (s, 1 H), 7,93 (s, 1 H); - **MS** (ESI-MS, H₂O/MeOH + 0.1 % TFA): m/z (%) = 630.1 (100, (M⁺)); - **MG** = 630.68 + 79.90 g/mol - **MF** = C₃₀H₄₀N₅O₁₀Br

### VIII) Triethyl-[7-methyl-2,4-dioxo-10-(2,3,4,5-tetraacetoxy-pentyl)-2,3,4,10-tetrahydro-benzo[g]pteridin-8-ylmethyl]-ammonium bromid (68)

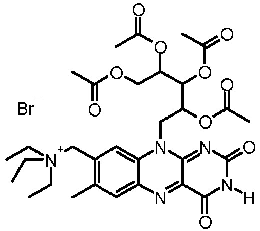

8a-Bromo-tetraacetylriboflavin **(67)** (0,78 g, 1,25 mmol) wurde in DMF (10 mL) gelöst, die Lösung wurde entgast und Triethylamin (0,60 g, 0,76 mL, 6.0 mmol) wurde über 5 min. zugetropft. Der Ansatz wurde bei 50°C über Nacht unter N₂ im Dunklen gerührt. Nach Abkühlen auf Raumtemperatur wurde die Reaktionsmischung in 200 mL eiskalten Diethylether eingetropft, das Produkt wurde abgefiltert, mit Diethylether gewaschen und bei vermindertem Druck getrocknet. Der Rückstand wurde in Chloroform gelöst, mit Diethylether gefällt, abzentrifugiert und getrocknet. Zur weiteren Reinigung wurde aus Wasser umkristallisiert. Hellbrauner Feststoff (0.31 g, 0.43 mmol, 34 % der Theorie).

**¹H-NMR** (300 MHz, MeOD): δ [ppm] = 1,25 (t, 9 H, J = 7,3 Hz), 1,71 (s, 3 H), 2,02 (s, 3 H), 2,21 (s, 3 H), 2,29 (s, 3 H,), 2,42 (s, 3 H), 2,59 (m, 2 H), 3,15 - 3,30 (m, 6 H), 4,20 - 4,28 (m, 1 H), 4,52 (m, 1 H), 5,18 (m, 2 H), 5,44 (m, 1 H), 5,54 (m, 1 H), 5,68 (m, 1 H), 7,89 (s, 1 H), 7,93 (s, 1 H), 11,32 (s, 1 H); - **MS** (ESI-MS, H₂O/MeOH + 0.1 % TFA): m/z (%) = 644.1 (100, (M⁺)); - **MG** = 644.71 + 79.90 - **MF** = C₃₁H₄₂N₃O₁₀Br

### IX) 2-Carbamoyl-1-13-[7,8-dimethyl-2,4-dioxo-10-(2,3,4,5-tetraacetoxy-pentyl)-4,10-dihydro-2H-benzo[g]pteridin-3-yl]-propyl]-pyridinium iodid (72)

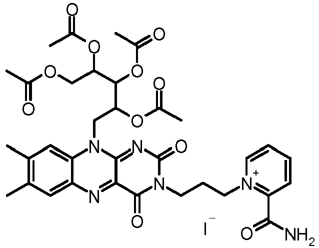

Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester **(70)** (0.75 g, 1.05 mmol) wurde mit Nicotinsäureamid (146 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 277 mg hellbrauner Feststoff (0,33 mmol, 32 % der Theorie) erhalten.

**¹H-NMR** (300 MHz, MeOD): δ [ppm] = 1,70 (s, 3 H), 2,03 (s, 3 H), 2,20 (s, 3 H), 2,28 (s, 3 H,), 2,42 (s, 3 H), 2,48 (m, 2 H), 2,52 (s, 3 H), 4,18 - 4,31 (m, 3 H), 4,51 (m, 1 H), 4,88 (m, 2 H), 5,17 (m, 2 H), 5,45 (m, 1 H), 5,52 (m, 1 H), 5,67 (m, 1 H), 7,86 (s, 1 H), 7,92 (s, 1 H), 8,70 - 8,78 (m, 2 H), 9,23 (d, J = 5,6 Hz, 1 H), 9,38 (d, J = 5.6 Hz, 1 H); - **MS** (ESI-MS, H₂O/MeOH + 0.1 % TFA): m/z (%) = 707.1 (100, (M⁺)); - **MG** = 707.72 + 126.90 - **MF** = C₃₄H₃₉N₆O₁₁I

### X) 3-(10-Butyl-7,8-dimethyl-2,4-dioxo-4,10-dihydro-2H-benzo[g]pteridin-3-yl)-propyliodid (80)

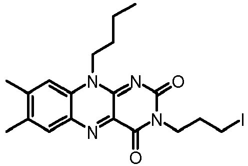

Verbindung **(56)** (0.6 g, 2.0 mmol) und Cs₂CO₃ (1.8 g, 6 mmol) wurden in trockenem DMF (40 mL) vorgelegt. 1,3-Diiodopropan (1.5 g, 5.0 mmol) wurde zugegeben und der Ansatz 2 h im Dunklen unter N₂ bei Raumtemperatur gerührt. Das Lösungsmittel wurde unter vermindertem Druck abgezogen, der Rückstand in Ethylacetat (200 mL) gelöst und mit H₂O (2x 100 mL) gewaschen. Nach Trocknen der organischen Phase über MgSO₄ wurde das Lösungsmittel bei vermindertem Druck abgezogen. Nach Reinigung durch Säulenchromatographie (Kieselgel, CHCl₃ /MeOH, 98:2), wurde die Zielverbindung (0.82 g, 1.76 mmol, 88%) als gelber Feststoff erhalten.

**¹H-NMR** (300 MHz, CDCl₃): δ [ppm] = 0,97 (t, *J* = 7,4 Hz, 3 H), 1,48 (m, 2 H), 1,72 (m, 2 H), 2,23-2,36 (m, 2 H), 2,41 (s, 3 H), 2,50 (s, 3 H), 3,24 (t, *J* = 7,4 Hz, 2 H), 4,15 - 4,26 (m, 2 H), 4,56 (t, *J=* 7,8 Hz, 2 H), 7,77 (s, 1 H), 7,89 (s, 1 H); - **MS** (ESI-MS, H₂O/MeOH + 0.1 % TFA): m/z (%) = 467.1 (100, (MH⁺)); - **MG** = 466.33 g/mol - **MF** = C₁₉H₂₃N₄O₂I

### XI) 2-Carbamoyl-1-{3-[7,8-dimethyl-2,4-dioxo-10-butyl-4,10-dihydro-2H-benzo[g]pteridin-3-yl]-propyl}-pyridinium iodid (73)

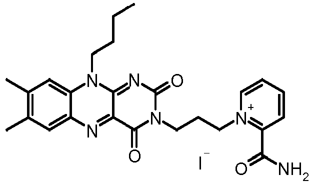

3-(10-Butyl-7,8-dimethyl-2,4-dioxo-4,10-dihydro-2H-benzo[g]pteridin-3-yl)-propyliodid **(80)** (0.51 g, 1.05 mmol) wurde mit Nicotinsäureamid (146 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 276 mg oranger Feststoff (0,47 mmol, 46 % der Theorie) erhalten.

**¹H-NMR** (300 MHz, DMSO): δ [ppm] = 0,91 (t, 3 H), 1,43 (m, 2 H), 1,72 (m, 2H), 2,31 (m, 2 H), 2,41 (s, 3 H), 2,58 (s, 3 H,), 4,02 (m, 2 H), 4,63 (m, 2 H), 4,71 (m, 2 H), 5,31 (s, 2 H), 7,86 (s, 1 H), 7,95 (m, 1 H), 8,18 (s, 1 H), 8,51 (s, 1 H), 8,94 (d, J = 5,6 Hz, 1 H), 9,23 (d, J = 5.6 Hz, 1 H), 9.52 (m, 1 H); - **MS** (ESI-MS, H₂O/MeOH + 0.1 % TFA): m/z (%) = 461.1 (100, (M⁺)); - **MG** = 461.55 + 126.90 - **MF** = C₂₅H₂₉N₆O₃I

### XII) 10-Hexadecyl-7,8-dimethyl-10H-benzo[g]pteridin-2,4-dion (87)

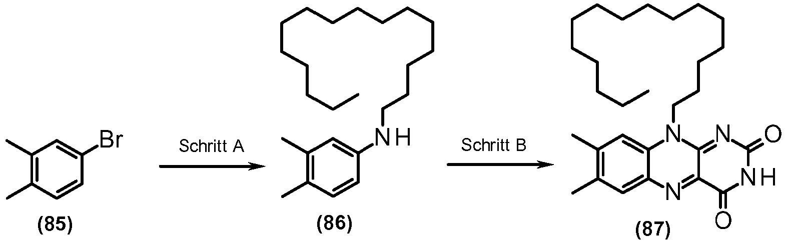

### Schritt A. Synthese des Amins (86)

3,4-Dimethyl-1-brombenzen (85) (0.37 g, 2 mmol) wurde zu einer Suspension von Palladium(II)acetat (22 mg, 0.1 mmol) und Natrium-tert-butylat (224 mg, 2.3 mmol) in trockenem Dioxan (5 mL) unter Argon in einem trockenem Schlenckrohr zugegeben. Nach Zugabe von Hexadecylamin (0.72 g, 3 mmol) und Tri(tert-butyl)phosphin in Toluol (0.3 mL) wurde über Nacht bei 80°C gerührt. Die Reaktionsmischung wurde nach Abkühlen auf Raumtemperatur mit Dichlormethan (30 mL) verdünnt und mit H₂O (2x 10 mL) gewaschen. Nach Trocknen der organischen Phase über MgSO₄ wurde das Lösungsmittel bei vermindertem Druck abgezogen. Nach Reinigung durch Säulenchromatographie (Kieselgel, Ethylacetat/EtOH, 9:1), wurde die Zielverbindung (86) (0.62 g, 1.79 mmol, 90%) als farbloser Feststoff erhalten.

### Schritt B. Synthese des Flavins (87)

Das Amin (86) aus dem vorherigen Schritt (0.62 g, 1.79 mmol) wurde mit Violursäure (320 mg, 2 mmol) in Eisessig (10 mL) in Stickstoffatmosphäre im Dunklen für 24 h refluxiert. Violursäure (320 mg, 2 mmol) wurde zugegeben und der Ansatz weitere 24h bei denselben Bedingungen gehalten. Das Lösungsmittel wurde bei vermindertem Druck abgezogen und der Rückstand durch präparative Dünnschichtchromatographie gereinigt (CH₂Cl₂/EtOH 20:1). Es wurden 254 mg oranger Feststoff (0,54 mmol, 30 % der Theorie) erhalten.

**¹H-NMR** (300 MHz, DMSO-d6): δ [ppm] = 1,02 (t, 3 H), 1,20 - 1,37 (m, 26 H), 1,73 (m, 4H), 2,43 (s, 3 H), 2,56 (s, 3 H,), 7,48 (s, 1 H), 8,04 (s, 1 H), 11,30 (bs, 1 H); - **MS** (ESI-MS, H₂O/MeOH + 0.1 % TFA): m/z (%) = 467.1 (100, (M⁺)); - **MG** = 466.67 - **MF** = C₂₈H₄₂N₄O₂

### Allgemeine Vorschrift XIII): Funktionalisierung von Flavinen in Position 3 durch 3-(N,N,N-Triethylammonium)-propyl-1-iodid iodid oder 3-(Pyridinium)-propyl-1-iodid iodid

**Tabelle 2: verwendete Flavine und**

| Beispiel | Zielverbindung | Flavin |
|---|---|---|
| XIa) | (74) | (56) |
| XIb) | (75) | (56) |
| XIc) | (76) | (87) |

Das jeweils in Tabelle 2 angegebene Flavin (1,0 mmol) wurde in trockenem DMF (20 mL) gelöst, Kaliumcarbonat (0,69 g, 5 mmol) und 3-(N,N,N-Triethylammonium)-propyl-1-iodid iodid oder 3-(Pyridinium)-propyl-1-iodid iodid (0,8 g bzw. 0,76 g, 2.0 mmol) wurden zugegeben und der Ansatz für 1d bei Raumtemperatur im Dunklen gerührt. Es wurde erneut 3-(N,N,N-Triethylammonium)-propyl-1-iodid iodid oder 3-(Pyridinium)-propyl-1-iodid iodid (0,8 g bzw. 0,76 g, 2.0 mmol) zugegeben und der Ansatz einen weiteren Tag bei Raumtemperatur im Dunklen gerührt. Das DMF wurde abgezogen. Das Rohprodukt wurde durch Säulenchromatographie an Kieselgel (CHCl₃/MeOH - 20:1 → 6:1) gereinigt.

### XIIIa) [3-(10-Butyl-7,8-dimethyl-2,4-dioxo-4,10-dihydro-2H-benzo[g]pteridin-3-yl)-propyl]-triethyl-ammonium iodid (74)

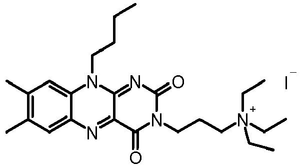

Aus 10-Butyl-7,8-dimethyl-10H-benzo[g]pteridin-2,4-dion **(56)** (310 mg, 1.0 mmol) und 3-(N,N,N-Triethylammonium)-propyl-1-iodid iodid durch Umsetzung gemäß der allgemeinen Vorschrift XII) als oranger Feststoff in einer Ausbeute von 188 mg (0,331 mmol, 33 % der Theorie) erhalten.

R_{f} = 0,05 (CHCl₃:MeOH - 6:1)

**¹H-NMR** (300 MHz, DMSO): δ [ppm] = 0,92 (t, 3 H), 1,18 (m, 9 H), 1,45 (m, 2 H), 1,68 (m, 2H), 1,97 (m, 2 H), 2,39 (s, 3 H), 2,52 (s, 3 H,), 3,21 (m, 8 H), 3,31 (s, 6 H), 3,97 (m, 2 H), 4,01 (m, 2 H), 7,86 (s, 1 H), 7,98 (s, 1 H); - **MS** (ESI-MS, H₂O/MeOH + 0.1 % TFA): m/z (%) = 440.2 (100, (M⁺)); **-MG** = 440.61 + 126.90 g/mol - **MF** = C₂₅H₃₈N₅O₂I

### XIIIb) [3-(10-Butyl-7,8-dimethyl-2,4-dioxo-4,10-dihydro-2H-benzo[g]pteridin-3-yl)-propyl]-pyridinium iodid (75)

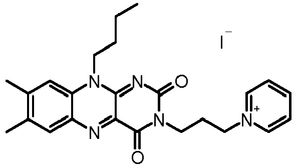

Aus 10-Butyl-7,8-dimethyl-10H-benzo[g]pteridin-2,4-dion **(56)** (310 mg, 1.0 mmol) und 3-(Pyridinium)-propyl-1-iodid iodid durch Umsetzung gemäß der allgemeinen Vorschrift XIII) als oranger Feststoff in einer Ausbeute von 196 mg (0,36 mmol, 36 % der Theorie) erhalten.

Rf = 0.05 (CHCl₃:MeOH - 6:1)

**¹H-NMR** (300 MHz, DMSO): δ [ppm] = 0,93 (t, 3 H), 1,48 (m, 2 H), 1,71 (m, 2H), 2,30 (m, 2 H), 2,46 (s, 3 H), 2,54 (s, 3 H,), 3,98 (m, 2 H), 4,61 (m, 2 H), 4,72 (m, 2 H), 5,31 (s, 2 H), 7,82 (s, 1 H), 7,92 (s, 1 H), 8,19 (m, 2 H), 8,61 (m, 1 H), 9,22 (d, J = 5.6 Hz, 1 H); - **MS** (ESI-MS, H₂O/MeOH + 0.1 % TFA): m/z (%) = 418.1 (100, (M⁺)); - **MS** (ESI-MS, H₂O/MeOH + 0.1 % TFA): m/z (%) = 418.2 (100, (M⁺)); - **MG** = 418.51 + 126.90 g/mol - **MF** = C₂₄H₂₈N₅O₂I

### XIIIc) [3-(10-Hexadecyl-7,8-dimethyl-2,4-dioxo-4,10-dihydro-2H-benzo[g]pteridin-3-yl)-propyl]-triethyl-ammonium iodid (76)

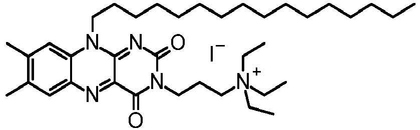

Das unter XII) erhaltene Flavin (87) (466 mg, 1.0 mmol) ergab bei Umsetzung nach der allgemeinen Vorschrift XIII) mit 3-(N,N,N-Triethylammonium)-propyl-1-iodid iodid 131 mg eines orangen Feststoffes (0,178 mmol, 18 % der Theorie)

R_{f} = 0.1 (CHCl₃:MeOH - 6:1)

**¹H-NMR** (300 MHz, DMSO): δ [ppm] = 0,85 (t, 3 H), 1,17 (m, 9 H), 1,03 (t, 3 H), 1,20 - 1,39 (m, 26 H), 1,52 (m, 2 H), 1,73 (m, 4H), 1,81 (m, 2 H), 2,26 (m, 2 H), 2,42 (s, 3 H), 2,53 (s, 3 H,), 3,28 (s, 6 H), 3,74 (m, 2 H), 3,46 (m, 2 H), 4,30 (m, 2 H), 4,72 (m, 2 H), 7,48 (s, 1 H), 8,05 (s, 1 H); - **MS** (ESI-MS, H₂O/MeOH + 0.1 % TFA): m/z (%) = 608.2 (100, (M⁺));-**MG** = 608.90 + 126.90 g/mol - **MF** = C₃₇H₆₂N₅O₂I

### XIV) [3-(10-Butyl-7,8-dimethyl-2,4-dioxo-4,10-dihydro-2H-benzo[g]pteridin-3-yl)-propyl]-tri(dioxa-3,6-heptyl)-ammonium iodid (77)

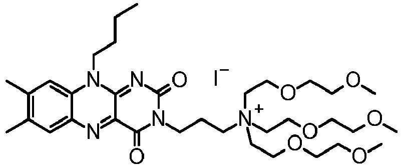

3-(10-Butyl-7,8-dimethyl-2,4-dioxo-4,10-dihydro-2H-benzo[g]pteridin-3-yl)-propyliodid **(80)** (0.51 g, 1.05 mmol) wurde mit Tris(dioxa-3,6-heptyl)amine (646 mg, 2 mmol) und NaHCO₃ (0.1 g, 1.2 mmol) in DMF (4 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 103 mg braunrotes, zähes Öl (0,13 mmol, 12 % der Theorie) erhalten.

**MS** (ESI-MS, H₂O/MeOH + 0.1 % TFA): m/z (%) = 662.1 (100, (M⁺)); - **MG** = 662.55 + 126.90 - **MF** = C₃₄H₅₆N₅O₈I

### XV) [3-(10-Butyl-7,8-dimethyl-2,4-dioxo-4,10-dihydro-2H-benzo[g]pteridin-3-yl)-propyl]-dimethyl-hexadecyl-ammonium iodid (78)

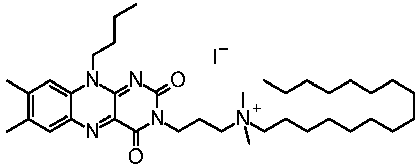

3-(10-Butyl-7,8-dimethyl-2,4-dioxo-4,10-dihydro-2H-benzo[g]pteridin-3-yl)-propyliodid **(80)** (0.51 g, 1.05 mmol) wurde mit N,N-Dimethyl-hexadecylamin (540 mg, 2 mmol) und NaHCO₃ (0.1 g, 1.2 mmol) in DMF (4 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Es wurde 147 mg hellbrauner Feststoff (0,20 mmol, 19 % der Theorie) erhalten.

**¹H-NMR** (300 MHz, CDCl₃): δ [ppm] = 0,83 (t, 3 H), 1,04 (t, 3 H), 1,21 - 1,38 (m, 26 H), 1,52 (m, 2 H), 1,71 (m, 4H), 1,82 (m, 2 H), 2,28 (m, 2 H), 2,41 (s, 3 H), 2,54 (s, 3 H,), 3,29 (s, 6 H), 3,75 (m, 2 H), 3,43 (m, 2 H), 4,29 (m, 2 H), 4,70 (m, 2 H), 7,41 (s, 1 H), 8,06 (s, 1 H); - **MS** (ESI-MS, H₂O/MeOH + 0.1 % TFA): m/z (%) = 608.1 (100, (M⁺)); - **MG** = 608.90 + 35.45 - **MF** = C₃₇H₆₂N₅O₂Cl

### Beispiel 2) Phototoxizitätsexperimente

### a) Herstellung der Testplatten und Bakterienstämme

Eine Probe des Bakterienstammes *Staphylococcus. aureus* (ATCC-Nummer: 25923) *oder Escherichia. coli* (ATCC-Nummer: 25922) wurde aus einer Cryo-Gefrierkultur entnommen, auf Müller-Hinton-Agar-Platten vereinzelt und unter aeroben Bedingungen bei 37°C in einer Übernachtkultur kultiviert. Daran anschließend wurden 5 ml Müller-Hinton-Flüssigmedium mit einem Abstrich der Bakterienkultur (Einzelkolonie) beimpft und über Nacht bei 37°C bebrütet. Die so gewonnene Bakteriensuspension wurde 10 min bei 2500 Upm zentrifugiert und das erhaltene Bakterienpellet in 5 ml sterilem PBS resuspendiert. Die optische Dichte der Bakteriensupensionen für die Phototoxizitätstests betrug OD₆₀₀ₙₘ= 0.6, was einer Bakterienzahl von ~1-8×10⁸⁻¹² Bakterien pro ml entspricht. Die biochemische Analyse und Resistenzbestimmung der Bakterien wurde mit dem VITEK2-System gemäß den Richtlinien M100-S14 des NCCLS (2004) durchgeführt.

Zur Empfindlichkeitsprüfung medizinisch bedeutender Krankheitserreger gegenüber Antibiotika und Sulfonamiden wurden gemäß der NCCLS Richtlinien Müller-Hinton-Medien verwendet (Deutsche Gesellschaft für Hygiene und Mikrobiologie (DGHM), Institut für Hygiene und Mikrobiologie, Universität Bonn, Deutschland):

### a) Müller-Hinton-Bouillon (Oxoid, Wesel, Deutschland)

2,0 g/l Rindfleisch, getrocknete Infusion aus 300 g, 17,5 g/l Caseinhydrolysat,
1,5 g/l Stärke, pH: 7,4 + 0,2

### b) Müller Hinton-Agar (Oxoid, Wesel, Deutschland)

2,0 g/l Rindfleisch, getrocknete Infusion aus 300 g, 17,5 g/l Caseinhydrolysat,
1,5 g/l Stärke, pH: 7,4 + 0,2
13 g/l Agaragar

### b) Durchführung des Phototoxizitätstests:

200 µl einer Bakteriensuspension (Bakteriendichte: 10⁸- 10¹² /ml) wurden mit je 200 µl verschiedener Konzentrationen der zu testenden Photosensibilisatoren bei Raumtemperatur für 10 min oder 30 min inkubiert. Danach wurden die Bakterien zweimal mit Aqua dest. gewaschen, in 200 µl Aqua dest. resuspendiert, das gesamte Volumen auf eine 96-well Mikrotiterplatte übertragen und anschließend bestrahlt. Die verwendeten Photosensibilisatoren wurden in Aqua dest. gelöst und verschiedene Verdünnungsreihen hergestellt (0µM, 1µM, 10µM, 100µM).

Zur Sensibilisierung wurde die Lampe Omnicure Series 2000 (Photonics Solutions Inc., Edinburgh, UK) benutzt, die Licht aus einem Bereich von 390 nm bis 500nm emittiert und Emissionsmaxima Eₘₐₓ bei 405 nm und 436 nm aufweist. Die applizierte Leistung betrug jeweils 50mW/cm².

Als Kontrollen wurden bestrahlte und nicht bestrahlte Proben verwendet. Ebenso wurden nur mit Photosensibilisator inkubierte Bakteriensuspensionen mitgeführt (Dunkelkontrolle).

Die Bestimmung der koloniebildenden Einheiten (KBE bzw. CFU) pro ml wurde gemäß der von Miles und Misra publizierten Methode durchgeführt (Miles, AA; Misra, SS, Irwin, JO (1938 Nov). "The estimation of the bactericidal power of the blood.". The Journal of hygiene 38 (6): 732-49). Dazu wurden serielle Verdünnungen von 10⁻² bis 10⁻⁹ der entsprechenden Bakteriensuspension hergestellt. Je 3x 20 µl der entsprechenden Bakterienverdünnungen wurden dann auf Müller-Hinton Platten aufgetropft und bei 37° C für 24 h inkubiert. Danach wurde die Anzahl der überlebenden koloniebildenden Einheiten (KBE bzw. CFU) bestimmt. Alle Versuche wurden jeweils dreimal wiederholt.

### c) Ergebnis der Phototoxizitätsexperimente:

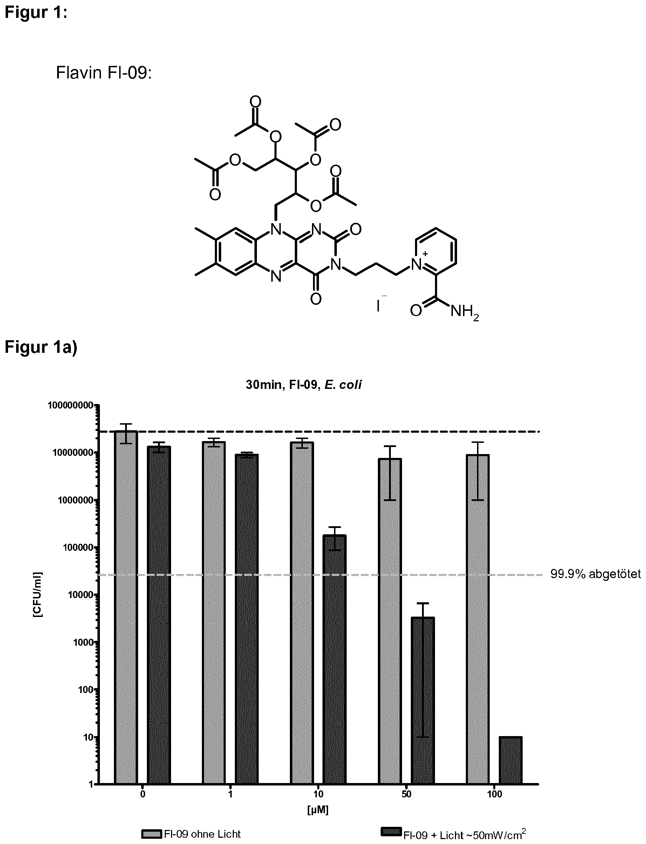

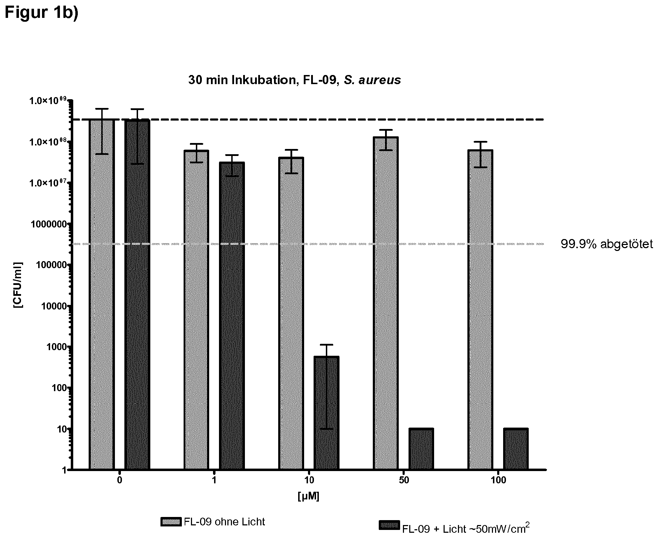

Die Ergebnisse der Phototoxizitätsexperimente sind in den Figuren 1-7 dargestellt.

Die Figuren 1-8 zeigen die logarithmische Abnahmen der CFU/ml 24 Std nach Bestrahlung sowie die dazugehörigen Kontrollen (nur bestrahlte Bakterien; mit Photosensibilisator inkubierte Bakterien, aber nicht bestrahlt; unbehandelte Bakterien) für den jeweils angegeben Photosensibilisator.

Die angegebenen koloniebildenden Einheiten (KBE bzw. CFU) pro ml sind jeweils der Median aus drei Versuchen.

Figur 1 zeigt die Wirkung von Flavin FL-09 (Iodid der Verbindung mit den Formeln (36)) auf E. coli und S. aureus.

Figur 1a: E. coli Proben wurden mit verschiedenen Konzentrationen (0 µM, 1 µM, 10 µM, 50 µM, 100 µM) von Flavin FL-09 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-09, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 1b: S. aureus Proben wurden mit verschiedenen Konzentrationen (0 µM, 1 µM, 10 µM, 50 µM, 100 µM) von Flavin FL-09 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-09, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

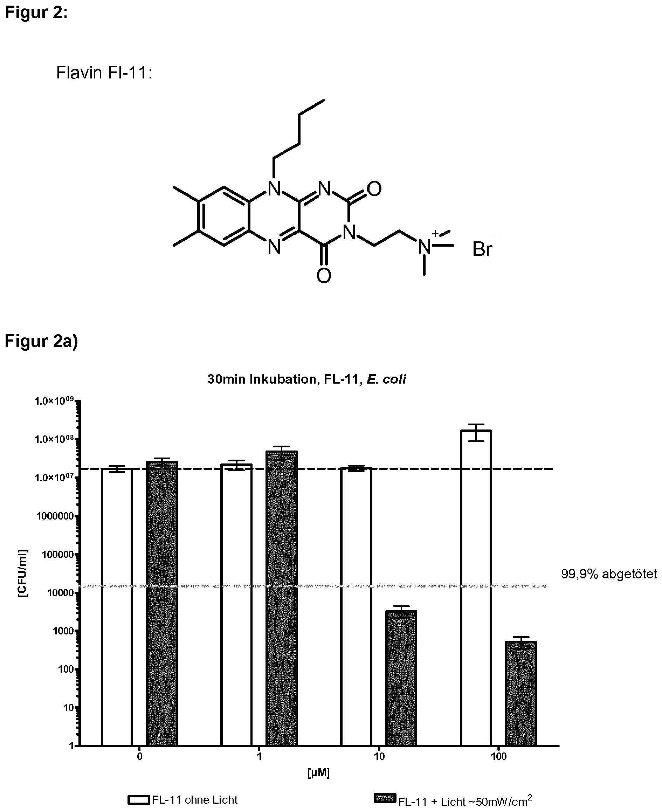

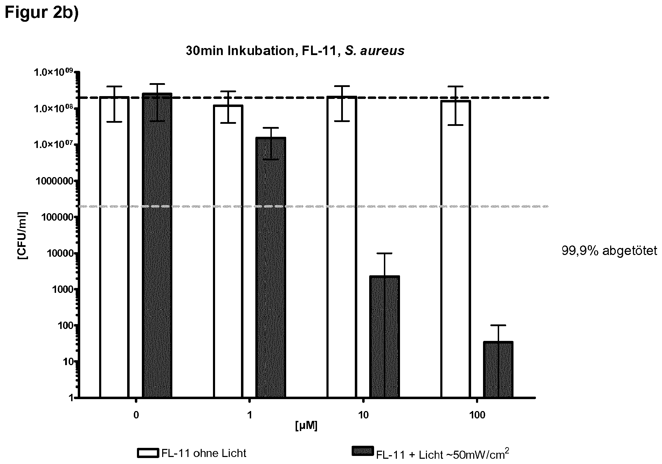

Figur 2 zeigt die Wirkung von Flavin FL-11 (Bromid der Verbindung mit den Formeln (32)) auf E. coli und S. aureus.

Figur 2a: E. coli Proben wurden mit verschiedenen Konzentrationen (0 µM, 1 µM, 10 µM, 100 µM) von Flavin FL-11 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-11, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 2b: S. aureus Proben wurden mit verschiedenen Konzentrationen (0 µM, 1 µM, 10 µM, 50 µM, 100 µM) von Flavin FL-11 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-11, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

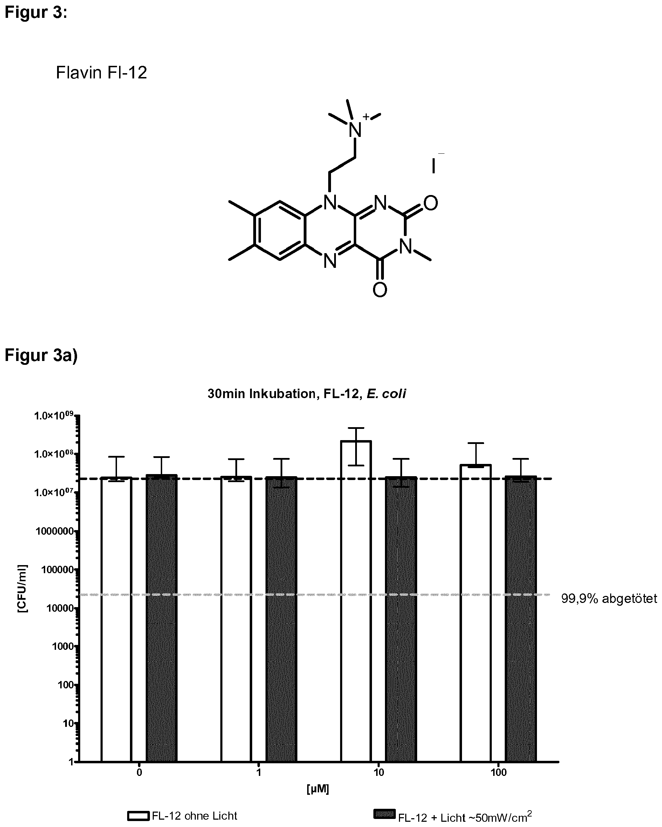

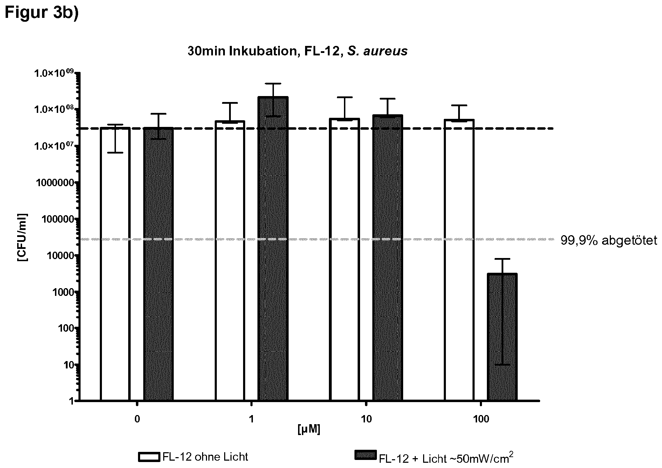

Figur 3 zeigt die Wirkung von Flavin FL-12 (Iodid der Verbindung mit den Formeln (30)) auf E. coli und S. aureus.

Figur 3a: E. coli Proben wurden mit verschiedenen Konzentrationen (0 µM, 1 µM, 10 µM, 100 µM) von Flavin FL-12 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-12, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 3b: S. aureus Proben wurden mit verschiedenen Konzentrationen (0 µM, 1 µM, 10 µM, 50 µM, 100 µM) von Flavin FL-12 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-12, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

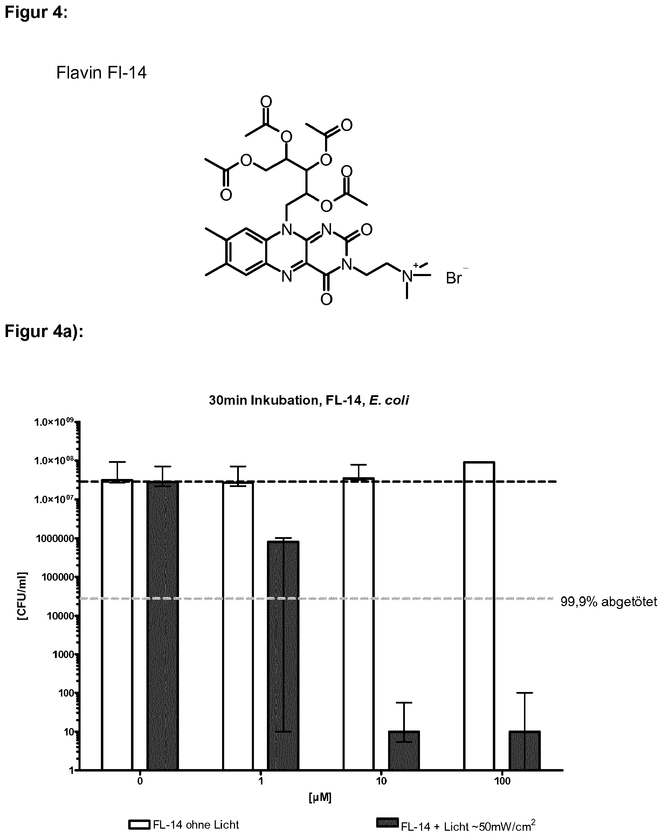

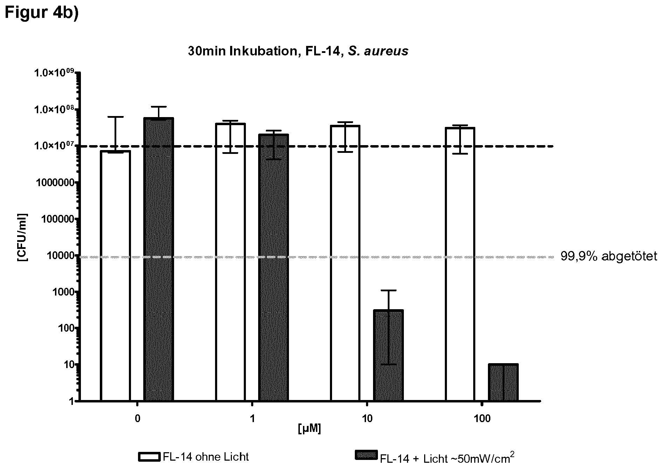

Figur 4 zeigt die Wirkung von Flavin FL-14 (Bromid der Verbindung mit den Formeln (35)) auf E. coli und S. aureus.

Figur 4a: E. coli Proben wurden mit verschiedenen Konzentrationen (0 µM, 1 µM, 10 µM, 100 µM) von Flavin FL-14 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-14, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 4b: S. aureus Proben wurden mit verschiedenen Konzentrationen (0 µM, 1 µM, 10 µM, 50 µM, 100 µM) von Flavin FL-14 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-14, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

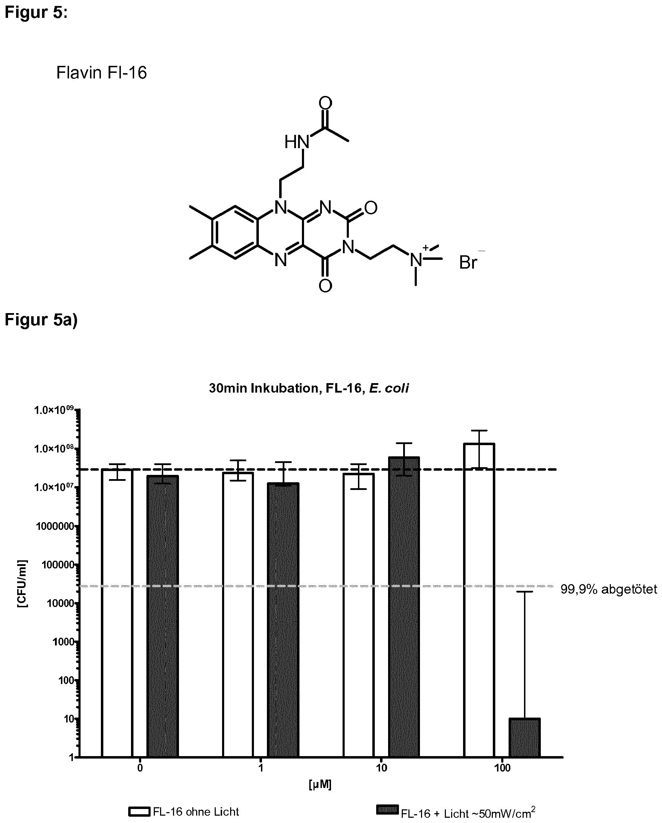

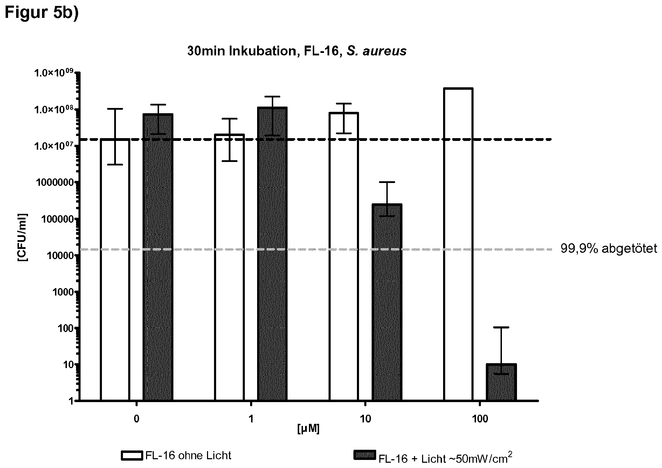

Figur 5 zeigt die Wirkung von Flavin FL-16 (Bromid der Verbindung mit den Formeln (33)) auf E. coli und S. aureus.

Figur 5a: E. coli Proben wurden mit verschiedenen Konzentrationen (0 µM, 1 µM, 10 µM, 100 µM) von Flavin FL-16 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-16, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 5b: S. aureus Proben wurden mit verschiedenen Konzentrationen (0 µM, 1 µM, 10 µM, 50 µM, 100 µM) von Flavin FL-16 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-16, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

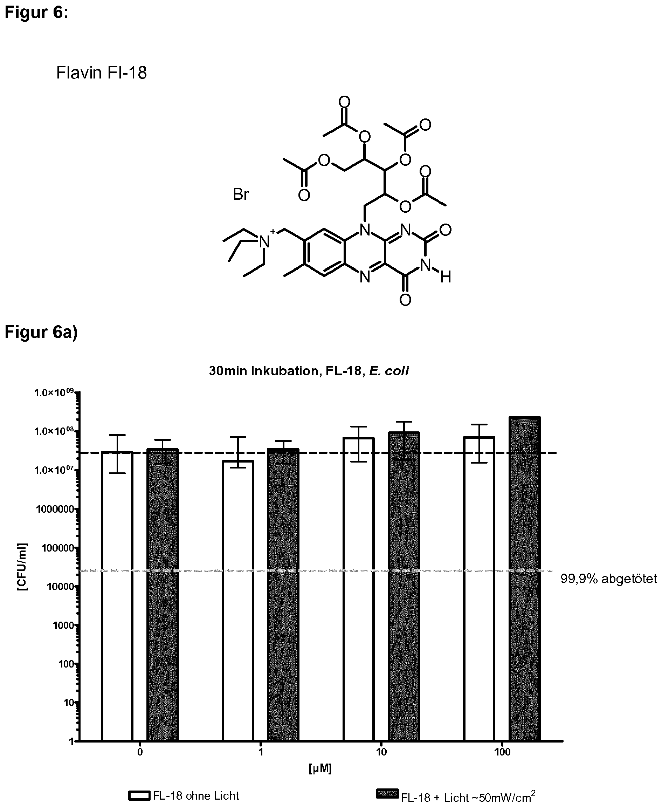

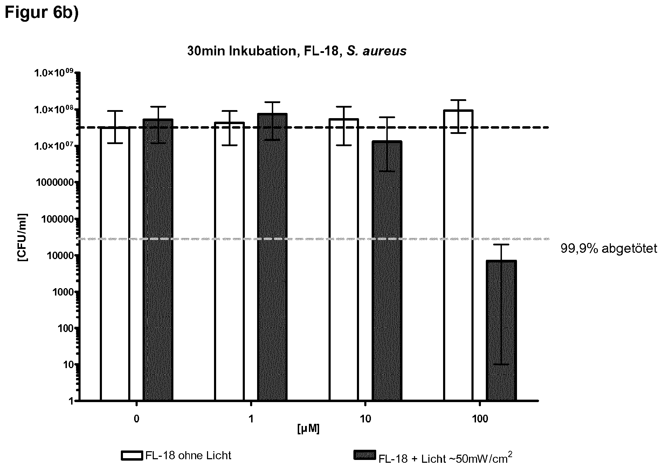

Figur 6 zeigt die Wirkung von Flavin FL-18 (Bromid der Verbindung mit den Formeln (37)) auf E. coli und S. aureus.

Figur 6a: E. coli Proben wurden mit verschiedenen Konzentrationen (0 µM, 1 µM, 10 µM, 100 µM) von Flavin FL-18 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-18, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 6b: S. aureus Proben wurden mit verschiedenen Konzentrationen (0 µM, 1 µM, 10 µM, 50 µM, 100 µM) von Flavin FL-18 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-18, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

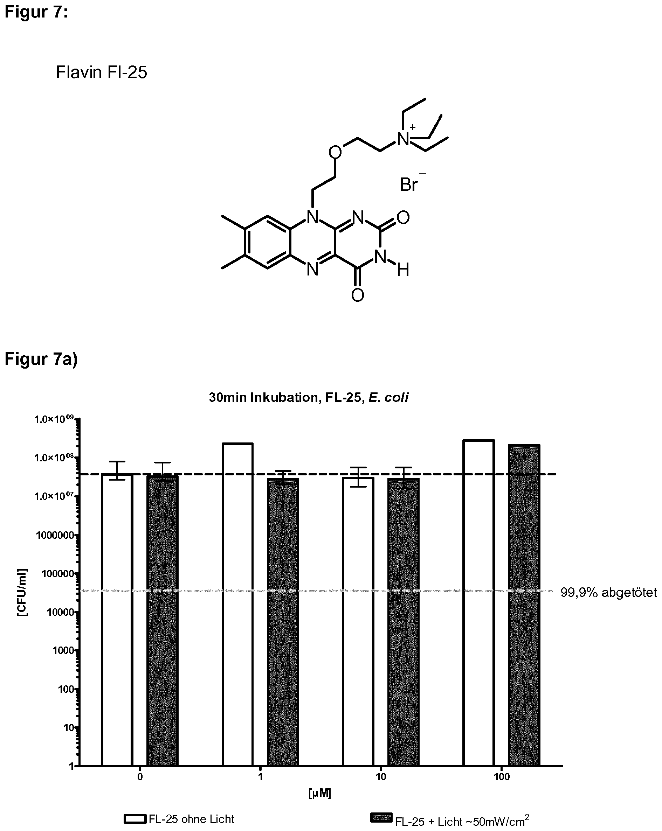

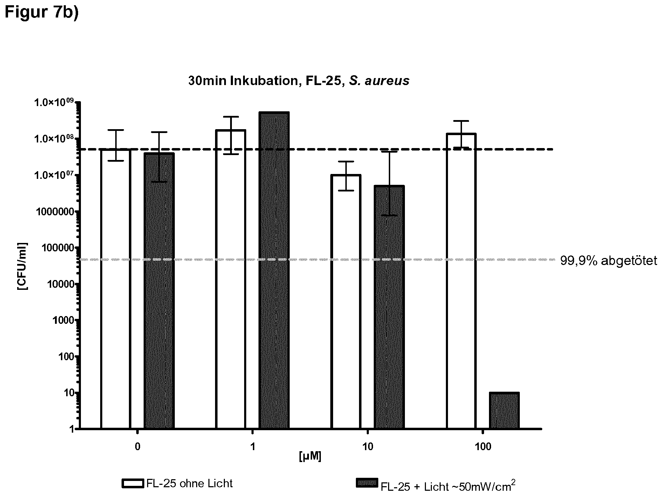

Figur 7 zeigt die Wirkung von Flavin FL-25 (Bromid der Verbindung mit den Formeln (31)) auf E. coli und S. aureus.

Figur 7a: E. coli Proben wurden mit verschiedenen Konzentrationen (0 µM, 1 µM, 10 µM, 100 µM) von Flavin FL-25 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-25, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 7b: S. aureus Proben wurden mit verschiedenen Konzentrationen (0 µM, 1 µM, 10 µM, 50 µM, 100 µM) von Flavin FL-25 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-25, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

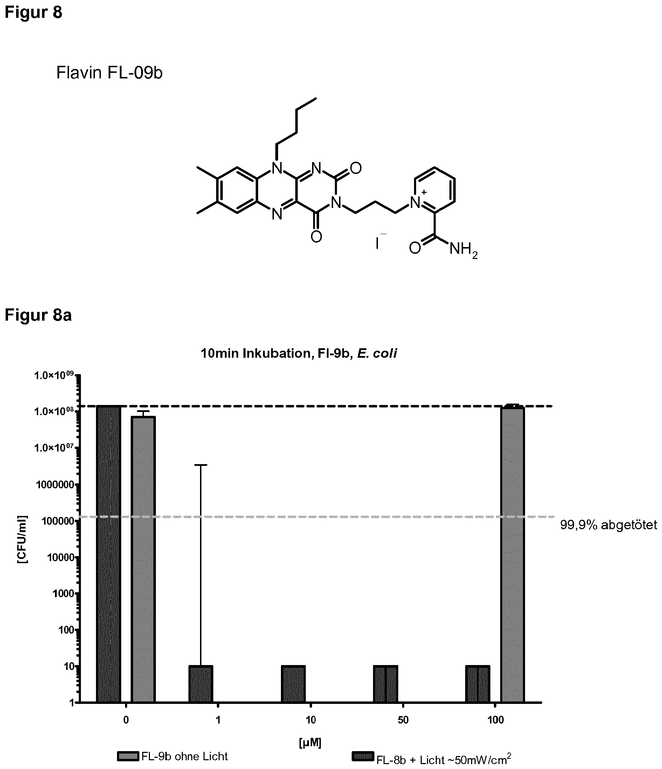

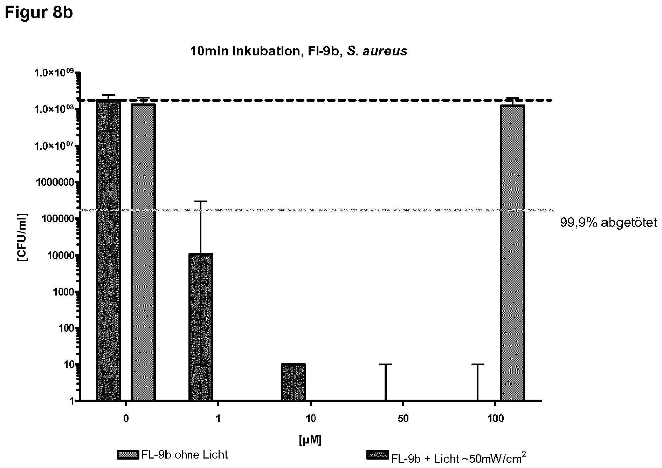

Figur 8 zeigt die Wirkung von Flavin FL-9a (Jodid der Verbindung mit den Formeln (43)) auf E. coli und S. aureus.

Figur 8a: E. coli wurde mit verschiedenen Konzentrationen von FL-09b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.

Figur 8b: S. aureus wurde mit verschiedenen Konzentrationen von FL-09b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.

Wie aus den Figuren 1-8 ersichtlich, hat eine Bestrahlung der verwendeten Mikroorganismen *Staphylococcus aureus (S. aureus)* und *Escherichia coli (E. coli)* mit einer Lichtdosis von 10.5J/cm² mit blauem Licht (390nm - 500nm) in Abwesenheit eines Photosensibilisators (0µM des jeweiligen Flavins) keinen Einfluss auf die Anzahl der überlebenden Mikroorganismen im Vergleich zur unbelichteten Kontrolle.

Darüber hinaus zeigen die in den Figuren 1-8 dargestellten Ergebnisse, dass die Inkubation (10min bzw. 30min) des jeweiligen Photosensibilisators mit den Mikroorganismen ohne nachfolgende Belichtung ebenfalls keinen Einfluss auf die Anzahl der überlebenden Mikroorganismen hat.

Wie aus den Figuren 1-8 ersichtlich erfolgt nach Inkubation (10min bzw. 30min) der Mikroorganismen in Abhängigkeit der verwendeten Konzentration der jeweiligen Photosensibilisatoren und nachfolgender Bestrahlung mit einer Lichtdosis von 10.5J/cm² eine Abnahme der KBE/ml und somit eine Inaktivierung von *E. coli* und S. *aureus.*

Die Effektivität der Phototoxizität gegenüber Bakterien nach Bestrahlung wurde nach folgenden Richtlinen für Handhygiene im Gesundheitswesen festgelegt (Boyce, J. M., and D. Pittet. 2002. Guideline for Hand Hygiene in Health-Care Settings: recommendations of the Healthcare Infection Control Practices Advisory Committee and the HICPAC/SHEA/APIC/IDSA Hand Hygiene Task Force. Infect Control Hosp Epidemiol 23:S3-40.):

| | |
|---|---|
| - Reduktion der KBE/ml um 1 log₁₀-Stufe | ≙ 90% Effektivität |
| - Reduktion der KBE/ml um 3 log₁₀-Stufen | ≙ 99,9% Effektivität |
| - Reduktion der KBE/ml um 5 log₁₀-Stufen | ≙ 99,999% Effektivität |

Für eine effektive Inaktivierung kann daher die Abnahme von ≥3log₁₀ Stufen angenommen werden, wobei S. *aureus* und *E. coli* als Beispiele für Vertreter aus der Gruppe der Gram-positiven und Gram-negativen Bakterien ausgewählt wurden (siehe Boyce J.M and D. Pittet 2009).

Die benötigte Konzentration, um jeweils eine Reduktion um ≥3log₁₀ Stufen zu erzielen, ist in Tabelle 2 dargestellt.

**Tabelle 2: Zusammenfassung photodynamische Inaktivierung**

| **Photosensibilisator** | **Benötigte Konzentration [µM], um eine Reduktion um ≥ 3log₁₀ Stufen zu erzielen (Abnahme um 99.9%), Bestrahlung mit 10.5 J/cm²** | |
|---|---|---|
| | ***E .coli*** | **S. aureus** |
| **FL-09** | 50 | 10 |
| **FL-09b** | 1 | 1 |
| **FL-11** | 10 | 10 |
| **FL-12** | ---(*) | 100 |
| **FL-14** | 10 | 10 |
| **FL-16** | 100 | 100 |
| **FL-18** | ---(*) | 100 |
| **FL-25** | ---(*) | 100 |

| | | |
|---|---|---|
| (*) Reduktion weniger als 1log₁₀ unter den bisher untersuchten Bedingungen^ | | |

## Patentansprüche

1. Verbindung mit der Formel (1) 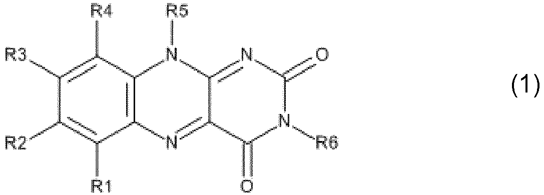 oder ein pharmakologisch verträgliches Salz und/oder Ester und/oder Komplex davon zur Anwendung als Photosensibilisator zur photodynamischen Inaktivierung von Mikroorganismen bei der photodynamischen Therapie, wobei
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und wobei jeder der Reste R5 oder R6 jeweils unabhängig voneinander gleich oder voneinander verschieden ist und Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder wobei der Rest R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und wobei R6 Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist,
und
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Ether mit 2 bis 20 C-Atomen, Thioether mit 2 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei nur der Rest R6 ein organischer Rest der allgemeinen Formel -(C(D)(E))ₕ-X oder -(C(D)(E))ₖ-Aryl-(C(D)(E))ₗ-X, ist, und wobei der Rest R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet,
und wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten.

2. Verbindung zur Anwendung nach Anspruch 1, wobei die Reste R1 und R4, die jeweils unabhängig voneinander gleich oder voneinander verschieden sein können, Wasserstoff oder Methyl sind und wobei nur 1 Rest R2, R3, R5 oder R6 ein organischer Rest der allgemeinen Formel -(C(D)(E))ₕ-X oder -(C(D)(E))ₖ-Aryl-(C(D)(E))ₗ-X ist.

3. Verbindung zur Anwendung nach einem der Ansprüche 1 oder 2, wobei X einen Rest der allgemeinen Formel (2): 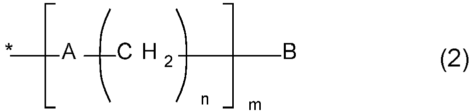 bedeutet und wobei A ein Sauerstoff- oder ein Schwefelatom ist und wobei n eine ganze Zahl von 1 bis 8 und m eine ganze Zahl von 0 bis 100 ist und wobei B ein Rest der Formel (3), (4a), (4b), (5a) oder (5b): 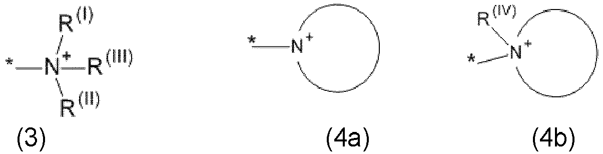 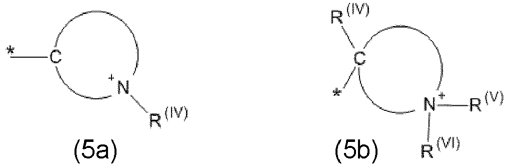 ist und wobei jeder der Reste R^{(I)}, R^{(II)}, R^{(III)}, R^{(IV)} und R^{(V)} unabhängig voneinander ein C1 - C20 Aryl, ein C1 - C20 Alkyl, das geradkettig oder verzweigt sein kann,
oder ein C1 - C20 Ether ist und wobei der Rest mit der Formel (4a) und der Rest mit der Formel (5a): 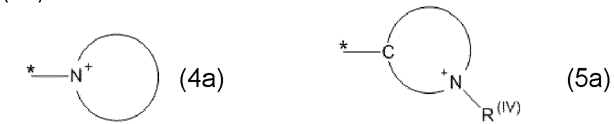 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 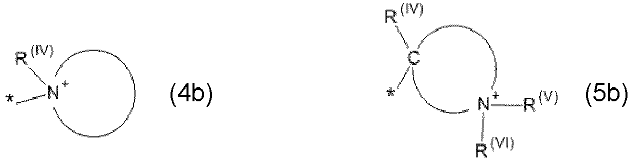 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Einfachbindung ausbildet, darstellt.

4. Verbindung zur Anwendung nach einem der Ansprüche 1 bis 3, wobei die Verbindung mit der Formel (1) aus der Gruppe, die aus Verbindungen der Formeln (30) bis (43) besteht, ausgewählt wird:

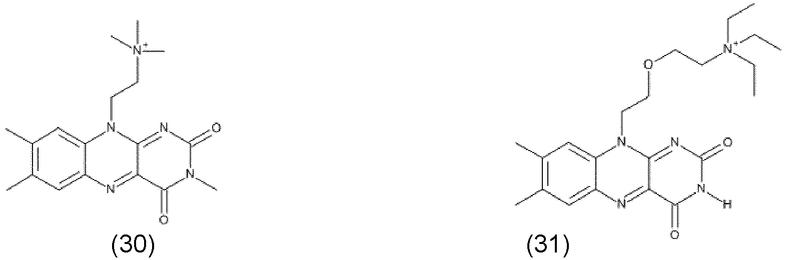

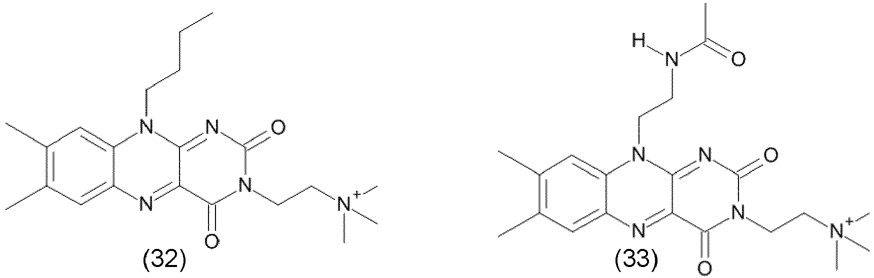

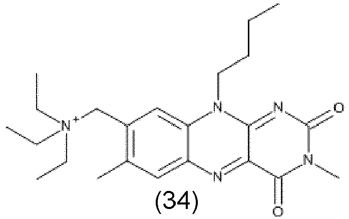

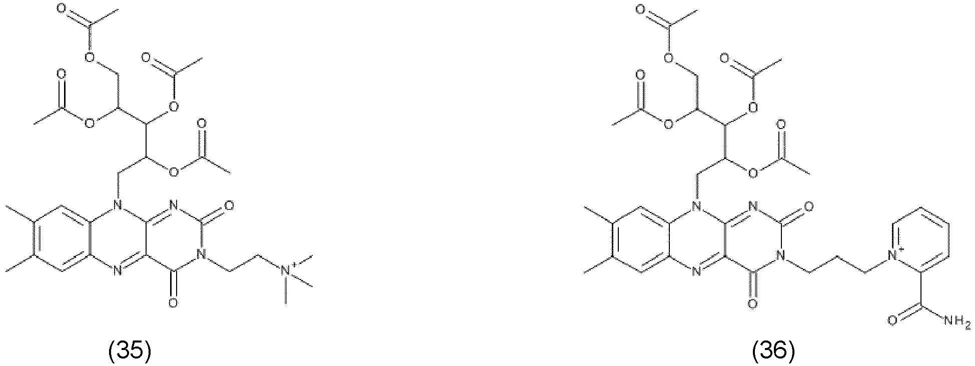

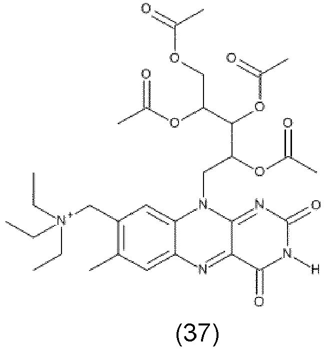

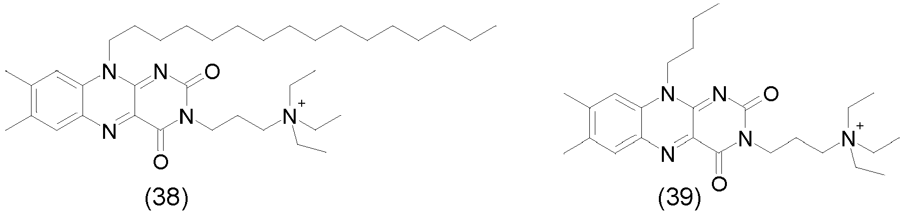

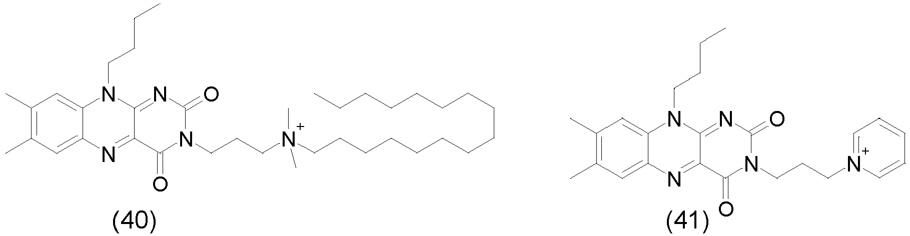

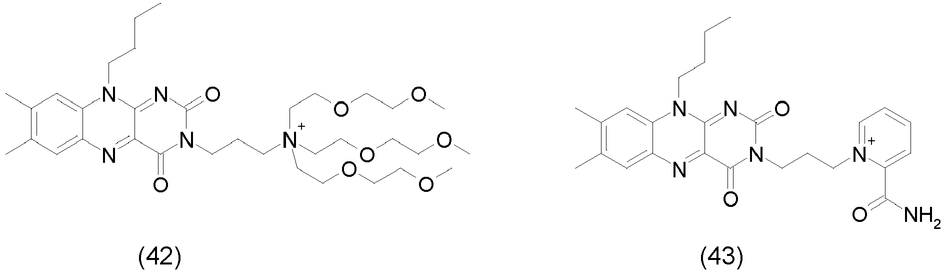

5. Pharmazeutische Zusammensetzung, umfassend wenigstens eine Verbindung gemäß Formel (1) 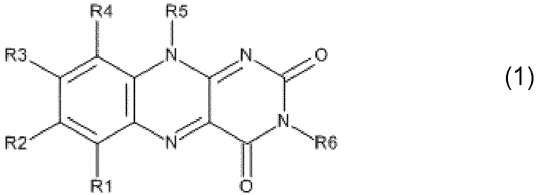 oder ein pharmakologisch verträgliches Salz und/oder Ester und/oder Komplex davon und wenigstens einen physiologisch annehmbaren Hilfsstoff, zur Anwendung als Photosensibilisator zur photodynamischen Inaktivierung von Mikroorganismen bei der photodynamischen Therapie,
wobei A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist und
wobei die Reste R1, R2, R3 oder R4, die nicht -(C(D)(E))ₕ-X oder - (C(D)(E))ₖ-Aryl-(C(D)(E))ₗ-X sind, jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten und wobei jeder der Reste R5 oder R6 jeweils unabhängig voneinander gleich oder voneinander verschieden ist und Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet, oder
wobei der Rest R5 ein nichtcyclischer Polyolrest der allgemeinen Formel - CH₂(CH(OH))_{g}CH₂OH ist oder ein Ether, Ester oder Acetal davon, wobei g eine ganze Zahl von 1 bis 10, vorzugsweise 1 bis 4, bedeutet, und wobei R6 Wasserstoff, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
oder
wobei B) nur 1 Rest R5 oder R6 ein organischer Rest der allgemeinen Formel -(C(D)(E))ₕ-X oder -(C(D)(E))ₖ-Aryl-(C(D)(E))ₗ-X, wobei h eine ganze Zahl von 1 bis 20 ist und k, l jeweils unabhängig voneinander eine ganze Zahl von 0 bis 6 bedeuten, wobei D und E jeweils unabhängig voneinander Wasserstoff, Halogen, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, bedeuten und wobei X ein organischer Rest mit nur einem quartären Stickstoffatom ist und Aryl einen substituierten oder unsubstituierten Aromat oder einen substituierten oder unsubstituierten Heteroaromat, der kein Stickstoffatom enthält, bedeutet, ist, und
wobei der Rest R5 oder R6, der nicht -(C(D)(E))ₕ-X oder -(C(D)(E))ₖ-Aryl-(C(D)(E))ₗ-X ist, Wasserstoff, Alkyl mit 1 bis 20 C-Atomen, Alkenyl mit 2 bis 20 C-Atomen, Cycloalkyl mit 3 bis 20 C-Atomen, Ether mit 2 bis 20 C-Atomen, Thioether mit 2 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei nur der Rest R6 ein organischer Rest der allgemeinen Formel -(C(D)(E))ₕ-X oder -(C(D)(E))ₖ-Aryl-(C(D)(E))ₗ-X, ist, und wobei der Rest R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet, und
wobei die Reste R1 bis R4 jeweils unabhängig voneinander gleich oder voneinander verschieden sein können und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeuten.

6. Pharmazeutische Zusammensetzung zur Anwendung nach Anspruch 5, wobei die Reste R1 und R4, die jeweils unabhängig voneinander gleich oder voneinander verschieden sein können, Wasserstoff oder Methyl sind und wobei nur 1 Rest R2, R3, R5 oder R6 ein organischer Rest der allgemeinen Formel -(C(D)(E))ₕ-X oder - (C(D)(E))ₖ-Aryl-(C(D)(E))ₗ-X ist.

7. Pharmazeutische Zusammensetzung zur Anwendung nach einem der Ansprüche 5 oder 6, wobei X einen Rest der allgemeinen Formel (2): 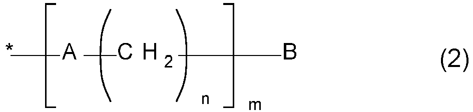 bedeutet und wobei A ein Sauerstoff- oder ein Schwefelatom ist und wobei n eine ganze Zahl von 1 bis 8 und m eine ganze Zahl von 0 bis 100 ist und wobei B ein Rest der Formel (3), (4a), (4b), (5a) oder (5b): 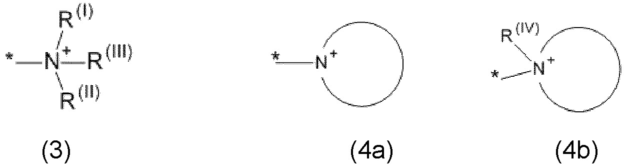 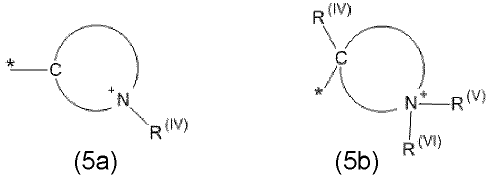 ist und wobei jeder der Reste R^{(I)}, R^{(II)}, R^{(III)}, R^{(IV)} und R^{(V)} unabhängig voneinander ein C1 - C20 Aryl, ein C1 - C20 Alkyl, das geradkettig oder verzweigt sein kann, oder ein C1 - C20 Ether ist und wobei der Rest mit der Formel (4a) und der Rest mit der Formel (5a): 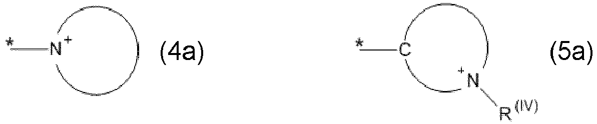 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 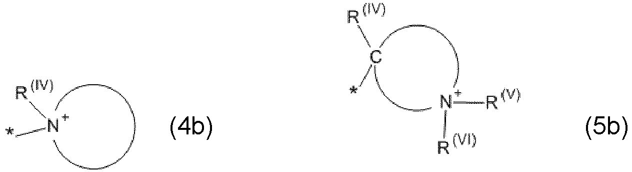 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei 1 Stickstoffatom eine Einfachbindung ausbildet, darstellt.

8. Pharmazeutische Zusammensetzung zur Anwendung nach einem der Ansprüche 5 bis 7, wobei die Verbindung mit der Formel (1) aus der Gruppe, die aus Verbindungen der Formeln (30) bis (43) besteht, ausgewählt wird

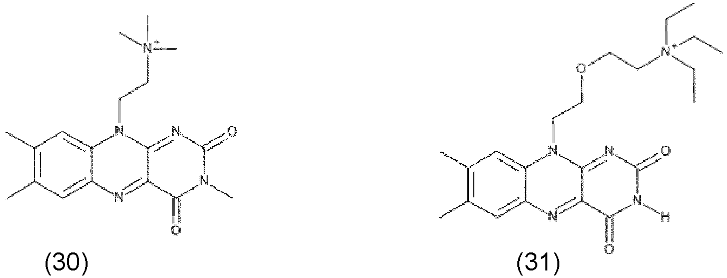

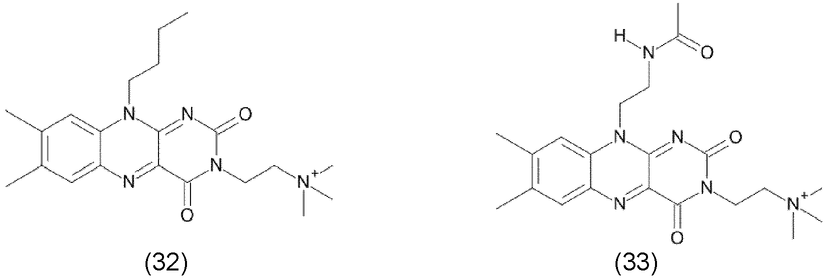

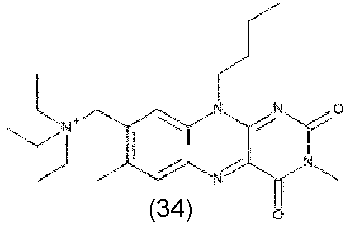

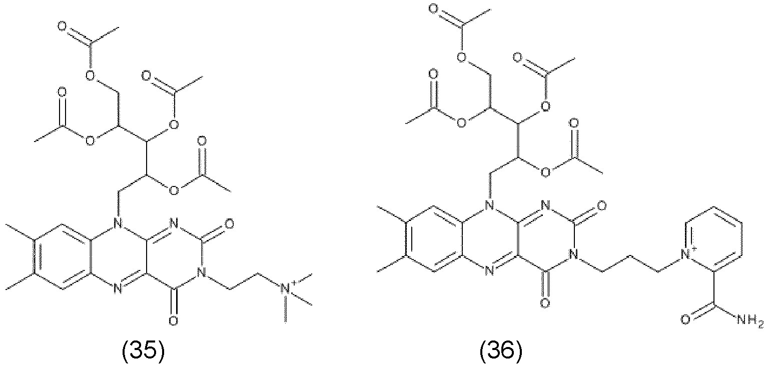

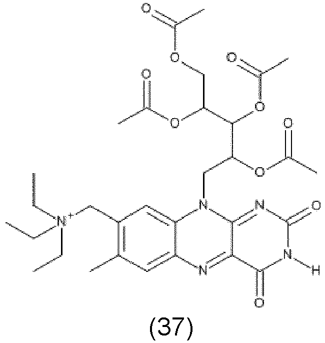

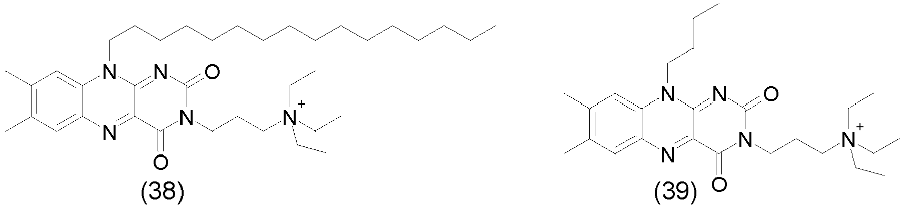

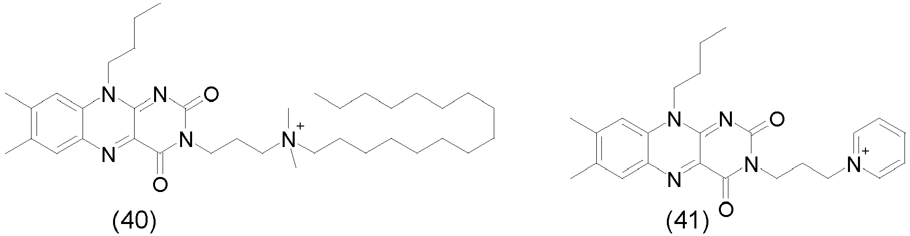

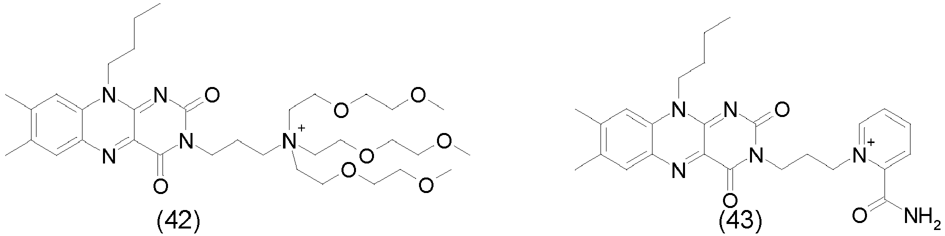

9. Pharmazeutische Zusammensetzung zur Anwendung nach einem der Ansprüche 5 bis 8, wobei der physiologisch annehmbare Hilfsstoff aus gewählt ist aus der Gruppe, die aus pharmazeutisch üblichen flüssigen oder festen Füllstoffen und Streckmittel, Lösemittel, Emulgatoren, Gleitstoffe, Geschmackskorrigentien, Farbstoffe und/oder Puffersubstanzen.

10. Verbindung zur Anwendung nach einem der Ansprüche 1 bis 4 oder pharmazeutische Zusammensetzung zur Anwendung nach Anspruch 5 bis 9, wobei die Mikroorganismen aus der Gruppe, die aus Viren, Archäeen, Bakterien, Bakteriensporen, Pilzen, Pilzsporen, Protozoen, Algen und blutübertragbaren Parasiten besteht, ausgewählt werden.

11. Verbindung zur Anwendung nach einem der Ansprüche 1 bis 4 oder 10 oder pharmazeutische Zusammensetzung zur Anwendung nach einem der Ansprüche 5 bis 10, bei der Desinfektion und/oder Dekolonisierung von Körperoberflächen, vorzugsweise Haut oder Schleimhaut, eines Säugetiers, vorzugsweise Mensch.

12. Verbindung zur Anwendung nach einem der Ansprüche 1 bis 4, 10 oder 11 oder pharmazeutische Zusammensetzung zur Anwendung nach einem der Ansprüche 5 bis 11, zur topischen, vorzugsweise nasalen, oralen, analen, vaginalen oder dermalen, Applikation.

13. Verbindung zur Anwendung nach einem der Ansprüche 1 bis 4, 10, 11 oder 12 oder pharmazeutische Zusammensetzung zur Anwendung nach einem der Ansprüche 5 bis 12, bei der Prophylaxe und/oder Behandlung einer infektiösen Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht.

14. Verbindung zur Anwendung nach einem der Ansprüche 1 bis 4, 10, 11 oder 12 oder pharmazeutische Zusammensetzung zur Anwendung nach einem der Ansprüche 5 bis 12, bei der Desinfektion und/oder Reduktion der Keimzahl in infizierten Wunden.

15. Verbindung zur Anwendung nach einem der Ansprüche 1 bis 4, 10, 11 oder 12 oder pharmazeutische Zusammensetzung zur Anwendung nach einem der Ansprüche 5 bis 12, bei der Prophylaxe und/oder Behandlung von infektiösen Erkrankungen des Ohrs, der oberen Luftwege, der Mundhöhle, des Rachens, des Kehlkopfes, der unteren Luftwege und/oder der Speiseröhre.

16. Verbindung zur Anwendung nach einem der Ansprüche 1 bis 4, 10, 11 oder 12 oder pharmazeutische Zusammensetzung zur Anwendung nach einem der Ansprüche 5 bis 12, bei der Behandlung und/oder Prophylaxe einer infektiösen des Zahngewebes, vorzugsweise Plaque, Karies oder Pulpitis, und/oder infektiösen Erkrankung des Zahnhalteapparates, vorzugsweise Gingivitis, Paradontitis, Endodontitis oder Periimplantitis.

## Claims

1. Compound having the formula (1) 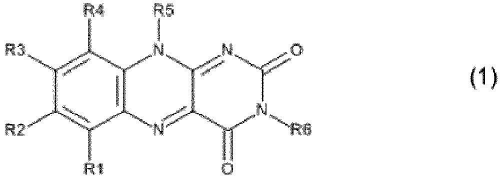 or a pharmacologically compatible salt and/or ester and/or complex thereof for use as photosensitizer for photodynamic inactivation of microorganisms in photodynamic therapy, wherein
A) only 1 radical R1, R2, R3 or R4 is an organic radical having the general formula -(C(D)(E))ₕ-X or -(C(D)(E))ₖ-aryl-(C(D)(E))ₗ-X, wherein h is an integer from 1 to 20 and k, 1 are each independently an integer from 0 to 6, wherein D and E are - independently of one another - hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom, and aryl is a substituted or unsubstituted aromatic, or a substituted or unsubstituted heteroaromatic, containing no nitrogen atom, and
wherein the radicals R1, R2, R3 or R4 which are not - (C(D)(E))ₕ-X or -(C(D)(E))ₖ-aryl-(C(D)(E))ₗ-X may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ether having 2 to 20 C atoms, thioether having 2 to 20 C atoms, carboxylic acid ester having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms, and wherein each of the radicals R5 or R6 is, independently of one another, identical to or different from one another, and is hydrogen, alkyl having 1 to 20 C atoms, alkenyl having 1 to 20 C atoms, ether having 2 to 20 C atoms, thioether having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms, or wherein the radical R5 is a non-cyclic polyol radical having the general formula -CH₂(CH(OH))_{g}CH₂OH or an ether, ester or acetal thereof, wherein g is an integer from 1 to 10, preferably 1 to 4, and wherein R6 is hydrogen, alkyl having 1 to 20 C atoms, alkenyl having 1 to 20 C atoms, ether having 2 to 20 C atoms, thioether having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms,
or
wherein B) only 1 radical R5 or R6 is an organic radical having the general formula -(C(D)(E))ₕ-X or -(C(D)(E))ₖ-aryl-(C(D)(E))ₗ-X, wherein h is an integer from 1 to 20 and k, 1 are independently in each case an integer from 0 to 6, wherein D and E are each independently hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom and aryl is a substituted or unsubstituted aromatic or a substituted or unsubstituted heteroaromatic, not containing any nitrogen atom, and
wherein the radical R5 or R6 which is not -(C(D)(E))ₕ-X or -(C(D)(E))ₖ-aryl-(C(D)(E))ₗ-X is hydrogen, alkyl having 1 to 20 C atoms, alkenyl having 2 to 20 C atoms, cycloalkyl having 3 to 20 C atoms, ether having 2 to 20 C atoms, thioether having 2 to 20 C atoms, cycloalkenyl having 3 to 20 C atoms, aryl having 5 to 20 C atoms or heteroaryl having 4 to 20 C atoms, not containing any N atoms, or
wherein the radical R6 only is an organic radical having the general formula -(C(D)(E))ₕ-X or -(C(D)(E))ₖ-aryl-(C(D)(E))ₗ-X, and wherein the radical R5 is a non-cyclic polyol radical having the general formula -CH₂(CH(OH))_{g}CH₂OH or an ether, ester or acetal thereof, wherein g is an integer from 1 to 10, preferably 1 to 4,
and wherein the radicals R1 to R4 may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ether having 2 to 20 C atoms, thioether having 2 to 20 C atoms, carboxylic acid ester having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms.

2. Compound for use according to Claim 1, wherein the radicals R1 and R4, each of which may be identical to or different from one another, are hydrogen or methyl, and wherein only 1 radical R2, R3, R5 or R6 is an organic radical having the general formula -(C(D)(E))ₕ-X or -(C(D)(E))ₖ-aryl-(C(D)(E))ₗ-X.

3. Compound for use according to either of Claims 1 and 2, wherein X is a radical having the general formula (2): 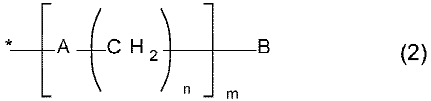 and wherein A is an oxygen or a sulfur atom and wherein n is an integer from 1 to 8, and m is an integer from 0 to 100, and wherein B is a radical having the formula (3), (4a), (4b), (5a) or (5b): 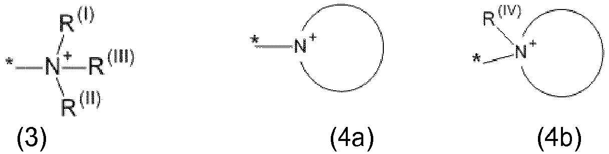 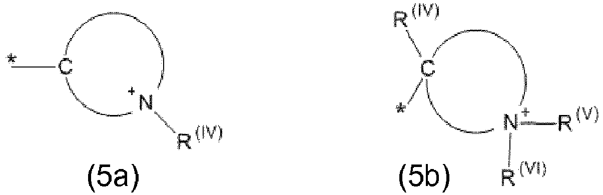 and wherein each of the radicals R^{(I)}, R^{(II)}, R^{(III)}, R^{(IV)} and R^{(V)} is, independently of one another, a C1-C20 aryl, a C1-C20 alkyl which may be straight-chain or branched,
or a C1-C20 ether, and wherein the radical having the formula (4a) and the radical having the formula (5a): 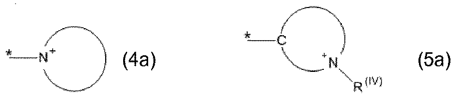 represents a substituted or unsubstituted heterocyclic radical having from 5 to 7 ring atoms, comprising at least one 1 carbon atom and 1 nitrogen atom, and optionally 1 or 2 oxygen or sulfur atoms, wherein 1 nitrogen atom forms a double bond, and wherein the radical having the formula (4b) and the radical having the formula (5b): 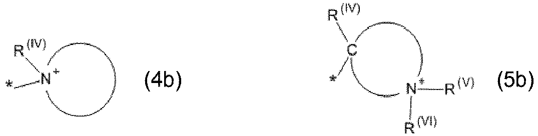 represents a substituted or unsubstituted heterocyclic radical having from 5 to 7 ring atoms, comprising at least one 1 carbon atom and 1 nitrogen atom, and optionally 1 or 2 oxygen or sulfur atoms, wherein 1 nitrogen atom forms a single bond.

4. Compound for use according to any one of Claims 1 to 3, wherein the compound having the formula (1) is selected from the group consisting of compounds having the formulas (30) to (43):

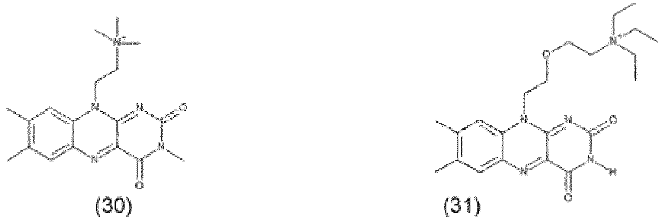

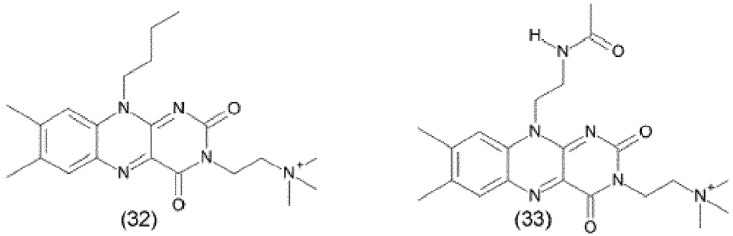

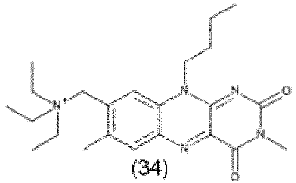

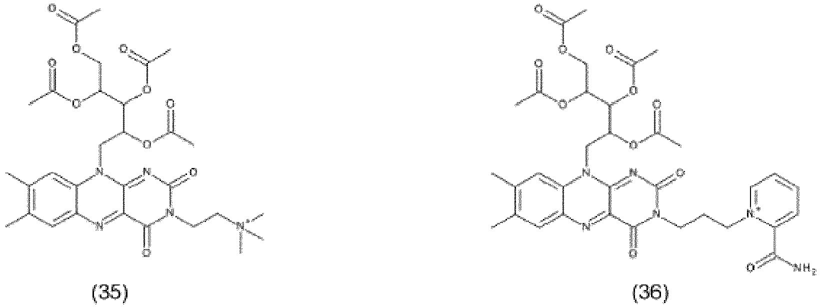

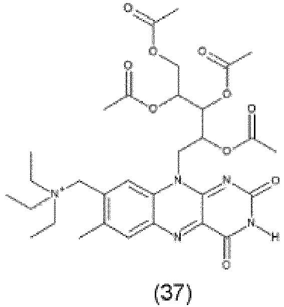

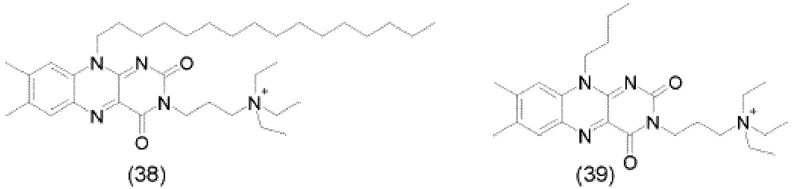

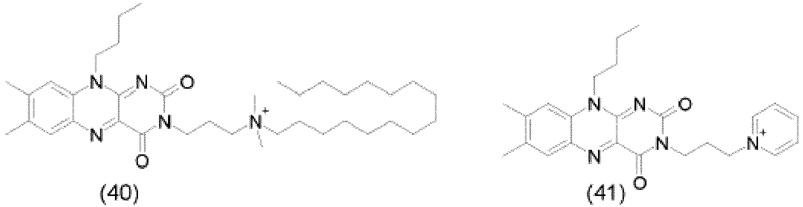

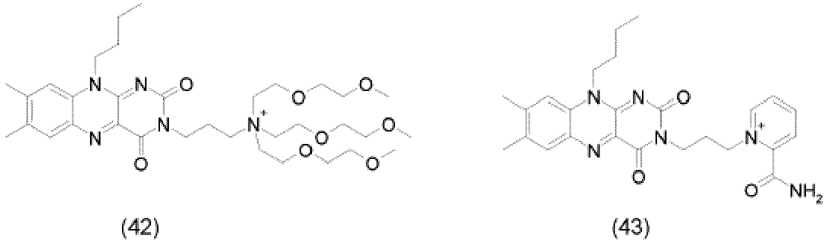

5. Pharmaceutical composition comprising at least one compound of formula (1) 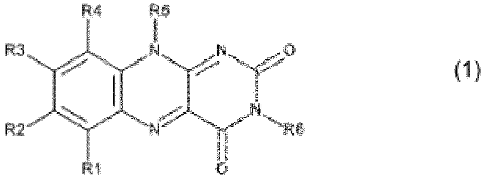 or a pharmacologically compatible salt and/or ester and/or complex thereof and at least one physiologically acceptable excipient, for use as photosensitizer for photodynamic inactivation of microorganisms in photodynamic therapy,
wherein A) only 1 radical R1, R2, R3 or R4 is an organic radical having the general formula —(C(D)(E))ₕ-X or -(C(D)(E))ₖ-aryl-(C(D)(E))ₗ-X, wherein h is an integer from 1 to 20 and k, 1 are each independently an integer from 0 to 6, wherein D and E are - independently of one another - hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom, and aryl is a substituted or unsubstituted aromatic, or a substituted or unsubstituted heteroaromatic, containing no nitrogen atom, and
wherein the radicals R1, R2, R3 or R4 which are not - (C(D)(E))ₕ-X or -(C(D)(E))ₖ-aryl-(C(D)(E))ₗ-X may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ether having 2 to 20 C atoms, thioether having 2 to 20 C atoms, carboxylic acid ester having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms, and wherein each of the radicals R5 or R6 is, independently of one another, identical to or different from one another, and is hydrogen, alkyl having 1 to 20 C atoms, alkenyl having 1 to 20 C atoms, ether having 2 to 20 C atoms, thioether having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms, or
wherein the radical R5 is a non-cyclic polyol radical having the general formula -CH₂(CH(OH))_{g}CH₂OH or an ether, ester or acetal thereof, wherein g is an integer from 1 to 10, preferably 1 to 4, and wherein R6 is hydrogen, alkyl having 1 to 20 C atoms, alkenyl having 1 to 20 C atoms, ether having 2 to 20 C atoms, thioether having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms,
or
wherein B) only 1 radical R5 or R6 is an organic radical having the general formula -(C(D)(E))ₕ-X or - (C(D)(E))ₖ-aryl-(C(D)(E))ₗ-X, wherein h is an integer from 1 to 20 and k, 1 are independently in each case an integer from 0 to 6, wherein D and E are each independently hydrogen, halogen, hydroxyl, O-R^{(VIII)}, wherein R^{(VIII)} is methyl, ethyl, n-propyl, n-butyl, phenyl or benzyl, O-C(=O)-R^{(IX)}, wherein R^{(IX)} is hydrogen, methyl, ethyl, n-propyl, n-butyl, phenyl or benzyl, or thiol, and wherein X is an organic radical having only one quaternary nitrogen atom and aryl is a substituted or unsubstituted aromatic or a substituted or unsubstituted heteroaromatic, not containing any nitrogen atom, and
wherein the radical R5 or R6 which is not - (C(D)(E))ₕ-X or -(C(D)(E))ₖ-aryl-(C(D)(E))ₗ-X is hydrogen, alkyl having 1 to 20 C atoms, alkenyl having 2 to 20 C atoms, cycloalkyl having 3 to 20 C atoms, ether having 2 to 20 C atoms, thioether having 2 to 20 C atoms, cycloalkenyl having 3 to 20 C atoms, aryl having 5 to 20 C atoms or heteroaryl having 4 to 20 C atoms, not containing any N atoms, or
wherein the radical R6 only is an organic radical having the general formula -(C(D)(E))ₕ-X or -(C(D)(E))ₖ-aryl-(C(D)(E))ₗ-X, and wherein the radical R5 is a non-cyclic polyol radical having the general formula -CH₂(CH(OH))_{g}CH₂OH or an ether, ester or acetal thereof, wherein g is an integer from 1 to 10, preferably 1 to 4, and
wherein the radicals R1 to R4 may, independently of one another, be identical to or different from one another, and are hydrogen, halogen, hydroxyl, thiol, nitro, carboxylate, aldehyde having 1 to 20 C atoms, ketone having 2 to 20 C atoms, O-alkyl having 1 to 20 C atoms, S-alkyl having 1 to 20 C atoms, O-alkenyl having 2 to 20 C atoms, S-alkenyl having 2 to 20 C atoms, O-aryl having 5 to 20 C atoms, S-aryl having 5 to 20 C atoms, ether having 2 to 20 C atoms, thioether having 2 to 20 C atoms, carboxylic acid ester having 1 to 20 C atoms, carboxylic acid amide having 1 to 20 C atoms, thioester having 1 to 20 C atoms, alkyl having 1 to 20 C atoms, alkenyl having 2 to 20 C atoms, cycloalkyl having 3 to 20 C atoms, cycloalkenyl having 3 to 20 C atoms, aryl having 5 to 20 C atoms or heteroaryl having 4 to 20 C atoms, not containing any N atoms.

6. Pharmaceutical composition for use according to Claim 5, wherein the radicals R1 and R4, each of which may be identical to or different from one another, are hydrogen or methyl, and wherein only 1 radical R2, R3, R5 or R6 is an organic radical having the general formula -(C(D)(E))ₕ-X or -(C(D)(E))ₖ-aryl-(C(D)(E))ₗ-X.

7. Pharmaceutical composition for use according to one of Claims 5 and 6, wherein X is a radical having the general formula (2): 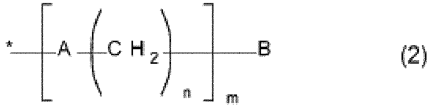 and wherein A is an oxygen or a sulfur atom and wherein n is an integer from 1 to 8, and m is an integer from 0 to 100, and wherein B is a radical having the formula (3), (4a), (4b), (5a) or (5b): 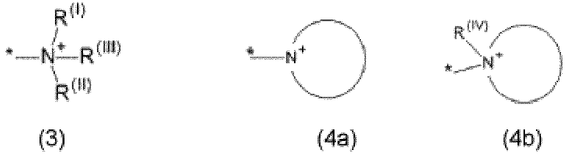 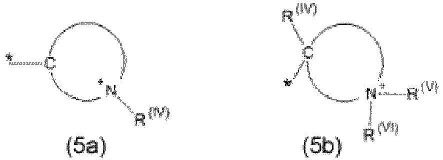 and wherein each of the radicals R^{(I)}, R^{(II)}, R^{(III)}, R^{(IV)} and R^{(V)} is, independently of one another, a C1-C20 aryl, a C1-C20 alkyl which may be straight-chain or branched, or a C1-C20 ether, and wherein the radical having the formula (4a) and the radical having the formula (5a): 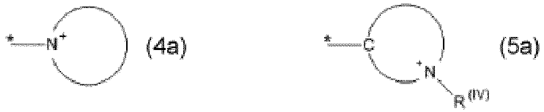 represents a substituted or unsubstituted heterocyclic radical having from 5 to 7 ring atoms, comprising at least one 1 carbon atom and 1 nitrogen atom, and optionally 1 or 2 oxygen or sulfur atoms, wherein 1 nitrogen atom forms a double bond, and wherein the radical having the formula (4b) and the radical having the formula (5b): 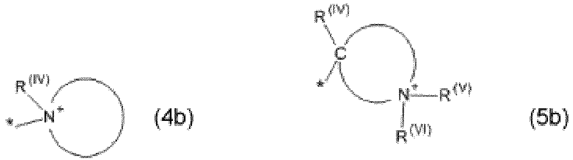 represents a substituted or unsubstituted heterocyclic radical having from 5 to 7 ring atoms, comprising at least one 1 carbon atom and 1 nitrogen atom, and optionally 1 or 2 oxygen or sulfur atoms, wherein 1 nitrogen atom forms a single bond.

8. Pharmaceutical composition for use according to any one of Claims 5 to 7, wherein the compound having the formula (1) is selected from the group consisting of compounds having the formulas (30) to (43):

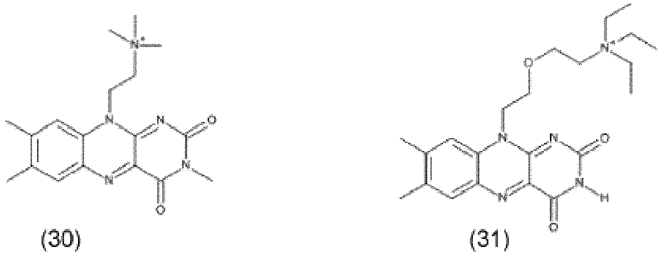

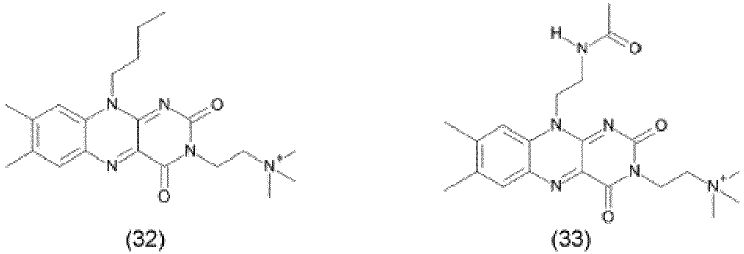

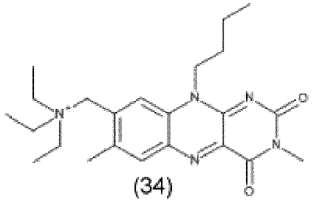

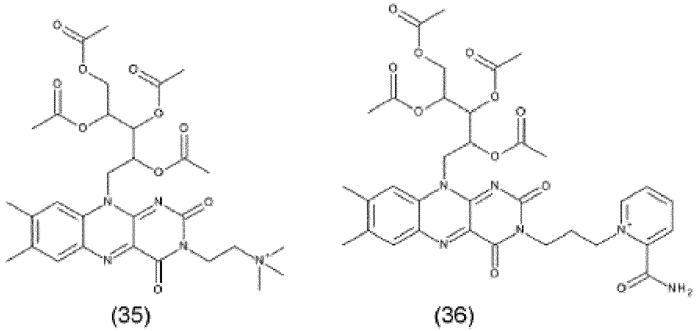

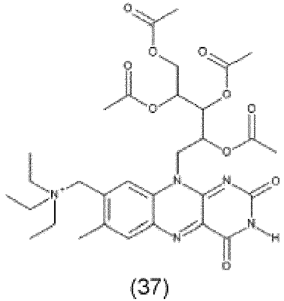

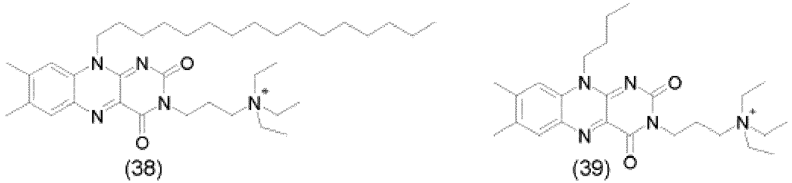

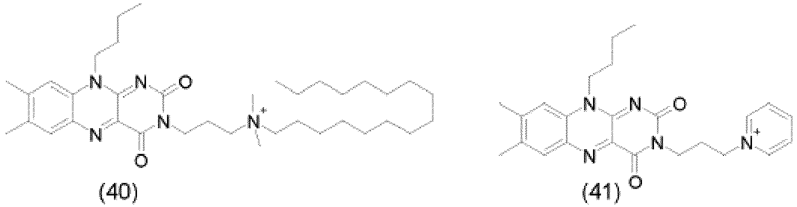

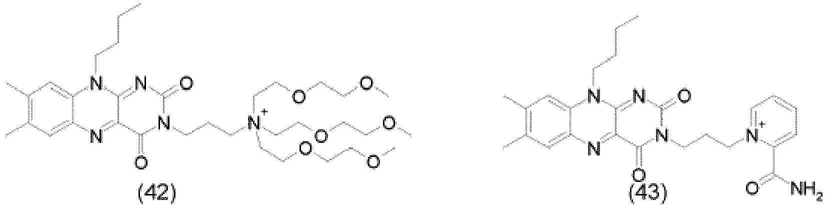

9. Pharmaceutical composition for use according to any one of Claims 5 to 8, wherein the physiologically acceptable excipient is selected from the group which of pharmaceutically customary liquid or solid fillers and extenders, solvents, emulsifiers, glidants, flavour correctors, dyes and/or buffer substances.

10. Compound for use according to any one of Claims 1 to 4 or pharmaceutical composition for use according to Claim 5 to 9, wherein the microorganisms are selected from the group consisting of viruses, archaea, bacteria, bacterial spores, fungi, fungal spores, protozoa, algae and blood-transmissible parasites.

11. Compound for use according to any one of Claims 1 to 4 or 10 or pharmaceutical composition for use according to any one of Claims 5 to 10, in the disinfection and/or decolonization of surfaces of the body, preferably skin or mucosa, of a mammal, preferably human.

12. Compound for use according to any one of Claims 1 to 4, 10 or 11 or pharmaceutical composition for use according to any one of Claims 5 to 11, for topical, preferably nasal, oral, anal, vaginal or dermal, administration.

13. Compound for use according to any one of Claims 1 to 4, 10, 11 or 12 or pharmaceutical composition for use according to any one of Claims 5 to 12, in the prophylaxis and/or treatment of an infectious skin disease which is preferably selected from the group consisting of staphylococcal scalded skin syndrome, impetigo, skin abscess, furuncle, carbuncle, phlegmon, cellulitis, acute lymphadenitis, pilonidal cysts, pyoderma, purulent dermatitis, septic dermatitis, suppurative dermatitis, erythrasma, erysipelas, acne vulgaris and fungal infection.

14. Compound for use according to any one of Claims 1 to 4, 10, 11 or 12 or pharmaceutical composition for use according to any one of Claims 5 to 12, in the disinfection and/or reduction of the microbe count in infected wounds.

15. Compound for use according to any one of Claims 1 to 4, 10, 11 or 12 or pharmaceutical composition for use according to any one of Claims 5 to 12, in the prophylaxis and/or treatment of infectious disorders of the ear, of the upper respiratory pathway, of the oral cavity, of the throat, of the larynx, of the lower respiratory pathway and/or of the oesophagus.

16. Compound for use according to any one of Claims 1 to 4, 10, 11 or 12 or pharmaceutical composition for use according to any one of Claims 5 to 12, in the treatment and/or prophylaxis of an infectious of the dental tissue, preferably plaque, caries or pulpitis, and/or infectious disorder of the periodontium, preferably gingivitis, paradontitis, endodontitis or periimplantitis.

## Revendications

1. Composé de Formule (1) 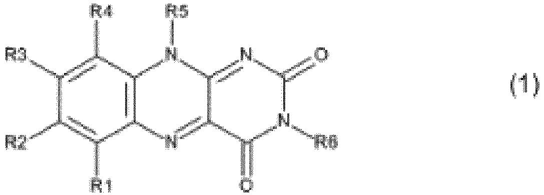 ou sel et/ou ester et/ou complexe de celui-ci pharmacologiquement compatible pour une utilisation comme photosensibilisateur pour l'inactivation photodynamique de microorganismes dans le cadre d'une thérapie photodynamique, dans lequel
A) seul un radical R1, R2, R3 ou R4 est un radical organique de Formule générale -(C(D)(E))ₕ-X ou -(C(D)(E))ₖ-aryl-(C(D)(E))ₗ-X, h représentant un nombre entier de 1 à 20 et k, l représentant chacun indépendamment de l'autre un nombre entier de 0 à 6, D et E représentant chacun indépendamment de l'autre un hydrogène, un halogène, un hydroxyle, O-R^{(VIII)}, R^{(VIII)} représentant un méthyle, un éthyle, un n-propyle, un n-butyle, un phényle ou un benzyle, O-C(=O)-R^{(IX)}, R^{(IX)} représentant un hydrogène, un méthyle, un éthyle, un n-propyle, un n-butyle, un phényle ou un benzyle, ou un thiol, et X représentant un radical organique n'ayant qu'un atome d'azote quaternaire et aryle représentant un radical aromatique substitué ou non substitué ou un radical hétéroaromatique substitué ou non substitué ne contenant aucun atome d'azote, et
dans lequel les radicaux R1, R2, R3 ou R4 qui ne sont pas -(C(D)(E))ₕ-X ou -(C(D)(E))ₖ-aryl-(C(D)(E))ₗ-X, peuvent chacun indépendamment des autres être identiques ou différents, et représentent chacun un hydrogène, un halogène, un hydroxyle, un thiol, un nitro, un carboxylate, un aldéhyde ayant 1 à 20 atomes de carbone, une cétone ayant 2 à 20 atomes de carbone, un O-alkyle ayant 1 à 20 atomes de carbone, un S-alkyle ayant 1 à 20 atomes de carbone, un O-alcényle ayant 2 à 20 atomes de carbone, un S-alcényle ayant 2 à 20 atomes de carbone, un O-aryle ayant 5 à 20 atomes de carbone, un S-aryle ayant 5 à 20 atomes de carbone, un éther ayant 2 à 20 atomes de carbone, un thioéther ayant 2 à 20 atomes de carbone, un ester d'acide carboxylique ayant 1 à 20 atomes de carbone, un carboxamide ayant 1 à 20 atomes de carbone, un thioester ayant 1 à 20 atomes de carbone, un alkyle ayant 1 à 20 atomes de carbone, un alcényle ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N, et chacun des radicaux R5 ou R6 étant, chacun indépendamment de l'autre, identique ou différent, et représentant un hydrogène, un alkyle ayant 1 à 20 atomes de carbone, un alcényle ayant 1 à 20 atomes de carbone, un éther ayant 2 à 20 atomes de carbone, un thioéther ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N, ou le radical R5 représentant un radical polyol acyclique de Formule générale -CH₂(CH(OH))_{g}CH₂OH ou un éther, un ester ou un acétal de celui-ci, g représentant un nombre entier de 1 à 10, de préférence de 1 à 4, et R6 représentant un hydrogène, un alkyle ayant 1 à 20 atomes de carbone, un alcényle ayant 1 à 20 atomes de carbone, un éther ayant 2 à 20 atomes de carbone, un thioéther ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N,
ou dans lequel
B) seul un radical R5 ou R6 représente un radical organique de Formule générale -(C(D)(E))ₕ-X ou -(C(D)(E))ₖ-aryl-(C(D)(E))ₗ-X, dans lequel h représente un nombre entier de 1 à 20 et k, l représentent chacun indépendamment de l'autre un nombre entier de 0 à 6, D et E représentent chacun indépendamment de l'autre un hydrogène, un halogène, un hydroxyle, O-R^{(VIII)}, dans lequel R^{(VIII)} représente un méthyle, un éthyle, un n-propyle, un n-butyle, un phényle ou un benzyle, O-C(=O)-R^{(IX)}, dans lequel R^{(IX)} représente un hydrogène, un méthyle, un éthyle, un n-propyle, un n-butyle, un phényle ou un benzyle, ou un thiol, et X représente un radical organique ayant un seul atome d'azote quaternaire, et aryle représentant un radical aromatique substitué ou non substitué ou un radical hétéroaromatique substitué ou non substitué, qui ne contient aucun atome d'azote,
et
dans lequel le radical R5 ou R6 qui n'est pas -(C(D)(E))ₕ-X ou -(C(D)(E))ₖ-aryl-(C(D)(E))ₗ-X, représente un hydrogène, un alkyle ayant 1 à 20 atomes de carbone, un alcényle ayant 2 à 20 atomes de carbone, un cycloalkyle ayant 3 à 20 atomes de carbone, un éther ayant 2 à 20 atomes de carbone, un thioéther ayant 2 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N,
ou
dans lequel seul le radical R6 représente un radical organique de Formule générale -(C(D)(E))ₕ-X ou -(C(D)(E))ₖ-aryl-(C(D)(E))ₗ-X, et le radical R5 représentant un radical polyol acyclique de Formule générale -CH₂(CH(OH))_{g}CH₂OH ou un éther, un ester ou un acétal de celui-ci, g représentant un nombre entier de 1 à 10, de préférence de 1 à 4,
et dans lequel les radicaux R1 à R4 peuvent chacun indépendamment des autres être identiques ou différents, et représentant un hydrogène, un halogène, un hydroxyle, un thiol, un nitro, un carboxylate, un aldéhyde ayant 1 à 20 atomes de carbone, une cétone ayant 2 à 20 atomes de carbone, un O-alkyle ayant 1 à 20 atomes de carbone, un S-alkyle ayant 1 à 20 atomes de carbone, un O-alcényle ayant 2 à 20 atomes de carbone, un S-alcényle ayant 2 à 20 atomes de carbone, un O-aryle ayant 5 à 20 atomes de carbone, un S-aryle ayant 5 à 20 atomes de carbone, un éther ayant 2 à 20 atomes de carbone, un thioéther ayant 2 à 20 atomes de carbone, un ester d'acide carboxylique ayant 1 à 20 atomes de carbone, un carboxamide ayant 1 à 20 atomes de carbone, un thioester ayant 1 à 20 atomes de carbone, un alkyle ayant 1 à 20 atomes de carbone, un alcényle ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N.

2. Composé pour une utilisation selon la revendication 1, dans lequel les radicaux R1 et R4, qui peuvent chacun indépendamment de l'autre être identiques ou différents, représentent chacun un hydrogène ou un méthyle, et seul un radical R2, R3, R5 ou R6 représentant un radical organique de Formule générale - (C(D)(E))ₕ-X ou -(C(D)(E))ₖ-aryl-(C(D)(E))ₗ-X.

3. Composé pour une utilisation selon l'une des revendications 1 ou 2, X représentant un radical de Formule générale (2) : 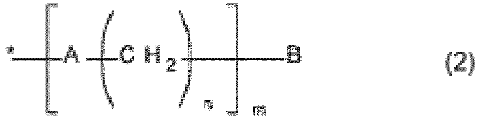 et A représentant un atome d'oxygène ou d'azote, et N représentant un nombre entier de 1 à 8 et m représentant un nombre entier de 0 à 100, et B représentant un radical de Formule (3), (4a), (4b), (5a) ou (5b) : 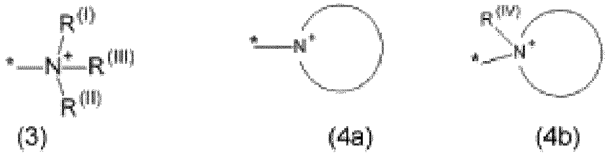 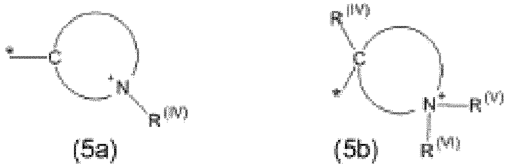 et chacun des radicaux R ^{(I)}, R ^{(II)}, R ^{(III)}, R ^{(IV)} et R ^{(V)} représentant chacun indépendamment des autres un aryle en C1 à C20, un alkyle en C1 à C20, qui peut être à chaîne droite ou ramifiée,
ou représente un éther en C1 à C20, et le radical de Formule (4a) et le radical de Formule (5a) : 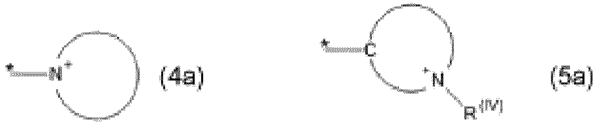 représentant un radical hétérocyclique substitué ou non substitué ayant 5 à 7 atomes nucléaires, qui comprennent au moins 1 atome de carbone et 1 atome d'azote ainsi qu'éventuellement 1 ou 2 atomes d'oxygène ou de soufre, 1 atome d'azote constituant une double liaison, et le radical de Formule (4b) et le radical de Formule (5b) représentant : 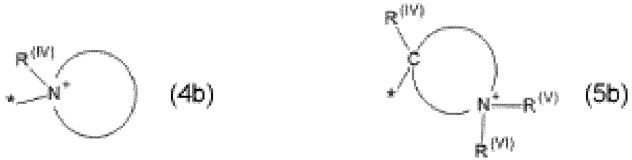 un radical hétérocyclique substitué ou non substitué ayant 5 à 7 atomes nucléaires, qui comprennent au moins 1 atome de carbone et 1 atome d'azote ainsi qu'éventuellement 1 ou 2 atomes d'oxygène ou de soufre, 1 atome d'azote formant une liaison simple.

4. Composé pour une utilisation selon l'une des revendications 1 à 3, le composé de Formule (1) étant dans le groupe constitué par les composés ayant les Formules (30) à (43) :

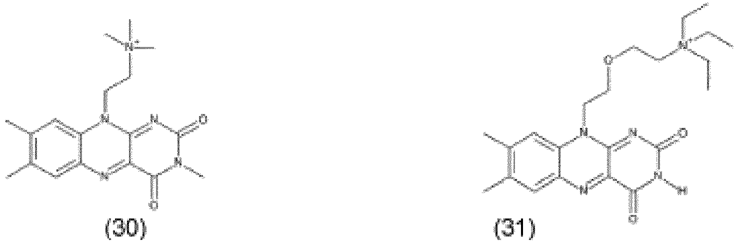

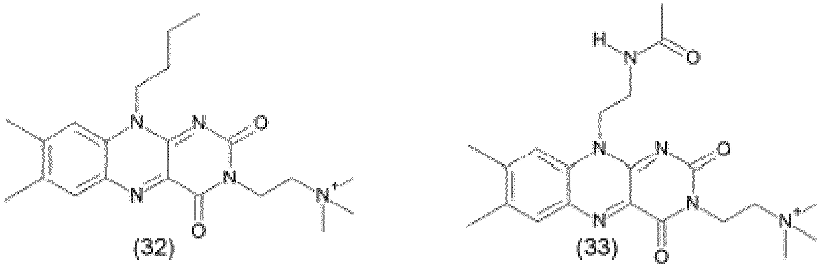

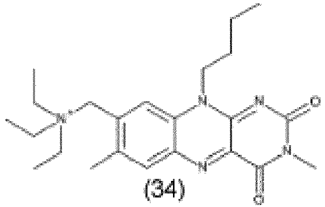

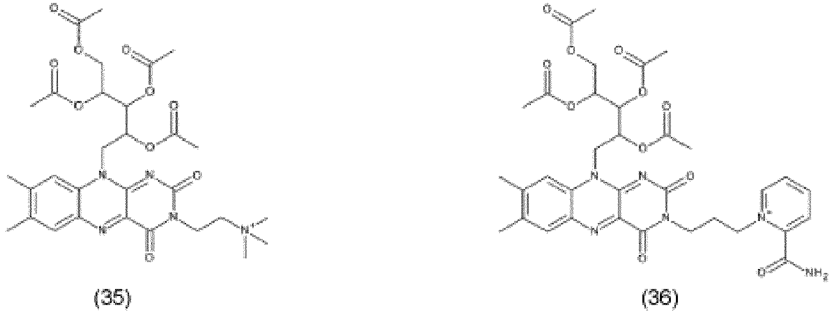

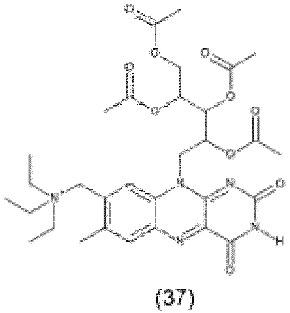

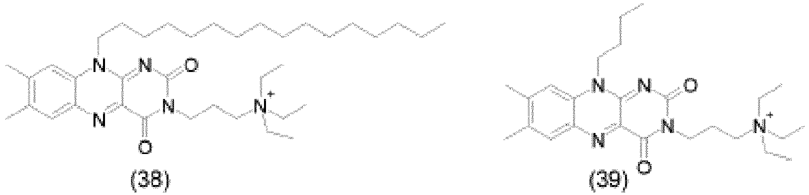

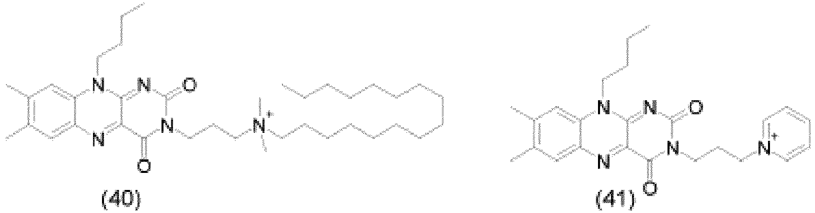

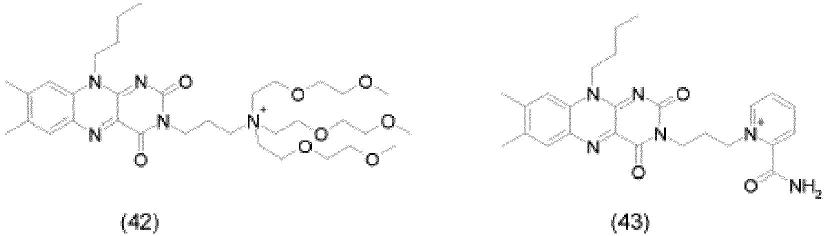

5. Composition pharmaceutique comprenant au moins un composé de Formule (1) 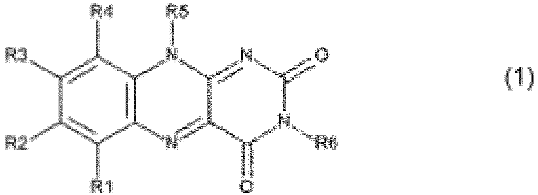 ou un sel et/ou un ester et/ou un complexe pharmacologiquement compatible de celui-ci, et au moins un adjuvant physiologiquement acceptable, pour une utilisation en tant que photosensibilisateur pour l'inactivation photodynamique de microorganismes dans le cadre d'une thérapie photodynamique,
dans lequel
A) seul un radical R1, R2, R3 ou R4 est un radical organique de Formule générale -(C(D)(E))ₕ-X ou -(C(D)(E))ₖ-aryl-(C(D)(E))ₗ-X, dans lequel h représente un nombre entier de 1 à 20 et k, l représentent chacun indépendamment de l'autre un nombre entier de 0 à 6, dans lequel D et E représente chacun indépendamment de l'autre un hydrogène, un halogène, un hydroxyle, O-R^{(VIII)}, dans lequel R^{(VIII)} représente un méthyle, un éthyle, un n-propyle, un n-butyle, un phényle ou un benzyle, O-C(=O)-R^{(IX)}, dans lequel R^{(IX)} représente un hydrogène, un méthyle, un éthyle, un n-propyle, un n-butyle, un phényle ou un benzyle, ou un thiol, et X représente un radical organique n'ayant qu'un atome d'azote quaternaire et aryle représentant un radical aromatique substitué ou non substitué ou un radical hétéroaromatique substitué ou non substitué ne contenant aucun atome d'azote, et
dans lequel les radicaux R1, R2, R3 ou R4 qui ne sont pas -(C(D)(E))ₕ-X ou -(C(D)(E))ₖ-aryl-(C(D)(E))ₗ-X, peuvent chacun indépendamment des autres être identiques ou différents, et représentant chacun un hydrogène, un halogène, un hydroxyle, un thiol, un nitro, un carboxylate, un aldéhyde ayant 1 à 20 atomes de carbone, une cétone ayant 2 à 20 atomes de carbone, un O-alkyle ayant 1 à 20 atomes de carbone, un S-alkyle ayant 1 à 20 atomes de carbone, un O-alcényle ayant 2 à 20 atomes de carbone, un S-alcényle ayant 2 à 20 atomes de carbone, un O-aryle ayant 5 à 20 atomes de carbone, un S-aryle ayant 5 à 20 atomes de carbone, un éther ayant 2 à 20 atomes de carbone, un thioéther ayant 2 à 20 atomes de carbone, un ester d'acide carboxylique ayant 1 à 20 atomes de carbone, un carboxamide ayant 1 à 20 atomes de carbone, un thioester ayant 1 à 20 atomes de carbone, un alkyle ayant 1 à 20 atomes de carbone, un alcényle ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N, et chacun des radicaux R5 ou R6 étant, chacun indépendamment de l'autre, identique ou différent, et représentant un hydrogène, un alkyle ayant 1 à 20 atomes de carbone, un alcényle ayant 1 à 20 atomes de carbone, un éther ayant 2 à 20 atomes de carbone, un thioéther ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N, ou le radical R5 représentant un radical polyol acyclique de Formule générale -CH₂(CH(OH))_{g}CH₂OH ou un éther, un ester ou un acétal de celui-ci, g représentant un nombre entier de 1 à 10, de préférence de 1 à 4, et R6 représentant un hydrogène, un alkyle ayant 1 à 20 atomes de carbone, un alcényle ayant 1 à 20 atomes de carbone, un éther ayant 2 à 20 atomes de carbone, un thioéther ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N,
ou
dans lequel B) un seul radical R5 ou R6 représente un radical organique de Formule générale -(C(D)(E))ₕ-X ou -(C(D)(E))ₖ-aryl-(C(D)(E))ₗ-X, dans lequel h représente un nombre entier de 1 à 20 et k, l représentent chacun indépendamment de l'autre un nombre entier de 0 à 6, D et E représentant chacun indépendamment de l'autre un hydrogène, un halogène, un hydroxyle, O-R^{(VIII)}, dans lequel R^{(VIII)} représente un méthyle, un éthyle, un n-propyle, un n-butyle, un phényle ou un benzyle, O-C(=O)-R^{(IX)}, dans lequel R^{(IX)} représente un hydrogène, un méthyle, un éthyle, un n-propyle, un n-butyle, un phényle ou un benzyle, ou un thiol, et X représente un radical organique ayant un seul atome d'azote quaternaire, et aryle représentant un radical aromatique substitué ou non substitué ou un radical hétéroaromatique substitué ou non substitué, qui ne contient aucun atome d'azote, et
dans lequel le radical R5 ou R6 qui n'est pas -(C(D)(E))ₕ-X ou -(C(D)(E))ₖ-aryl-(C(D)(E))ₗ-X, représente un hydrogène, un alkyle ayant 1 à 20 atomes de carbone, un alcényle ayant 2 à 20 atomes de carbone, un cycloalkyle ayant 3 à 20 atomes de carbone, un éther ayant 2 à 20 atomes de carbone, un thioéther ayant 2 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N,
ou
dans lequel seul le radical R6 représente un radical organique de Formule générale -(C(D)(E))ₕ-X ou -(C(D)(E))ₖ-aryl-(C(D)(E))ₗ-X, et le radical R5 représentant un radical polyol acyclique de Formule générale -CH₂(CH(OH))_{g}CH₂OH ou un éther, un ester ou un acétal de celui-ci, g représentant un nombre entier de 1 à 10, de préférence de 1 à 4,
et dans lequel les radicaux R1 à R4 peuvent chacun indépendamment des autres être identiques ou différents, et représentant un hydrogène, un halogène, un hydroxyle, un thiol, un nitro, un carboxylate, un aldéhyde ayant 1 à 20 atomes de carbone, une cétone ayant 2 à 20 atomes de carbone, un O-alkyle ayant 1 à 20 atomes de carbone, un S-alkyle ayant 1 à 20 atomes de carbone, un O-alcényle ayant 2 à 20 atomes de carbone, un S-alcényle ayant 2 à 20 atomes de carbone, un O-aryle ayant 5 à 20 atomes de carbone, un S-aryle ayant 5 à 20 atomes de carbone, un éther ayant 2 à 20 atomes de carbone, un thioéther ayant 2 à 20 atomes de carbone, un ester d'acide carboxylique ayant 1 à 20 atomes de carbone, un carboxamide ayant 1 à 20 atomes de carbone, un thioester ayant 1 à 20 atomes de carbone, un alkyle ayant 1 à 20 atomes de carbone, un alcényle ayant 2 à 20 atomes de carbone, un cycloalkyle ayant 3 à 20 atomes de carbone, un cycloalcényle ayant 3 à 20 atomes de carbone, un aryle ayant 5 à 20 atomes de carbone ou un hétéroaryle ayant 4 à 20 atomes de carbone, qui ne contient pas d'atomes N.

6. Composition pharmaceutique pour une utilisation selon la revendication 5, les radicaux R1 et R4, qui peuvent chacun indépendamment de l'autre être identiques ou différents, représentent un hydrogène ou un méthyle, et seul un radical R2, R3, R5 ou R6 représentant un radical organique de Formule générale -(C(D)(E))ₕ-X ou -(C(D)(E))ₖ-aryl-(C(D)(E))ₗ-X.

7. Composition pharmaceutique pour une utilisation selon l'une des revendications 5 ou 6, X représentant un radical de Formule générale (2) : 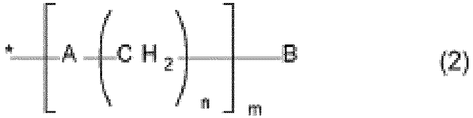 et A représentant un atome d'oxygène ou d'azote, et N représentant un nombre entier de 1 à 8 et m représentant un nombre entier de 0 à 100, et B représentant un radical de Formule (3), (4a), (4b), (5a) ou (5b) : 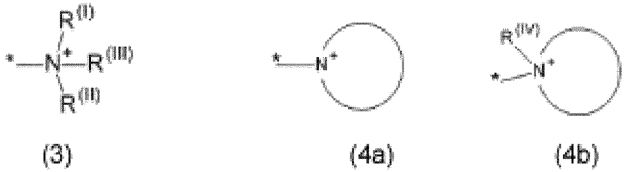 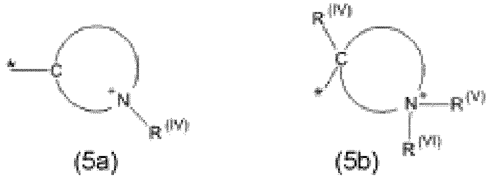 et chacun des radicaux R ^{(I)}, R ^{(II)}, R ^{(III)}, R ^{(IV)} et R ^{(V)} représentant chacun indépendamment des autres un aryle en C1 à C20, un alkyle en C1 à C20, qui peut être à chaîne droite ou ramifiée,
ou représente un éther en C1 à C20, et le radical de Formule (4a) et le radical de Formule (5a) : 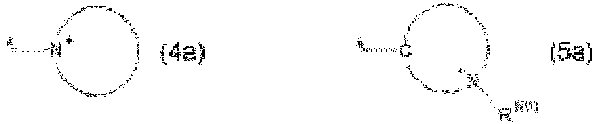 représentant un radical hétérocyclique substitué ou non substitué ayant 5 à 7 atomes nucléaires, qui comprennent au moins 1 atome de carbone et 1 atome d'azote ainsi qu'éventuellement 1 ou 2 atomes d'oxygène ou de soufre, 1 atome d'azote constituant une double liaison, et le radical de Formule (4b) et le radical de Formule (5b) représentant : 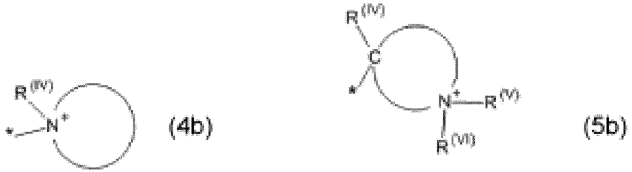 un radical hétérocyclique substitué ou non substitué ayant 5 à 7 atomes nucléaires, qui comprennent au moins 1 atome de carbone et 1 atome d'azote ainsi qu'éventuellement 1 ou 2 atomes d'oxygène ou de soufre, 1 atome d'azote formant une liaison simple.

8. Composition pharmaceutique pour une utilisation selon l'une des revendications 5 à 7, le composé de Formule (1) étant dans le groupe constitué par les composés ayant les Formules (30) à (43) :

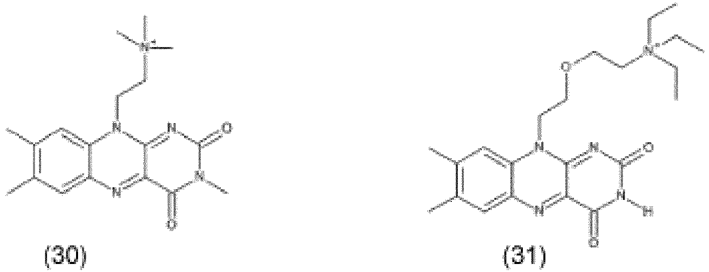

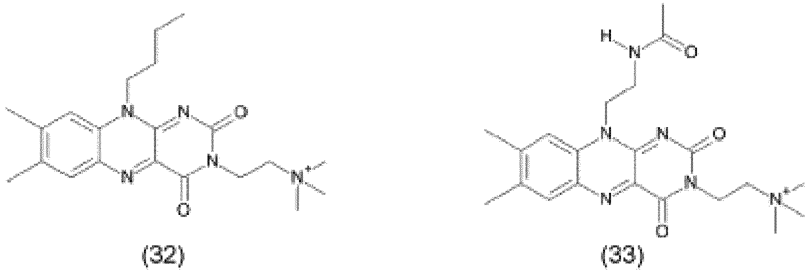

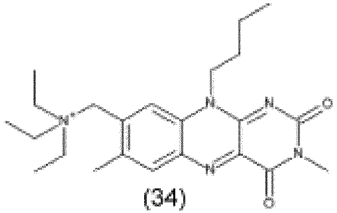

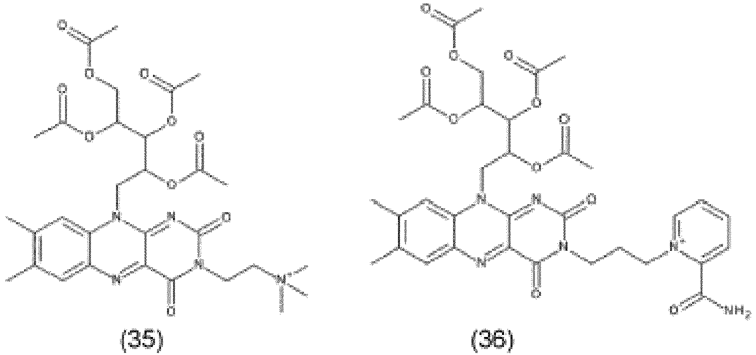

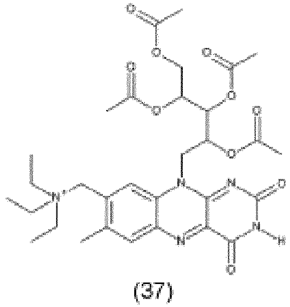

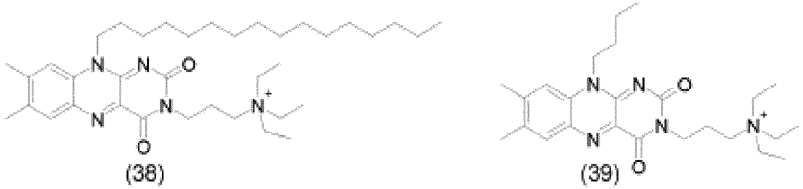

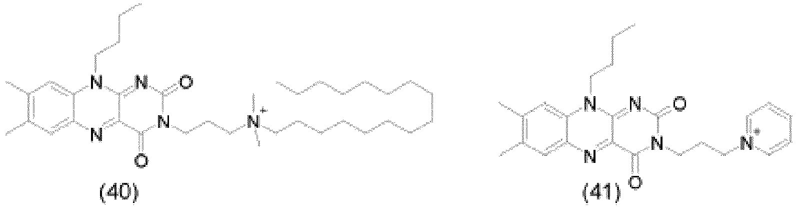

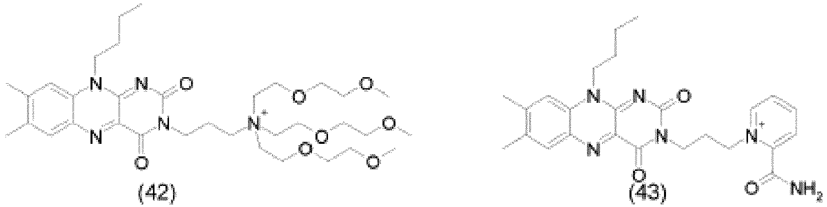

9. Composition pharmaceutique pour une utilisation selon l'une des revendications 5 à 8, l'adjuvant physiologiquement acceptable étant choisi dans le groupe constitué par les charges et les diluants, les solvants, les émulsifiants, les lubrifiants, les correcteurs de goût, les colorants et/ou les substances tampons, liquides ou solides, pharmaceutiquement usuels.

10. Composé pour une utilisation selon l'une des revendications 1 à 4 ou composition pharmaceutique pour une utilisation selon les revendications 5 à 9, les microorganismes étant choisis dans le groupe constitué par les virus, les archées, les bactéries, les spores bactériennes, les champignons, les spores fongiques, les protozoaires, les algues et les parasites transmissibles par le sang.

11. Composé pour une utilisation selon l'une des revendications 1 à 4 ou 10, ou composition pharmaceutique pour une utilisation selon l'une des revendications 5 à 10, pour la désinfection et/ou la décolonisation de surfaces corporelles, de préférence la peau ou une muqueuse, d'un mammifère, de préférence l'homme.

12. Composé pour une utilisation selon l'une des revendications 1 à 4, 10 ou 11 ou composition pharmaceutique pour une utilisation selon l'une des revendications 5 à 11, pour administration topique, de préférence nasale, orale, anale, vaginale ou dermique.

13. Composé pour une utilisation selon l'une des revendications 1 à 4, 10, 11 ou 12, ou composition pharmaceutique pour une utilisation selon l'une des revendications 5 à 12 pour la prophylaxie et/ou le traitement d'une maladie cutanée infectieuse, qui est de préférence choisie dans le groupe constitué par le syndrome de la peau ébouillantée staphylococcique, l'impetigo, un abcès cutané, un furoncle, une escarboucle, des flegmons, une cellulite, une lymphadénite aiguë, des kystes pilonidaux, une pyodermie, une dermatite purulente, une dermatite septique, une dermatite suppurante, un érythrasme, un érysipèle, un acné vulgaire ou une infection fongique.

14. Composé pour une utilisation selon l'une des revendications 1 à 4, 10, 11 ou 12 ou composition pharmaceutique pour une utilisation selon l'une des revendications 5 à 12, pour la désinfection et/ou la réduction du nombre de germes dans des plaies infectées.

15. Composé pour une utilisation selon l'une des revendications 1 à 4, 10, 11 ou 12 ou composition pharmaceutique pour une utilisation selon l'une des revendications 5 à 12, pour la prophylaxie et/ou le traitement de maladies infectieuses de l'oreille, des voies aériennes supérieures, de la cavité buccale, du pharynx, du larynx, des voies aériennes inférieures et/ou de l'œsophage.

16. Composé pour une utilisation selon l'une des revendications 1 à 4, 10, 11 ou 12 ou composition pharmaceutique pour une utilisation selon l'une des revendications 5 à 12, pour le traitement et/ou la prophylaxie d'une infection du tissu dentaire, de préférence une plaque, une carie ou une pulpite, et/ou d'une maladie infectieuse du parodonte, de préférence une gingivite, une paradontite, une endodontite ou une périimplantite.
